# EUROPEAN PATENT APPLICATION

(11) **EP 4 810 508 A1**
(43) Date of publication of application: **23.09.2026**
(21) Application number: 24913384.4
(22) Date of filing: 26.12.2024
(51) Int. Cl.: C07H 15/203, C07H 15/26, C07H 5/10, A61K 31/7034, A61K 31/7042, A61K 31/706, A61P 25/28

(54) **ARABINOSE DERIVATIVE COMPOUND, AND COMPOSITION COMPRISING SAME FOR PREVENTING OR TREATING DEGENERATIVE BRAIN DISEASES**

(30) Priority: 27.12.2023 KR 20230193324; 23.12.2024 KR 20240194319
(71) Applicant: Digmbio. Inc., Seongnam-si, Gyeonggi-do 13486 (KR)
(72) Inventor: JUNG, Myung Ho, Seongnam-si, Gyeonggi-do 13486 (KR); LEE, Ju Young, Seongnam-si, Gyeonggi-do 13486 (KR); KO, Dong Uk, Seongnam-si, Gyeonggi-do 13486 (KR); JEONG, Da Yeon, Seongnam-si, Gyeonggi-do 13486 (KR); SONG, Geon Hee, Seongnam-si, Gyeonggi-do 13486 (KR); GU, Ja Eun, Seongnam-si, Gyeonggi-do 13486 (KR); KIM, Eun Seo, Seongnam-si, Gyeonggi-do 13486 (KR); LIM, Sun Ha, Seongnam-si, Gyeonggi-do 13486 (KR); KIM, Hyun Jung, Seongnam-si, Gyeonggi-do 13486 (KR); JUNG, Myung Eun, Seongnam-si, Gyeonggi-do 13486 (KR); KIM, Jung-Ho, Seongnam-si, Gyeonggi-do 13486 (KR); KIM, Jeongmin, Seongnam-si, Gyeonggi-do 13486 (KR)
(74) Representative: Elend, Almut Susanne
(86) International application number: PCT/KR2024/021089
(87) International publication number: WO 2025/143777

(57) **Abstract**

The present invention relates to a compound represented by Formula I, or an isomer, hydrate, solvate, prodrug, or pharmaceutically acceptable salt thereof, which has efficacy as a neuroregenerative and protective agent targeting the taste receptor GPCR T1R3, as well as a method for preparing the compound and a pharmaceutical composition comprising the compound.

## Description

### [Technical Field]

The present invention relates to an arabinose derivative compound, in particular to an arabinose derivative compound that targets the GPCR T1R3 taste receptor and has efficacy as a brain nerve regeneration and protective agent, a method for producing the same, and a pharmaceutical composition comprising the same.

### [Background technology]

Dementia is a degenerative brain disease caused by Alzheimer's disease, vascular disease and other causes. Among them, Alzheimer's dementia has the characteristic of slow progression. It starts with memory impairment in the early stage and gradually accompanied by other cognitive function impairments such as language function and judgment ability, and finally loses all daily functions.

It is known that approximately 60-70% of dementia patients suffer from Alzheimer's disease. Currently available drugs include Aricept, Exelon, Razadyne, and Namenda, but they have limitations as they only maintain cognitive function and temporarily alleviate symptoms. On the other hand, no new Alzheimer's treatment drug has been approved since 2003.

Currently, most pathological research on the causes of Alzheimer's disease β has focused on β-Amyloid (Aβ)β and Tau protein, but as all clinical trials have failed so far, there is an urgent need for new target-based drug development efforts. The present invention has developed a novel drug candidate for a fundamental curable dementia treatment by targeting T1R3 GPCR, a new (Novel) target that is differentiated from the β-amyloid plaques and Tau aggregates generation inhibition/removal targets that have been most actively developed until recently.

T1R3 (Taste receptor type 1 member 3) is a constituent protein of the sweet taste receptors (T1R2/T1R3) and umami taste receptors (T1R1/T1R3) present on the tongue. It is a GPCR (G protein-coupled receptor) belonging to class C, and research on the in vivo function of T1R3 is actively underway as it has recently been confirmed that T1R3 is expressed not only in the tongue but also in various major organs within the human body. T1R3 shows high expression in the hippocampus and cortex, which are particularly closely associated with Alzheimer's disease (AD), and numerous studies are being conducted to prove the relationship between T1R3 and AD.

The pharmacological proof of concepts for T1R3 as an AD target can be confirmed through the following four studies.

First, unlike normal mice, T1R3 KO mice, in which only the T1R3 gene was knocked out, exhibit abnormally expanded cell bodies in the hippocampal CA1 region of the cerebrum, resulting in increased neuritic density, while spine density is relatively reduced. Additionally, dendrite length is increased compared to normal animals. Furthermore, when performing novel object preference (NOP) and Morris water maze (MWM) tests-representative behavioral observation methods for evaluating cognitive function, learning, and memory-T1R3 mice showed distinct differences from normal mice, exhibiting low interest in new objects and learning and memory impairments. In addition, various behavioral observations, including the Open Field Test, confirmed that T1R3 KO mice exhibited low sociality, reduced exploratory behavior, and a high anxiety response to new spaces. These results suggest that there is a very close relationship between AD dementia and the function of T1R3.

Second, in signal transduction via T1R3 GPCRs, T1R3 stimulates Gαs and Gαq, and 1) Gαs stimulation affects cAMP and calcium, and when PKA (protein kinase A) is activated by cAMP, CREB is phosphorylated to pCREB, which binds to CRE (cAMP response element), a binding site on DNA, and is involved in the expression of BDNF, DCX, and MAP-2. Also, 2) when Gαq is activated, PIP2 (phosphatidylinositol 4,5-bisphosphate) is converted into DAG (diacylglycerol) and IP3 (inositol triphosphate) by PLC (phospholipase C). When the generated IP3 binds to IP3R, an IP3 receptor located in the ER (endoplasmic reticulum), calcium in the ER is released into the cell and binds to CaM (calmodulin), activating the dephosphorylation enzyme CaN (calcineurin). When the activated CaN dephosphorylates and activates NFAT (nuclear factor of activated T-cells), a transcription factor that expresses genes related to axon growth and neurogenesis, NFAT binds to NFAT-RE, thereby promoting transcription.

Third, P/PS1 transgenic mice are a representative AD model that accumulates amyloid plaques in the cerebrum by increasing the levels of β-Amyloid (Aβ), the pathogenic factor of AD dementia. In the hippocampus of APP/PS1 transgenic mice, DCX (Doublecortin), synaptophysin, and progranulin were reduced, but it was confirmed that these levels significantly increased upon administration of T1R3 Agonist.

Finally, it has been reported that when the expression of T1R3 decreases in an abnormal or aging process, the awareness of sweetness has been reduced, and in particular, dementia patients including AD are known to have much reduced human intelligence with respect to sweetness and savory taste compared to normal individuals.

Based on the major literature review and experimental results above, we confirmed that T1R3 in the hippocampus and cortex can be a promising novel target for the treatment of Alzheimer's disease (AD). Furthermore, unlike existing neurogenesis targets which are endogenous enzymes, the T1R3 target is a novel GPCR agonist, which presents a significant distinction.

Accordingly, the inventors of the present invention searched through library data of approximately 4,500 types stored in a plant extract bank to select 12 types of extracts and selected one candidate extract through phenotype-based screening. Among the various mixed components of the extract, they derived a pre-candidate substance, a single active ingredient confirmed to bind to GPCR T1R3 and be effective against AD dementia, developed a derivative compound exhibiting useful effects as an Alzheimer's treatment, and completed the present invention by confirming that it can be usefully utilized as a pharmaceutical composition for prevention or treatment.

### [Prior Art Literature]

### [Patent Literature]

Korean Patent Publication No. 2010-0015967 (Publication Date: Feb. 12, 2010)

### [Summary of the Invention]

### [Technical Problem]

The basic objective of the present invention is to provide a compound represented by the following Chemical Formula I, or an isomer, hydrate, solvate, prodrug, or pharmaceutically acceptable salt thereof.

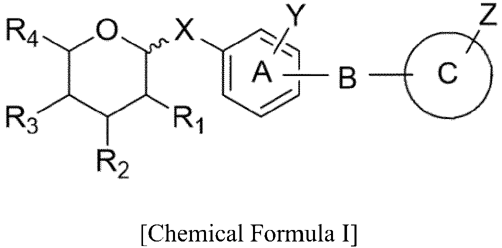

Specific forms of the above Chemical Formula I are as described in this specification.

Another object of the present invention is to provide a composition containing the aforementioned compound, or its isomer, hydrate, solvate, prodrug, or pharmaceutically acceptable salt as an active ingredient.

Another object of the present invention is to provide a production method for producing the compound of Formula 1.

### [Technical Solution]

To achieve the above object, one aspect of the present invention is a compound represented by the following Chemical Formula I, or an isomer, hydrate, solvate, prodrug, or pharmaceutically acceptable salt thereof: 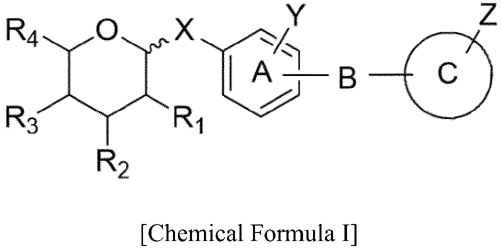 wherein,
R₁, R₂, and R₃ are each independently hydrogen, hydroxy, methoxy, fluoro, azide, amine, 10-aminodecanoate, acetyl, or benzyl;
R₄ is hydrogen or methyl;
X is oxygen, nitrogen, sulfur, or -CH₂-;
A is phenyl which is unsubstituted or substituted with one or more Y;
each substituent Y is selected from the group consisting of hydrogen, straight-chain or branched C₁₋₃ alkyl, and halogen;
B is a direct bond, -[CH₂]ₙ-, -C(CH₃)₂-, -C(CF₃)₂-, -O-, -NH-, -SO₂-, 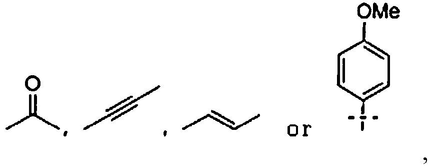 where n is an integer from 1 to 3;
ring C is C₅₋₇ cycloalkyl, C₅₋₇ heterocycloalkyl, phenyl, biphenyl, naphthyl, aryl, or heteroaryl which is unsubstituted or substituted with one or more Z; and
each substituent Z is selected from the group consisting of straight-chain or branched C₁-₆ alkyl, cycloalkyl, phenyl, heteroaryl, halogen, amine, cyano, nitro, alkoxy, and thiol.

The compound according to the present invention may be an arabinose derivative compound based on arabinose. As described above, the inventors screened approximately 4,500 library materials to select 12 extracts, and then selected 1 candidate extract through phenotype-based screening. Among the various mixed components of the selected extract, arabinose was derived as a pre-candidate substance that was confirmed to bind to GPCR T1R3 and have efficacy against AD dementia as a single active ingredient. Subsequently, an arabinose derivative compound was developed that exhibits useful effects as an Alzheimer's disease therapeutic agent.

The compound according to the present invention is such that R₁, R₂, and R₃ in the above formula I are each independently hydrogen, hydroxyl, methoxy, fluoro, azide, amine, 10-aminodecanoate, acetyl, or benzyl, and R₄ is hydrogen or methyl. As an example of the present invention, it is possible that at least two or more of R₁, R₂, and R₃ in the above formula I are each hydroxyl; and R₄ is hydrogen. Furthermore, it is also possible that R₁, R₂, and R₃ in the above formula I are each hydroxyl; and R₄ is hydrogen.

According to one embodiment of the present disclosure, X is oxygen, nitrogen, sulfur, or -CH₂-. As an example of the present invention, it is possible that X is oxygen.

According to one embodiment of the present disclosure, A is unsubstituted phenyl or phenyl substituted with Y. As an example of the present invention, it is possible that A is unsubstituted phenyl.

According to one embodiment of the present disclosure, the substituent Y is one or more selected from the group consisting of hydrogen, straight-chain or branched C₁-₃ alkyl, and halogen. As an example of the present invention, A may be phenyl substituted with Y, and the substituent Y may be halogen.

According to one embodiment of the present disclosure, X-A in formula I is 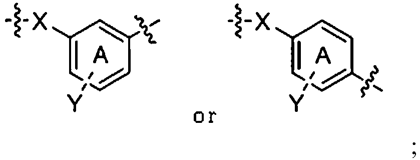 and the substituent Y is a straight or branched C₁-₃ alkyl or a halogen. In the present invention, B may be connected to positions C₂ to C₆ of A, and B may be substituted at position C₃, which is a meta position relative to X, or at position C₄, which is a para position in A (aryl group)

According to one embodiment of the present disclosure, B is a direct bond, -[CH₂]ₙ-, - C(CH₃)₂-, -C(CF₃)₂-, -O-, -NH-, -SO₂-, 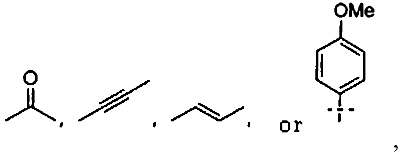 where n is an integer from 1 to 3. As an example of the present invention, B may be a direct bond. As an example of the present invention, B may be -CH₂-. As an example of the present invention, B may be -O-.

According to one embodiment of the present disclosure, ring C is C₅₋₇ cycloalkyl, C₅₋₇ heterocycloalkyl, phenyl, biphenyl, naphthyl, aryl, or heteroaryl which is unsubstituted or substituted with one or more Z. As an example of the present invention, ring C may be unsubstituted C₅₋₇ cycloalkyl, C₅₋₇ heterocycloalkyl, phenyl, biphenyl, naphthyl, aryl, or heteroaryl; and ring C substituted with Z may be Z-substituted C₅₋₇ cycloalkyl, C₅₋₇ heterocycloalkyl, phenyl, biphenyl, naphthyl, aryl, or heteroaryl. The cycloalkyl, heterocycloalkyl, aryl, or heteroaryl may be a monocyclic, bicyclic, or tricyclic ring compound, and may also be a ring compound having four or more rings. As an example of the present invention, ring C may be an unsubstituted or Z-substituted phenyl. As an example of the present invention, ring C may be an unsubstituted or Z-substituted naphthyl or heteroaryl.

According to one embodiment of the present disclosure, B-ring C may be 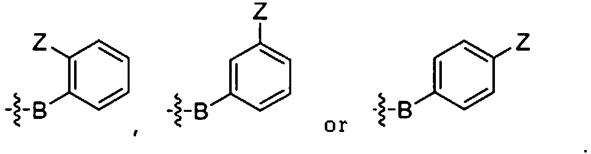 In the present disclosure, the substituent Z may be connected to or substituted at the ortho, meta, or para positions of ring C.

According to one embodiment of the present disclosure, B-ring C can be

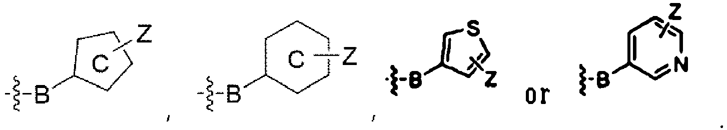

According to one embodiment of the present disclosure, the substituent Z is at least one or more selected from the group consisting of straight-chain or branched C₁-₆ alkyl, cycloalkyl, phenyl, heteroaryl, halogen, amine, cyano, nitro, alkoxy, and thiol. As an example of the present invention, the substituent Z may be a straight-chain or branched-chain C₁-₆ alkyl, halogen, amine, or alkoxy.

The compound according to one embodiment of the present invention may be formed by including X-A (with Y), B, and C (with Z) shown in Table 1 below as a moiety.

**[Table 1]**

| Example | R₁ | R₂ | R₃ | R₄ | X·A(with Y) | B | C(with Z) | Form | HRE assay^{(a)} |
|---|---|---|---|---|---|---|---|---|---|
| 1 | OH | OH | OH | H | 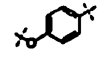 | -CH₂- | 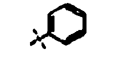 | β -form | •• |
| 2 | OH | OH | OH | H | 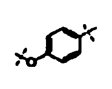 | -CH₂- | 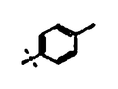 | β -form | ••• |
| 3 | OH | OH | OH | H | 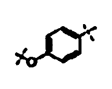 | -CH₂- | 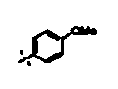 | β -form | •• |
| 4 | OH | OH | OH | H | 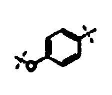 | -CH₂- | 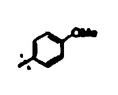 | α -form | α |
| 5 | OMe | OH | OH | H | 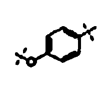 | -CH₂- | 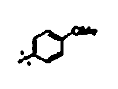 | β-form | |
| 6 | OH | OH | OH | Me | 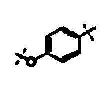 | -CH₂- | 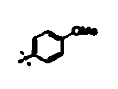 | β-form | • |
| 7 | OH | OH | OH | H | 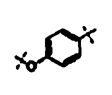 | -CH₂- | 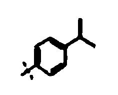 | β -form | ••• |
| 8 | OH | OH | OH | H | 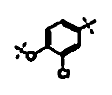 | -CH₂- | 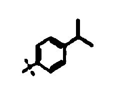 | β -form | ••• |
| 9 | OH | OH | OH | H | 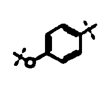 | -CH₂- | 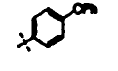 | β -form | • |
| 10 | OH | OH | OH | H | 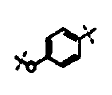 | -CH₂- | 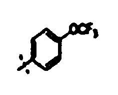 | β -form | • |
| 11 | OH | OH | OH | H | 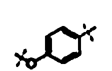 | -CH₂- | 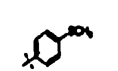 | β -form | ••• |
| 12 | OH | OH | OH | H | 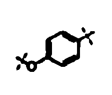 | -CH₂- | 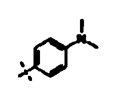 | α -form | • |
| 13 | OH | OH | OH | H | 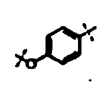 | -CH₂- | 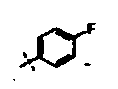 | β -form | •• |
| 14 | OH | OH | OH | H | 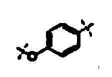 | -CH₂- | 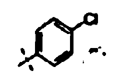 | β -form | •• |
| 15 | OH | OH | OH | H | 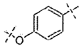 | -CH₂- | 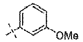 | β **-**form | ** |
| 16 | OH | OH | OH | H | 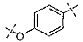 | -CH₂- | 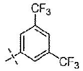 | β **-**form | * |
| 17 | OH | OH | OH | H | 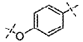 | -CH₂- | 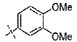 | β -form | * |
| 18 | OH | OH | OH | H | 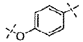 | -CH₂- | 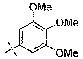 | β -form | * |
| 19 | OH | OH | OH | H | 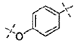 | -CH₂- | 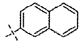 | β -form | * |
| 20 | OH | OH | OH | H | 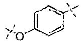 | -CH₂- | 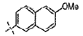 | β -form | * |
| 21 | OH | OH | OH | H | 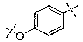 | -CH₂- | 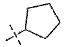 | β -form | *** |
| 22 | OH | OH | OH | H | 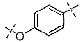 | -CH₂- | 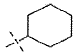 | β -form | *** |
| 23 | OH | OH | OH | H | 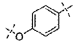 | -CH₂- | 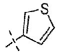 | β -form | ** |
| 24 | OH | OH | OH | H | 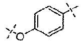 | -CH₂- | 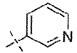 | α -form | * |
| 25 | OH | OH | OH | H | 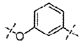 | -CH₂- | 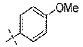 | β -form | - |
| 26 | OH | OH | OH | H | 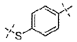 | -CH₂- | 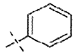 | β -form | *** |
| 27 | OH | OH | OH | H | 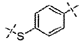 | -CH₂- | 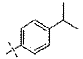 | β -form | *** |
| 28 | OH | OH | OH | H | 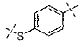 | -CH₂- | 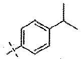 | α -form | ** |
| 29 | OH | OH | OH | H | 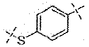 | -CH₂- | 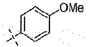 | β -form | *** |
| 30 | OH | OH | OH | H | 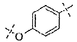 | 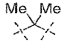 | 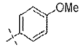 | β -form | * |
| 31 | OH | OH | OH | H | 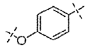 | 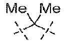 | 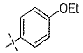 | β -form | * |
| 32 | OH | OH | OH | H | 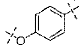 | 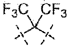 | 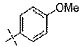 | β -form | * |
| 33 | OH | OH | OH | H | 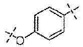 | -O- | 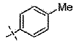 | β -form | *** |
| 34 | OH | OH | OH | H | 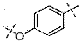 | -O- | 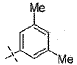 | β -form | * |
| 35 | OH | OH | OH | H | 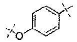 | -O- | 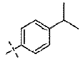 | β -form | *** |
| 36 | OH | OH | OH | H | 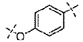 | -O- | 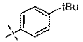 | β -form | *** |
| 37 | OH | OH | OH | H | 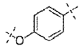 | -CH₂- | 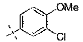 | β -form | * |
| 38 | OH | OH | OH | H | 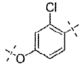 | -CH₂- | 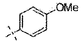 | β -form | *** |
| 39 | OH | OH | OH | H | 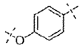 | -CH₂- | 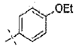 | β -form | * |
| 40 | OH | OH | OH | H | 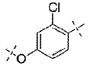 | -CH₂- | 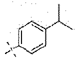 | β -form | **** |
| 41 | OH | OH | OH | H | 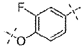 | -CH₂- | 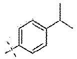 | β -form | **** |
| 42 | OH | OH | OH | H | 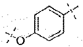 | -CH₂- | 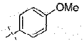 | β -form | **** |
| 43 | OH | OH | OH | H | 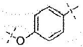 | -CH₂- | 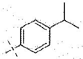 | β -form | **** |
| 44 | OH | OH | OH | H | 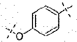 | -CH₂- | 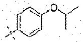 | β -form | * |
| 45 | OH | OH | OH | H | 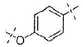 | -CH₂- | 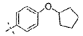 | β -form | * |
| 46 | OH | OH | OH | H | 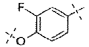 | -CH₂- | 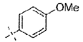 | β -form | * |
| 47 | OH | OH | OH | H | 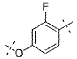 | -CH₂- | 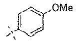 | β -form | * |
| 48 | OH | OH | OH | H | 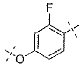 | -CH₂- | 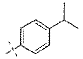 | β -form | *** |
| 49 | OH | OH | OH | H | 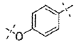 | -CH₂- | 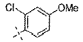 | β -form | * |
| 50 | OH | OH | OH | H | 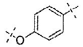 | -CH₂- | 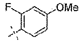 | β -form | * |
| 51 | OH | OH | OH | H | 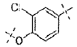 | -CH₂- | 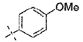 | β -form | *** |
| 52 | OH | OH | OH | H | 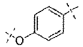 | -CH₂- | 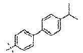 | β -form | **** |
| 53 | OH | OH | OH | H | 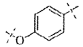 | -CH₂- | 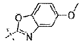 | β -form | - |
| 54 | OH | OH | OH | H | 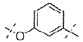 | -O- | 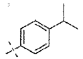 | β -form | * |
| 55 | OH | OH | OH | H | 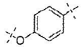 | -O- | 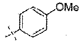 | β -form | *** |
| 56 | OH | OH | OH | H | 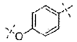 | -O- | 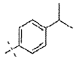 | β -form | **** |
| 57 | OH | OH | OH | H | 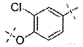 | -O- | 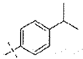 | β -form | **** |
| 58 | OH | OH | F | H | 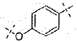 | -O- | 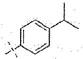 | β -form | **** |
| 59 | OH | OH | OH | CH₂OH | 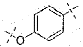 | -O- | 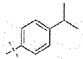 | β -form | *** |
| 60 | OH | OMe | OH | H | 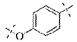 | -O- | 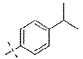 | β -form | **** |
| 61 | OH | OH | OMe | H | 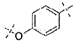 | -O- | 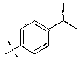 | β -form | * |
| 62 | OMe | OH | OH | H | 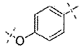 | -O- | 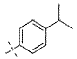 | β -form | **** |
| 63 | F | OH | OH | H | 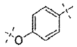 | -O- | 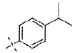 | β -form | **** |
| 64 | OH | OH | F | H | 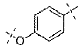 | -O- | 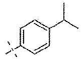 | β -form | **** |
| 65 | F | OH | OH | H | 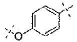 | -O- | 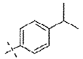 | β -form | **** |
| 66 | H | OH | OH | H | 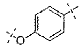 | -O- | 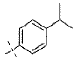 | β -form | **** |
| 67 | OH | OH | OH | H | 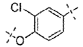 | -O- | 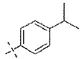 | β -form | **** |
| 68 | OH | OH | OH | H | 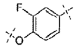 | -O- | 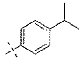 | β -form | **** |
| 69 | N₃ | OH | OH | H | 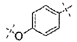 | -O- | 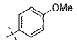 | β -form | *** |
| 70 | NH₂ | OH | OH | H | 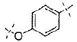 | -O- | 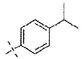 | β -form | *** |
| 71 | OH | OH | OH | H | 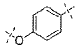 | 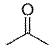 | 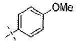 | β -form | *** |
| 72 | OH | OH | OH | H | 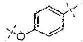 | 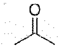 | 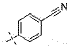 | α -form | * |
| 73 | OH | OH | OH | H | 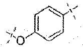 | -CH₂- | 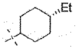 | β -form | **** |
| 74 | OH | OH | OH | H | 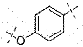 | -CH₂- | 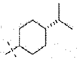 | β -form | *** |
| 75 | OH | OH | OH | H | 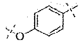 | -CH₂- | 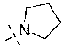 | α -form | * |
| 76 | OH | OH | OH | H | 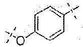 | -CH₂- | 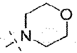 | α -form | * |
| 77 | N₃ | OH | OH | H | 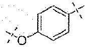 | -CH₂- | 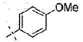 | β -form | * |
| 78 | NH₂ | OH | OH | H | 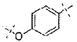 | -CH₂- | 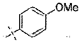 | β -form | *** |
| 79 | OOC-(CH₂)₁₀ -NH₂ | OH | OH | H | 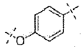 | -CH₂- | 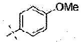 | β -form | * |
| 80 | OH | OH | OH | H | 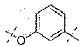 | - | 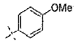 | β -form | - |
| 81 | OH | OH | OH | H | 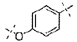 | - | 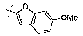 | β -form | **** |
| 82 | OH | OH | OH | H | 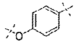 | - | 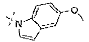 | β -form | *** |
| 83 | OH | OH | OH | H | 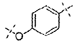 | - | 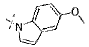 | α -form | **** |
| 84 | OH | OH | OH | H | 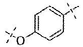 | - | 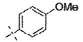 | β -form | **** |
| 85 | OH | OH | OH | H | 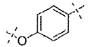 | - | 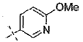 | β -form | *** |
| 86 | OH | OH | OH | H | 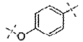 | - | 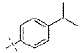 | β -form | *** |
| 87 | OH | OH | OH | H | 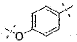 | - | 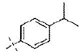 | β -form | **** |
| 88 | OH | OH | OH | H | 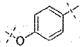 | - | 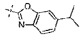 | β -form | *** |
| 89 | OH | OH | OH | H | 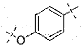 | - | 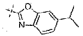 | α -form | **** |
| 90 | OH | OH | OH | H | 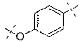 | - | 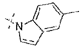 | β -form | *** |
| 91 | OH | OH | OH | H | 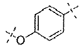 | - | 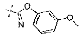 | β -form | **** |
| 92 | OH | OH | OH | H | 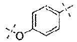 | - | 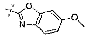 | α -form | **** |
| 93 | OH | OH | OH | H | 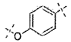 | - | 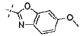 | β -form | **** |
| 94 | OH | OH | OH | H | 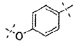 | - | 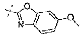 | α -form | **** |
| 95 | OH | OH | OH | H | 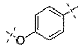 | - | 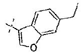 | β -form | **** |
| 96 | OH | OH | OH | H | 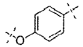 | - | 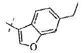 | α -form | **** |
| 97 | OH | OH | OH | H | 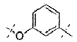 | - | 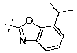 | β -form | * |
| 98 | OH | OH | OH | H | 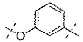 | - | 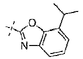 | α -form | * |
| 99 | OH | OH | OH | H | 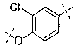 | - | 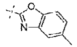 | β -form | **** |
| 100 | OH | OH | OH | H | 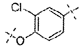 | - | 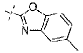 | α -form | **** |
| 101 | OH | OH | OH | H | 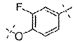 | -O- | 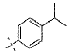 | β -form | ** |
| 102 | OH | OH | OH | H | 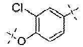 | - | 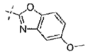 | α -form | **** |
| 103 | OH | OH | OH | H | 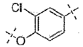 | - | 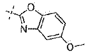 | β -form | **** |
| 104 | OH | OH | OH | H | 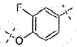 | - | 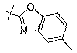 | α -form | **** |
| 105 | OH | OH | OH | H | 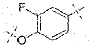 | - | 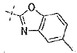 | β -form | **** |
| 106 | OH | OH | OH | H | 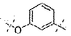 | - | 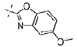 | α -form | **** |
| 107 | OH | OH | OH | H | 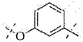 | - | 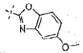 | β -form | **** |
| 108 | OH | OH | OH | H | 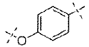 | - | 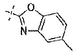 | α -form | **** |
| 109 | OH | OH | OH | H | 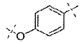 | - | 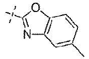 | β -form | **** |
| 110 | OH | OH | OH | H | 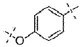 | 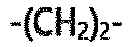 | 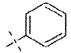 | β -form | **** |
| 111 | OH | OH | OH | H | 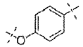 | 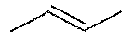 | 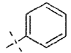 | β -form | - |
| 112 | OH | OH | OH | H | 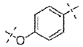 | 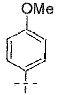 | 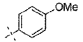 | β -form | - |
| 113 | OH | OH | OH | H | 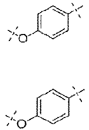 | - | 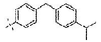 | β -form | - |
| 114 | OH | OH | OH | H | | 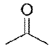 | 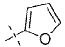 | β -form | * |
| 115 | OH | OH | OH | H | 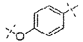 | 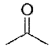 | 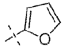 | α -form | * |
| 116 | OH | OH | OH | H | 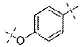 | -(CH₂)₂- | 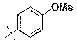 | β -form | - |
| 117 | OH | OH | OH | H | 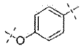 | -(CH₂)₂- | 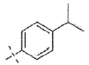 | β -form | - |
| 118 | OH | OH | OH | H | 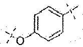 | 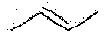 | 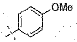 | β -form | - |
| 119 | OH | OH | OH | H | 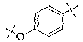 | 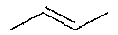 | 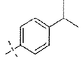 | β -form | - |
| 120 | OH | OH | OH | H | 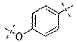 | - | 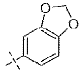 | β -form | - |
| 121 | OH | OH | OH | H | 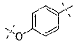 | - | 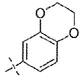 | β -form | - |
| 122 | OH | OH | OH | H | 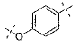 | - | 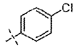 | β -form | - |
| 123 | OH | OH | OH | H | 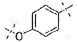 | - | 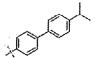 | β -form | - |
| 124 | OH | OH | OH | H | 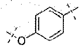 | 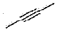 | 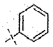 | β -form | - |

(a) HRE assay: Confirmation of increased in HRE expression levels by the test substance relative to the vehicle in HRE/Luc/HEK293 cells
   * Less than a twofold increase at 100 µM,
   ** More than a twofold increase at 100 µM,
   *** More than a twofold increase at 10 µM,
   **** More than a twofold increase at 1 µM,

   - Not performed.

The compounds in Table 1 above may be indicated by the following compound names in order.
<1>(2R,3R,4S,5S)-2-(4-benzyloxyphenoxy)tetrahydro-2*H*-pyran-3,4,5-triol,
<2>(2R,3R,4S,5S)-2-(4-(4-methylbenzyl)phenoxy)tetrahydro-2*H*-pyran-3,4,5-triol,
<3>(2R,3R,4S,5S)-2-(4-(4-methoxybenzyl)phenoxy)tetrahydro-2*H*-pyran-3,4,5-triol,
<4>(2S,3R,4S,5S)-2-(4-(4-methoxybenzyl)phenoxy)tetrahydro-2*H*-pyran-3,4-diol,
<5>(3 S,4S,5R,6R)-5-methoxy-6-(4-(4-methoxybenzyl)phenoxy)tetrahydro-2*H*-pyran-3,4-diol,
<6>(2R,3S,4R,5S,6S)-2-(4-(4-methoxybenzyl)phenoxy)-6-methyltetrahydro-2*H*-pyran-3,4,5-triol,
<7>(2R,3R,4S,5S)-2-(4-(4-isopropylbenzyl)phenoxy)tetrahydro-2*H*-pyran-3,4,5-triol,
<8>(2R,3R,4S,5S)-2-(2-chloro-4-(4-isopropylbenzyl)phenoxy)tetrahydro-2*H*-pyran-3,4,5-triol,
<9>(2R,3R,48S,5S)-2-(4-(4-phenoxybenzyl)phenoxy)tetrahydro-2*H*-pyran-3,4,5-triol,
<10>(2R,3R,4S,5S)-2-(4-(4-(trifluoromethoxy)benzyl)phenoxy)tetrahydro-2*H*-pyran-3,4,5-triol,
<11>(2R,3R,4S,5S)-2-(4-(4-(methylthio)benzyl)phenoxy)tetrahydro-2*H*-pyran-3,4,5-triol,
<12>(2S,3R,4S,5S)-2-(4-(4-(dimethylamino)benzyl)phenoxy)tetrahydro-2*H*-pyran-3,4,5-triol,
<13>(2R,3R,4S,5S)-2-(4-(4-fluorobenzyl)phenoxy)tetrahydro-2*H*-pyran-3,4,5-triol,
<14>(2R,3R,4S,5S)-2-(4-(4-chlorobenzyl)phenoxy)tetrahydro-2*H*-pyran-3,4,5-triol,
<15>(2R,3R,4S,5S)-2-(4-(3-methoxybenzyl)phenoxy)tetrahydro-2*H*-pyran-3,4,5-triol,
<16>(2R,3R,4S,5S)-2-(4-(3,5-bis(trifluoromethyl)benzyl)phenoxy)tetrahydro-2*H*-pyran-3,4,5-triol,
<17>(2R,3R,4S,5S)-2-(4-(3,4-dimethoxybenzyl)phenoxy)tetrahydro-2*H*-pyran-3,4-diol,
<18>(2R,3R,4S,5S)-2-(4-(3,4,5-trimethoxybenzyl)phenoxy)tetrahydro-2*H*-pyran-3,4,5-triol,
<19>(2R,3R,4S,5S)-2-(4-(naphthalen-2-ylmethyl)phenoxy)tetrahydro-2*H*-pyran-3,4,5-triol,
<20>(2R,3R,4S,5S)-2-(4-(6-methoxynaphthalen-2-yl)methyl)phenoxy)tetrahydro-2*H*-pyran-3,4,5-triol,
<21>(2R,3R,4S,5S)-2-(4-(cyclopentylmethyl)phenoxy)tetrahydro-2*H*-pyran-3,4,5-triol,
<22>(2R,3R,4S,5S)-2-(4-(cyclohexylmethyl)phenoxy)tetrahydro-2*H*-pyran-3,4,5-triol,
<23>(2R,3R,4S,5S)-2-(4-(thiophen-3-ylmethyl)phenoxy)tetrahydro-2*H*-pyran-3,4,5-triol,
<24>(2R,3R,4S,5S)-2-(4-(pyridin-3-ylmethyl)phenoxy)tetrahydro-2*H*-pyran-3,4,5-triol,
<25>(2R,3R,4S,5S)-2-(3-(4-methoxybenzyl)phenoxy)tetrahydro-2*H*-pyran-3,4,5-triol,
<26>(2R,3R,4S,5S)-2-((4-benzylphenyl)thio)tetrahydro-2*H*-pyran-3,4,5-thiol,
<27>(2R,3R,4S,5S)-2-((4-(4-isopropylbenzyl)phenyl)thio)tetrahydro-2*H*-pyran-3,4,5-triol,
<28>(2S,3R,4S,5S)-2-((4-(4-isopropylbenzyl)phenyl)thio)tetrahydro-2*H*-pyran-3,4,5-triol,
<29>(2R,3R,4S,5S)-2-((4-(4-methoxybenzyl)phenyl)thio)tetrahydro-2*H*-pyran-3,4,5-thiol,
<30>(2R,3R,4S,5S)-2-(4-(2-(4-methoxyphenyl)propan-2-yl)phenoxy)tetrahydro-2*H*-pyran-3,4,5-triol,
<31>(2R,3R,4S,5S)-2-(4-(2-(4-ethoxyphenyl)propan-2-yl)phenoxy)tetrahydro-2*H*-pyran-3,4,5-triol,
<32>(2R,3R,4S,5S)-2-(4-(1,1,1,3,3,3-hexafluoro-2-(4-methoxyphenyl)propan-2-yl)phenoxy)tetrahydro-2*H*-pyran-3,4,5-triol,
<33>((2R,3R,4S,5 S)-2-(4-(para-tolyloxy)phenoxy)tetrahydro-2H-pyran-3,4,5-triol,
<34>(2R,3R,4S,5S)-2-(4-(3,5-dimethylphenoxy)phenoxy)tetrahydro-2*H*-pyran-3,4,5-triol,
<35>(2R,3R,4S,5S)-2-(4-(4-isopropylphenoxy)phenoxy)tetrahydro-2*H*-pyran-3,4,5-triol,
<36>(2R,3R,4S,5S)-2-(4-(4-(tert-butyl)phenoxy)phenoxy)tetrahydro-2*H*-pyran-3,4,5-triol,
<37>(2R,3R,4S,5S)-2-(4-(3-chloro-4-methoxybenzyl)phenoxy)tetrahydro-2*H*-pyran-3,4,5-triol,
<38>(2R,3R,4S,5S)-2-(3-chloro-4-(4-methoxybenzyl)phenoxy)tetrahydro-2*H*-pyran-3,4,5-triol,
<39>(2R,3R,4S,5S)-2-(4-(4-ethoxybenzyl)phenoxy)tetrahydro-2*H*-pyran-3,4,5-triol,
<40>(2R,3R,4S,5S)-2-(3-chloro-4-(4-isopropylbenzyl)phenoxy)tetrahydro-2*H*-pyran-3,4,5-triol,
<41>(2R,3R,4S,5S)-2-(2-fluoro-4-(4-isopropylbenzyl)phenoxy)tetrahydro-2*H*-pyran-3,4,5-triol,
<42>(2R,3R,4S,5R)-2-(4-(4-methoxybenzyl)phenoxy)tetrahydro-2*H*-pyran 3,4,5-triol,
<43>(2R,3R,4S,5R)-2-(4-(4-isopropylbenzyl)phenoxy)tetrahydro-2*H*-pyran-3,4,5-triol,
<44>(2R,3R,4S,5S)-2-(4-(4-isopropoxybenzyl)phenoxy)tetrahydro-2*H*-pyran-3,4,5-triol,
<45>(2R,3R,4S,5S)-2-(4-(4-(cyclopentyloxy)benzyl)phenoxy)tetrahydro-2*H*-pyran-3,4,5-triol,
<46>(2R,3R,4S,5S)-2-(2-fluoro-4-(4-methoxybenzyl)phenoxy)tetrahydro-2*H*-pyran-3,4,5-triol,
<47>(2R,3R,4S,5S)-2-(3-fluoro-4-(4-methoxybenzyl)phenoxy)tetrahydro-2*H*-pyran-3,4,5-triol,
<48>(2R,3R,4S,5S)-2-(3-fluoro-4-(4-isopropylbenzyl)phenoxy)tetrahydro-2*H*-pyran-3,4,5-triol,
<49>(2R,3R,4S,5S)-2-(4-(2-chloro-4-methoxybenzyl)phenoxy)tetrahydro-2*H*-pyran-3,4,5-triol,
<50>(2R,3R,4S,5S)-2-(4-(2-fluoro-4-methoxybenzyl)phenoxy)tetrahydro-2*H*-pyran-3,4,5-triol,
<51>((2R,3R,4S,5S)-2-(2-chloro-4-(4-methoxybenzyl)phenoxy)tetrahydro-2*H*-pyran-3,4,5-triol,
<52>(2R,3R,4S,5S)-2-(4-((4'-isopropyl-[1,1'-biphenyl]-4yl)methyl)phenoxy)tetrahydro-2*H-*pyran-3,4,5-triol,
<53>(2R,3R,4S,5S)-2-(4-((5-methoxybenzo[d]oxazol-2yl)methyl)phenoxy)tetrahydro-2H-pyran-3,4,5-triol,
<54>(2R,3R,4S,5S)-2-(3-(4-isopropylphenoxy)phenoxy)tetrahydro-2*H*-pyran-3,4,5-triol,
<55>(2R,3R,4S,SS)-2-(4-(4-methoxyphenoxy)phenoxy)tetrahydro-2*H*-pyran-3,4,5-triol,
<56>(2R,3R,4S,5R)-2-(4-(4-isopropylphenoxy)phenoxy)tetrahydro-2H-pyran-3,4,5-triol,
<57>(2R,3R,4S,5 S)-2-(2-chloro-4-(4-isopropylphenoxy)phenoxy)tetrahydro-2*H*-pyran-3,4,5-triol,
<58>(2R,3R,4R,5S)-5-fluoro-2-(4-(4-isopropylphenoxy)phenoxy)tetrahydro-2*H*-pyran-3,4-diol,
<59>(2R,3R,4S,5R,6R)-2-(hydroxymethyl)-6-(4-(4-isopropylphenoxy)phenoxy)tetrahydro-2*H-*pyran-3,4,5-triol,
<60>(2R,3R,4S,5S)-2-(4-(4-isopropylphenoxy)phenoxy)-4-methoxy-tetrahydro-2*H*-pyran-3,5-diol,
<61>(2R,3R,4R,5S)-2-(4-(4-isopropylphenoxy)phenoxy)-5-methoxy-tetrahydro-2*H*-pyran-3,4-diol,
<62>(3S,4S,5R,6R)-6-(4-(4-isopropylphenoxy)phenoxy)-5-methoxytetrahydro-2*H*-pyran-3,4-diol,
<63>(3S,4S,5R,6R)-5-fluoro-6-(4-(4-isopropylphenoxy)phenoxy)tetrahydro-2*H*-pyran-3,4-diol,
<64>(2R,3R,4R,5R)-5-fluoro-2-(4-(4-isopropylphenoxy)phenoxy)tetrahydro-2*H*-pyran-3,4-diol,
<65>(3S,4S,5S,6R)-5-fluoro-6-(4-(4-isopropylphenoxy)phenoxy)tetrahydro-2*H*-pyran-3,4-diol,
<66>(3 S,4R,6R)-6-(4-(4-isopropylphenoxy)phenoxy)tetrahydro-2H-pyran-3,4-diol,
<67>(2R,3R,4S,5R)-2-(2-chloro-4-(4-isopropylphenoxy)phenoxy)tetrahydro-2*H*-pyran-3,4,5-triol,
<68>(2R,3R,4S,5S)-2-(2-fluoro-4-(4-isopropylphenoxy)phenoxy)tetrahydro-2*H*-pyran-3,4,5-triol,
<69>(3S,4R,5R,6R)-5-azido-6-(4-(4-isopropylphenoxy)phenoxy)tetrahydro-2*H*-pyran-3,4-diol,
<70>(3S,4R,5R,6R)-5-amino-6-(4-(4-isopropylphenoxy)phenoxy)tetrahydro-2*H*-pyran-3,4-diol,
<71>(4-methoxyphenyl)(4-(((2R,3R,4S,5S)-3,4,5-trihydroxytetrahydro-2*H*-pyran-2-yl)oxy)phenyl)methanone,
<72>(4-(4-(((2S,3R,4S,5S)-3,4,5-trihydroxytetrahydro-2H-pyran-2-yl)oxy)benzoyl)benzonitrile,
<73>(2R,3R,4S,5S)-2-(4-(((1r,4R)-4-ethylcyclohexyl)methyl)phenoxy)tetrahydro-2*H*-pyran-3,4,5-triol,
<74>(2R,3R,4S,5S)-2-(4-(((1r,4R)-4-isopropylcyclohexyl)methyl)phenoxy)tetrahydro-2*H-*pyran-3,4,5-triol,
<75>(2S,3R,4S,5S)-2-(4-((pyrrolidin-1-ylmethyl)phenoxy)tetrahydro-*2H*-pyran-3,4,5-triol,
<76>(2S,3R,4S,5S)-2-(4-(morpholinomethyl)phenoxy)tetrahydro-*2H*-pyran-3,4,5-triol,
<77>(3S,4R,5R,6R)-5-azido-6-(4-(4-methoxybenzyl)phenoxy)tetrahydro-2*H*-pyran-3,4-diol,
<78>(3S,4R,5R,6R)-5-azido-6-(4-(4-methoxybenzyl)phenoxy)tetrahydro-2*H*-pyran-3,4-diol,
<79>(2R,3R,4S,5S)-4,5-dihydroxy-2-(4-(4-methoxybenzyl)phenoxy)tetrahydro-2*H*-pyran-3-yl 10-aminodecanoate,
<80>(2R,3R,4S,5S)-2-((4'-methoxy-[1,1'-biphenyl]-3-yl)oxy)tetrahydro-2*H*-pyran-3,4,5-triol,
<81>(2R,3R,4S,5S)-2-(4-(6-methoxybenzofuran-2-yl)phenoxy)tetrahydro-2*H*-pyran-3,4,5-triol,
<82>(2R,3R,4S,5S)-2-(4-(5-methoxy-*1H*-indol-1-yl)phenoxy)tetrahydro-*2H*-pyran-3,4,5-triol,
<83>(2S,3R,4S,5S)-2-(4-(5-methoxy-*1H*-indol-1-yl)phenoxy)tetrahydro-*2H*-pyran-3,4,5-triol,
<84>(2R,3R,4S,5S)-2-((4'-methoxy-[1,1'-biphenyl]-4-yl)oxy)tetrahydro-*2H*-pyran-3,4,5-triol,
<85>(2R,3R,4S,5S)-2-(4-(6-methoxypyridin-3-yl)phenoxy)tetrahydro-*2H*-pyran-3,4,5-triol,
<86>(2R,3R,4S,5S)-2-((4'-isopropyl-[1,1'-biphenyl]-4-yl)oxy)tetrahydro-*2H*-pyran-3,4,5-triol,
<87>(2S,3R,4S,5S)-2-((4'-isopropyl-[1,1'-biphenyl]-4-yl)oxy)tetrahydro-*2H*-pyran-3,4,5-triol,
<88>(2R,3R,4S,5S)-2-(4-(6-isopropylbenzo[d]oxazol-2-yl)phenoxy)tetrahydro-*2H*-pyran-3,4,5-triol,
<89>(2S,3R,4S,5S)-2-(4-(6-isopropylbenzo[d]oxazol-2-yl)phenoxy)tetrahydro-*2H*-pyran-3,4,5-triol,
<90>(2R,3R,4S,5S)-2-(4-(5-methyl-*1H*-indol-1-yl)phenoxy)tetrahydro-*2H*-pyran-3,4,5-triol,
<91>(2R,3R,4S,5S)-2-(4-(5-methoxybenzo[d]oxazol-2-yl)phenoxy)tetrahydro-*2H*-pyran-3,4,5-triol,
<92>(2S,3R,4S,5S)-2-(4-(5-methoxybenzo[d]oxazol-2-yl)phenoxy)tetrahydro-*2H*-pyran-3,4,5-triol,
<93>(2R,3R,4S,5R)-2-(4-(5-methoxybenzo[d]oxazol-2-yl)phenoxy)tetrahydro-*2H*-pyran-3,4,5-triol,
<94>(2S,3R,4S,5R)-2-(4-(5-methoxybenzo[d]oxazol-2-yl)phenoxy)tetrahydro-*2*H-pyran-3,4,5-triol,
<95>(2R,3R,4S,5S)-2-(4-(6-ethylbenzofuran-3-yl)phenoxy)tetrahydro-*2H*-pyran-3,4,5-triol,
<96>(2S,3R,4S,5S)-2-(4-(6-ethylbenzofuran-3-yl)phenoxy)tetrahydro-*2H*-pyran-3,4,5-triol,
<97>(2R,3R,4S,5S)-2-(3-(7-isopropylbenzo[d]oxazol-2-yl)phenoxy)tetrahydro-*2H*-pyran-3,4,5-triol,
<98>(2S,3R,4S,5S)-2-(3-(7-isopropylbenzo[d]oxazol-2-yl)phenoxy)tetrahydro-*2H*-pyran-3,4,5-triol,
<99>(2R,3R,4S,5S)-2-(2-chloro-4-(5-methylbenzo[d]oxazol-2-yl)phenoxy)tetrahydro-*2H-*pyran-3,4,5-triol,
<100>(2S,3R,4S,5S)-2-(2-chloro-4-(5-methylbenzo[d]oxazol-2-yl)phenoxy)tetrahydro-*2H-*pyran-3,4,5-triol,
<101>(2R,3R,4S,5R)-2-(2-fluoro-4-(4-isopropylphenoxy)phenoxy)tetrahydro-*2H*-pyran-3,4,5-triol,
<102>(2S,3R,4S,5S)-2-(2-chloro-4-(5-methoxybenzo[d]oxazol-2-yl)phenoxy)tetrahydro-*2H-*pyran-3,4,5-triol,
<103>(2R,3R,4S,5S)-2-(2--chloro-4-(5-methoxybenzo[d]oxazol-2-yl)phenoxy)tetrahydro-*2H-*pyran-3,4,5-triol,
<104>(2S,3R,4S,5S)-2-(2-fluoro-4-(5-methylbenzo[d]oxazol-2-yl)phenoxy)tetrahydro-*2H-*pyran-3,4,5-triol,
<105>(2R,3,,4S,5S)-2-(2-fluoro-4-(5-methylbenzo[d]oxazol-2-yl)phenoxy)tetrahydro-*2H-*pyran-3,4,5-triol,
<106>(2S,3R,4S,5S)-2-(3-(5-methoxybenzo[d]oxazol-2-yl)phenoxy)tetrahydro-*2H*-pyran-3,4,5-triol,
<107>(2R,3R,4S,5S)-2-(3-(5-methoxybenzo[d]oxazol-2-yl)phenoxy)tetrahydro-*2H*-pyran-3,4,5-triol,
<108>(2S,3R,4S,5S)-2-(4-(5-methylbenzo[d]oxazol-2-yl)phenoxy)tetrahydro-*2H*-pyran-3,4,5-triol,
<109>(2R,3R,4S,5S)-2-(4-(5-methylbenzo[d]oxazol-2-yl)phenoxy)tetrahydro-*2H*-pyran-3,4,5-triol,
<110>(2R,3R,4S,5S)-2-(4-phenethylphenoxy)tetrahydro-*2H*-pyran-3,4,5-triol,
<111>(2R,3R,4S,5S)-2-(4-((E)-styryl)phenoxy)tetrahydro-*2*H-pyran-3,4,5-triol,
<112>(2R,3R,4S,5S)-2-(4-(bis(4-methoxyphenyl)methyl)phenoxy)tetrahydro-*2H*-pyran-3,4,5-triol,
<113>(2R,3R,4S,5S)-2-((4'-(4-isopropylbenzyl)-[1,1'-biphenyl]-4-yl)oxy)tetrahydro-*2H*-pyran-3,4,5-triol,
<114>(Furan-2-yl(4-(((2S,3R,4S,5S)-3,4,5-trihydroxytetrahydro-*2H*-pyran-2-yl)oxy)phenyl)methanone,
<115>(Furan-2-yl(4-(((2R,3R,4S,5S)-3,4,5-trihydroxytetrahydro-*2H*-pyran-2-yl)oxy)phenyl)methanone,
<116>(2R,3R,4S,5S)-2-(4-(4-methoxyphenethyl)phenoxy)tetrahydro-*2H*-pyran-3,4,5-triol,
<117>(2R,3R,4S,5S)-2-(4-(4-isopropylphenethyl)phenoxy)tetrahydro*-2H-*pyran-3,4,5-triol,
<118>(2R,3R,4S,5S)-2-(4-((E)-4-methoxystyryl)phenoxy)tetrahydro-*2H*-pyran-3,4,5-triol,
<119>(2R,3R,4S,5S)-2-(4-((E)-4-isopropylstyryl)phenoxy)tetrahydro-*2H*-pyran-3,4,5-triol,
<120>(2R,3R,4S,5S)-2-(4-(benzo[d][1,3]dioxol-5-yl)phenoxy)tetrahydro-*2H*-pyran-3,4,5-triol,
<121>(2R,3R,4S,5S)-2-(4-(2,3-dihydrobenzo[b][1,4]dioxin-6-yl)phenoxy)tetrahydro-*2H-*pyran-3,4,5-triol,
<122>(2R,3R,4S,5S)-2-((4'-chloro-[1,1'-biphenyl]-4-yl)oxy)tetrahydro-*2H*-pyran-3,4,5-triol,
<123>(2R,3R,4S,5S)-2-((4''-isopropyl-[1,1':4',1''-terphenyl-4-yl)oxy)tetrahydro-*2H*-pyran-3,4,5-triol,
<124>(2R,3R,4S,5S)-2-(3-(phenylethynyl)phenoxy)tetrahydro-*2H*-pyran-3,4,5-triol.

The compound according to the present invention may be one selected from the group consisting of < 1 > to < 124 >. Such a compound may be an arabinose derivative compound based on arabinose. Among these, the compound of Formula I according to the present invention may be any one selected from the group consisting of <1> to <57>, <60> to <62>, <67> to <68>, <71> to <76>, and <80> to <124>.

As an example of the present invention, the above-described compound may have brain neuroregenerative and protective functions by targeting GPCR T1R3. As described above, the inventors derived arabinose as a pre-candidate, a single active ingredient among various mixed components of multiple extracts that was confirmed to bind to GPCR T1R3 and be effective against AD dementia, and subsequently developed an arabinose derivative compound that exhibits useful effects as an Alzheimer's treatment. Furthermore, as can be confirmed in the experimental examples described below, it was confirmed that the developed compound induces an increase in the activity of the HRE (hypoxia response element).

As an example of the present invention, the above-described compound may be used for the prevention or treatment of degenerative brain diseases. As can be confirmed in the experimental examples described below, the compound according to the present invention exhibited excellent effects in the Scopolamine-induced cognitive impairment model experiment and the Long Term Potentiation (LTP) experiment.

Another embodiment of the present invention is a pharmaceutical composition for the prevention or treatment of degenerative brain diseases, comprising the above-mentioned compound, its isomer, hydrate, solvate, prodrug, or pharmaceutically acceptable salt. Since the above-mentioned compound has effects of improving cognitive impairment and enhancing long-term memory, it is possible to prevent or treat degenerative brain diseases..

In the present disclosure, degenerative brain diseases are not particularly limited and may include all various diseases known in this field of technology. For example, the degenerative brain disease may be one or more selected from the group consisting of stroke, dementia, Parkinson's disease, vertigo, epilepsy, peripheral neuromuscular disease, brain tumor, brain lesion disorder, cerebral edema, concussion, meningitis, encephalitis, concussion, pituitary tumor, Huntington's disease, tuberculous meningitis, panic disorder, mad cow disease, glioblastosis, agoraphobia, cluster headache syndrome, amnesia, Dandy-Walker syndrome, Tourette syndrome, Wernicke-Kosikoff syndrome, meningioma, and Gerstman syndrome. In addition, the above-mentioned degenerative brain disease may be one or more selected from the group consisting of Alzheimer's disease, Parkinson's disease, Huntington's disease, tauopathy, vascular dementia, acute stroke, trauma, cerebrovascular disease, brain cord trauma, spinal cord trauma, peripheral neuropathy, retinopathy, and glaucoma.

As an example of the present invention, the above-described composition may have brain neuroregeneration and protective functions by targeting GPCR T1R3.

The pharmaceutically acceptable salt of the compound of Formula I of the present invention may be a hydrochloride, sulfate, acetate, formate, succinate, malinate, fumarate, toluenesulfonate, or methanesulfonate of the compound of Formula I.

A pharmaceutical composition for the prevention or treatment of degenerative brain diseases may be provided, comprising the compound of Formula I of the present invention and a pharmaceutically acceptable excipient or carrier.

The above pharmaceutical composition may be prepared according to conventional methods using one or more pharmaceutically acceptable adjuvants or excipients. The adjuvants include, in particular, diluents, sterile aqueous media and various non-toxic organic solvents.

The above pharmaceutical composition may be provided in the form of tablets, pills, granules, powders, aqueous solutions or suspensions, injectable solutions, elixirs, or syrups, and may additionally include sweeteners, flavorings, colorings, or stabilizers to obtain pharmaceutically acceptable formulations.

The selection of the vehicle and the content of the active substance within the vehicle are generally determined by the solubility and chemical properties of the active compound, the specific mode of administration, and the conditions to be observed in pharmaceutical practice. For example, excipients such as lactose, sodium citrate, calcium carbonate, and dicalcium phosphate, combined with lubricants such as magnesium stearate, sodium lauryl sulfate, and talc, and disintegrants such as starch, alginate, and certain complex silicates can be used for the manufacture of tablets.

To manufacture capsules, it is advantageous to use lactose and high molecular weight polyethylene glycol. When an aqueous suspension is used, it may contain an emulsifier or a agent that facilitates suspension, and diluents such as sucrose, ethanol, polyethylene glycol, propylene glycol, glycerol, or chloroform, or mixtures thereof, may be used.

Another embodiment of the present invention is a food composition for preventing degenerative brain diseases comprising the above-mentioned compound, isomer thereof, hydrate, solvate, prodrug, or pharmaceutically acceptable salt. The food composition of the present invention includes various forms such as health functional foods, health foods, or general foods.

According to another aspect of the present invention, As shown in Reaction Scheme 1 below, (i) a step of reacting an arabinose derivative compound represented by the following chemical formula **a** or **a'** with a compound represented by the chemical formula **b** to obtain a compound represented by **c** in the form of a mixture of α and β forms or **c'** in the form of an α-form; (ii) A method for preparing a compound of Formula I, comprising the step of obtaining a target compound of Formula 1 by hydrolyzing the above compound **c** or **c'** using sodium methoxide.

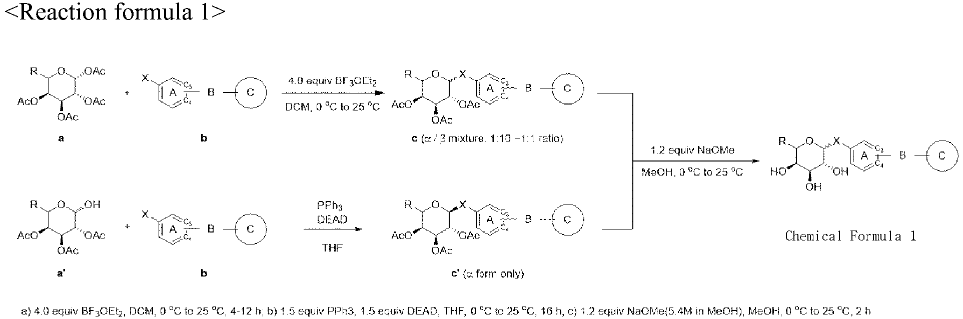

### [Effects of the Invention]

The present invention may provide a compound represented by the above chemical formula I, or an isomer, hydrate, solvate, prodrug, or pharmaceutically acceptable salt thereof.

The present invention may provide a composition containing the above compound, or an isomer, hydrate, solvate, prodrug, or pharmaceutically acceptable salt thereof as an active ingredient.

The present invention can provide a method for manufacturing a compound of the above chemical formula 1.

### [Form for carrying out the invention]

The below, the present invention is to be described more specifically below with reference to the following examples or drawings. However, the description of the following examples or drawings is intended only to specify and explain concrete embodiments of the present invention, and is not intended to limit or restrict the scope of the rights of the present invention to the contents described therein.

In the manufacturing examples and embodiments of the present invention, compounds were purified by the following method and their structures were analyzed.
1. HPLC analysis condition
   - Instrument Name : Waters Arc HPLC Core
   - Column : XBridge C18 5µm 4.6x250mm, 40°C
   - Mobile Phase: 20% → 95% Acetonitrile/Water + 0.1% Formic Acid
   - Analysis Time: 20 min, Flow Rate: 1 mL/min
   - UV Detector: 254 nm
2. LC-MS Analysis Description
   - Instrument Name: Agilent 1260
   - Column: Agilent ZORBAX SB-C18 1.8 um, 50 x 2.1 mm, 45°C
   - Mobile Phase: Acetonitrile/Water + 0.1% Formic Acid
   - Flow Rate: 0.4 mL/min
   - UV Detector: 254 nm and 300 nm
3. NMR
   - Instrument Product Name: Agilent Technologies 400-MR (400 MHz)

Commercially available reagents were used without additional application. In the present invention, room temperature (r.t.) or ambient temperature refers to a temperature of 5 to 40°C, for example, 10 to 30°C or 15 to 27°C, and is not strictly limited to within the above range. A rotary evaporator was used for concentration under reduced pressure or solvent distillation removal.

### Preparation Example 1. Synthesis of Benzyl Phenol Derivative

### Step 1-1. Grignard reaction

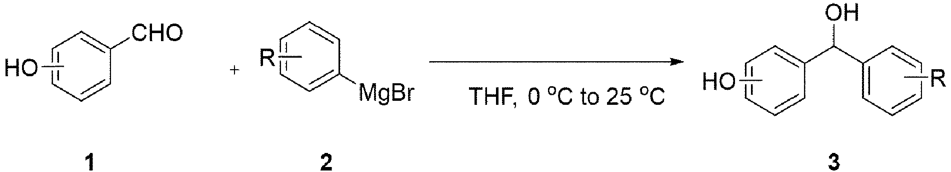

In the presence of argon, Compound **1** (30 mmol) was added to anhydrous THF (150 mL) at 0 °C, followed by the dropwise addition of Compound **2** (75 mmol, 2.5 equivalents), and the mixture was stirred overnight at room temperature. An aqueous ammonium chloride solution was added to the residue, and it was extracted with ethyl acetate. The combined organic layer was washed with a saturated brine solution, dried with anhydrous sodium sulfate, filtered, and concentrated under vacuum. The residue was purified by column chromatography (100-200 silica gel, 100 g, 20% EtOAc in hexane) to obtain Compound **3.**

### Step 1-2. Benzyl alcohol reduction reaction

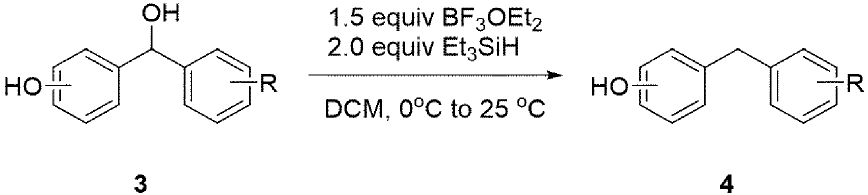

In the presence of argon, After suspending compound 3 (4.0 mmol) in anhydrous DCM (12.0 mL), Triethylsilane (8 mmol, 2.0 equivalents) and BF₃.Et₂ (6 mmol, 1.5 equivalents) were added and the mixture was stirred at 0 °C for 45 minutes. After stirring for an additional 30 minutes at room temperature, an aqueous sodium bicarbonate solution was added to the mixture, and the mixture was extracted with DCM. The combined organic layer was washed with a saturated brine solution, dried with anhydrous magnesium sulfate, filtered, and concentrated under vacuum. The residue was purified with (100-200 silica gel, 100 g, 20-33% EtOAc in hexane) to obtain Compound **4.**

### Step 2-1. Synthesis of 4-((4-(dimethylamino)phenyl)(hydroxy)methyl)phenol

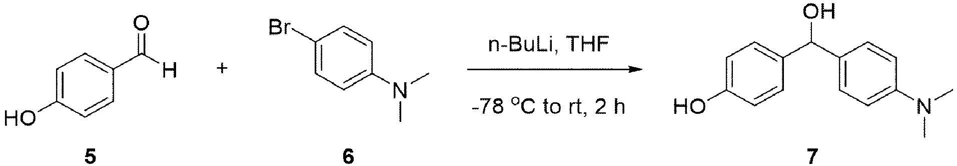

In the presence of argon, 9.6 mL of 2.5 M n-BuLi was added dropwise at -78 °C to a stirred solution of compound **6** (24 mmol, 2.4 equivalents) in 80 mL of anhydrous THF and stirred for 30 minutes. A solution of compound 5 (10 mmol) was added dropwise to 20 mL of anhydrous THF and stirred at room temperature for 2 hours. Once it was confirmed that the reaction was complete, it was extracted with 200 mL of saturated NaHCO3 aqueous solution. Water was removed with Na₂SO₄, filtered, and then concentrated under reduced pressure. The reaction mixture was filtered through a 9% EtOAc solution in hexane to obtain compound **7** (1.87 g, 77%) as a white solid.

### Step 2-2. Synthesis of 4-(4-(dimethylamino)benzyl)phenol

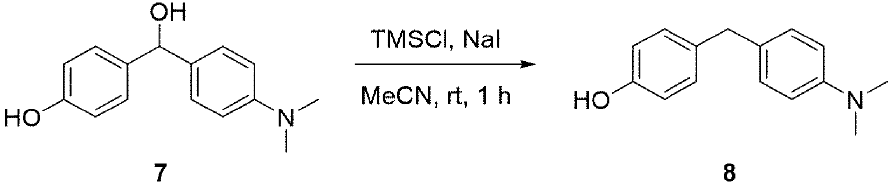

Under an argon atmosphere, TMSCl (39.53 mmol, 7 equivalents) was added dropwise to a stirred solution of Compound **7** (5.65 mmol) and sodium iodide (39.53 mmol, 7 equivalents) in 56 mL of anhydrous acetonitrile, and the mixture was stirred at room temperature. Once the reaction was confirmed to be complete, the mixture was diluted with DCM and washed with 100 mL of saturated aqueous Na₂S₂O₃ solution. It was extracted with 100 mL of EtOAc, water was removed with Na₂SO₄, and the mixture was filtered. The reaction mixture was concentrated under reduced pressure and purified by column chromatography (100-200 silica gel, 100 g, 33% EtOAc in hexane) to obtain Compound **8** (768 mg, 60%) as a white solid.

### Step 3-1. Synthesis of (4-methoxyphenyl)(pyridine-3-yl)methanol

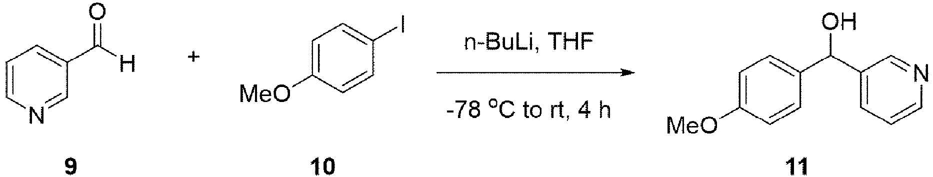

4.8 mL of 2.5 M n-BuLi was added dropwise at -78 °C to a stirred solution of Compound **10** (12 mmol, 2.4 equivalents) in 50 mL of anhydrous THF in the presence of argon, and the mixture was stirred for 30 minutes. After adding Compound **9** (5 mmol), the mixture was stirred at room temperature for 4 hours. Once the reaction was confirmed to be complete, 50 mL of a saturated aqueous NaHCO3 solution was added, and the mixture was extracted with 100 mL of EtOAc. The water was removed with Na₂SO₄, filtered, and then concentrated under reduced pressure. The reaction mixture was purified by column chromatography (using 100-200 silica gel, 100 g, and a 50% EtOAc solution in hexane) to obtain Compound **11** (689 mg, 64%) as a white solid.

### Step 3-2. Synthesis of 3-(4-methoxybenzyl)pyridine

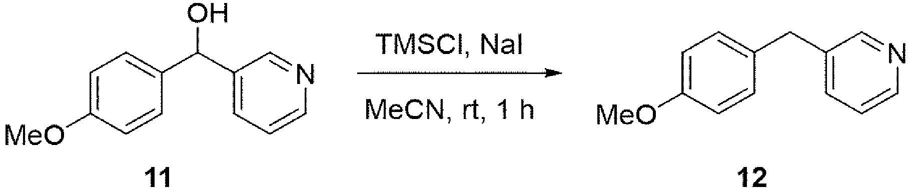

TMSCl (22.1 mmol, 7 equivalents) was added dropwise to a stirred solution of Compound **11** (3.2 mmol) and sodium iodide (22.1 mmol, 7 equivalents) in 32 mL of anhydrous acetonitrile in the presence of argon, and the mixture was stirred at room temperature. Once the reaction was confirmed to be complete, the solution was diluted with DCM and washed with 100 mL of saturated aqueous Na₂S₂O₃ solution. The solution was extracted with 100 mL of EtOAc, water was removed with Na₂SO₄, and the solution was filtered. Compound **12** (383 mg, 61%) was obtained as a white solid by purification by column chromatography (100-200 silica gel, 100 g, 50% EtOAc in hexane).

### Step 3-3. Synthesis of 4-(pyridine-3-ylmethyl)phenol

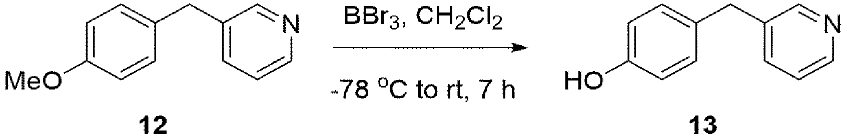

1 M BBr₃ (2.043 mmol, 1.1 equivalents) was added dropwise at -78 °C to a stirred solution of compound **12** (1.857 mmol) in anhydrous DCM under an argon atmosphere and stirred at room temperature for 7 hours. Once the reaction was confirmed to be complete, 3.7 mL of saturated aqueous NaHCO₃ solution was added and extracted with DCM (7.4 mL × 2). The water was removed with Na₂SO₄, filtered, and then concentrated under reduced pressure. The reaction mixture was purified by column chromatography (100-200 silica gel, 100 g, 33% EtOAc in hexane) to obtain compound **13** (250 mg, 73%) as a white solid.

### Step 4-1. Synthesis of 3-chloro-4-methoxybenzaldehyde

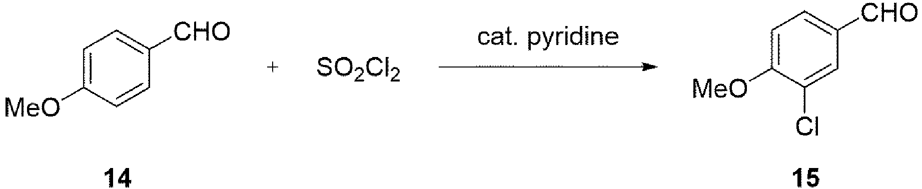

Under an argon atmosphere, sulferyl chloride (17.6 mmol, 1.2 equivalents) was added dropwise to a mixture of 4-methoxy-benzaldehyde **14** (14.7 mmol) and pyridine (30 µL, catalytic) over 30 minutes while maintaining the internal reaction temperature between 25 and 30 °C. Vigorous gas generation occurred during this process. The reaction mixture was stirred at room temperature for 30 minutes, then heated to 70 °C and stirred for 4 hours. The reaction mixture was concentrated under reduced pressure to remove excess reactants and diluted with 50 ml of isopropyl ether. Subsequently, 500 ml of hexane was added while vigorously stirring, resulting in the formation of a precipitate. The solid was washed with hexane, filtered, and vacuum-dried to obtain Compound **15** (2.1 g, 83%).

### Step 4-2. Synthesis of (3-chloro-4-methoxyphenyl)(4-isopropylphenyl)methanol

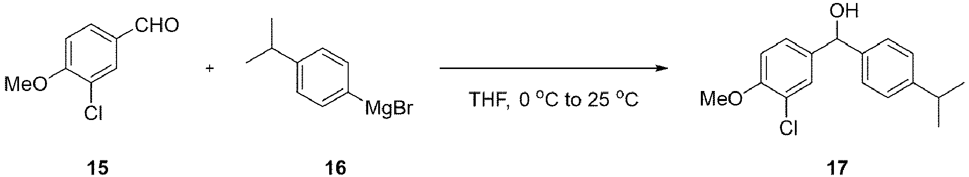

Compound **15** (10 mmol) was added to anhydrous THF (50 mL) under an argon atmosphere at 0°C, followed by the addition of compound **16** (25 mmol, 2.5 equivalents) dropwise and stirring overnight at room temperature. An aqueous ammonium chloride solution was added to the residue and extracted with ethyl acetate. The combined organic layer was washed with saturated brine, dried with anhydrous sodium sulfate, filtered, and concentrated under reduced pressure. The residue was purified by column chromatography (100-200 silica gel, 100 g, 20% EtOAc in hexane) to obtain compound **17** (2.3 g, 79%).

### Step 4-3 Synthesis of 4-(4-(dimethylamino)benzyl)phenol

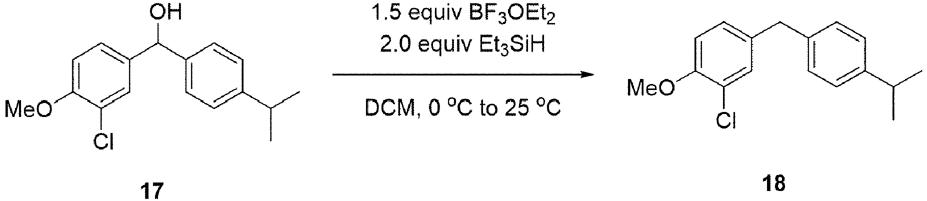

In the presence of argon, Compound **3** (4.0 mmol) was added to anhydrous DCM (12.0 mL) and to the stirred solution triethylsilane (8 mmol, 2.0 equiv.) and BF₃.Et₂ (6 mmol, 1.5 equiv.) were added and the mixture was stirred at 0°C for 45 minutes. The mixture is stirred at room temperature for 30 minutes, and then sodium carbonate aqueous solution is added to the mixture and extracted with DCM. The combined organic layer is washed with saturated brine solution, dried over anhydrous magnesium sulfate, filtered and concentrated under vacuum. The residue was purified (100-200 silica gel, 100 g, 20-33% EtOAc in hexane) to obtain compound **18** (790 mg, 72%).

### Step 4-4. Synthesis of 2-chloro-4-(4-isopropylbenzyl) phenol

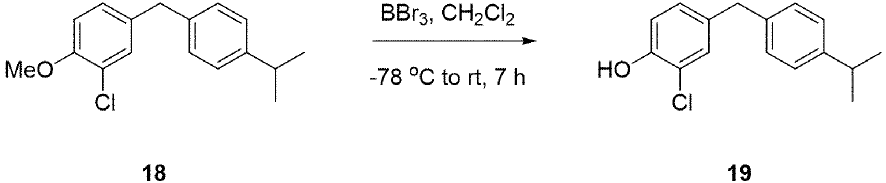

To a stirred solution of compound **18** (2.88 mmol) in anhydrous DCM under an argon atmosphere, 1 M BBr₃ (3.17 mmol, 1.1 equiv.) was added dropwise at -78°C and stirred at room temperature for 7 hours. After the completion of the reaction, 3.7 mL of saturated NHCO3 aqueous solution was added and extracted with DCM (15 mL × 2). Na₂SO₄, filtered, and concentrated under reduced pressure. The reaction mixture was purified by column chromatography (100-200 silica gel, 100 g, 33% etOAC in hexane) to afford Compound **19** (571 mg, 76%) as a white solid phase.

### Step 5. Asymmetric alkylation reaction of bisphenol compounds having substituents

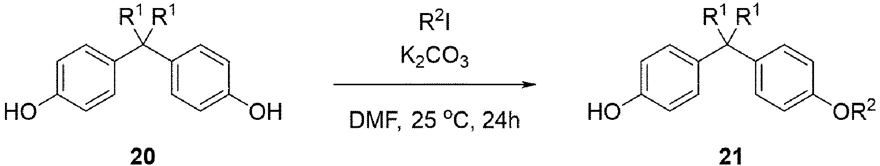

Bisphenol compound **20** (43 mmol) and potassium carbonate (65 mmol, 1.5 equivalent) were added to DMF (20 ml) and mixed, and then iodoalkyl (39 mmol, 0.9 equivalent) was added thereto, followed by reaction at 25°C for 12 hours. after the completion of the reaction, potassium carbonate was filtered and removed, and the filtrate was extracted with EtOAC, and then moisture was removed with anhydrous magnesium sulfate and filtered. The reaction mixture was concentrated under reduced pressure and purified by column chromatography (100-200 silica gel, 100 g, 20% etOAC in hexane) to prepare chemical **21.**

### Step 6-1. Synthesis of Iodobenzyl alcohol derivatives

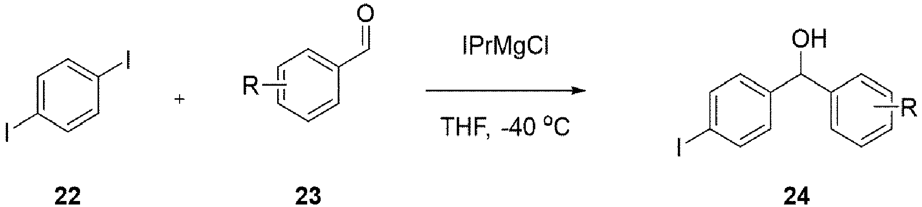

Compound **22** (30 mmol) was added in anhydrous THF (80 mL) at -40°C in the presence of argon, and compound isopropylmagnesium chloride (33 mmol, 1.1 eq) was added dropwise thereto. **23** (33 mmol, 1.1 eq) was added dropwise after 30 minutes. The ammonium chloride aqueous solution is added to the residue and extracted with ethyl acetate. The combined organic layer is washed with saturated brine solution and dried with anhydrous sodium sulfate, filtered and concentrated under vacuum. The residue was purified by column chromatography (100-200 silica gel, 100 g, 10% etOAC in hexane) to obtain compound **24**.

### Step 6-2. Iodobenzyl alcohol reduction reaction

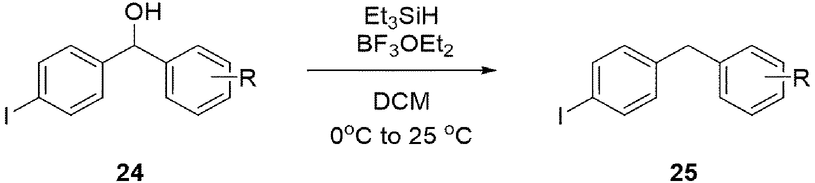

After compound **24 (**10.0 mmol) was suspended in anhydrous DCM (100.0 mL), triethylsilane (15 mmol, 1.5 equiv.) and BF₃·Et₂ (20 mmol, 2.0 equiv.) were sequentially added dropwise at 0° C. and the mixture was stirred for 2 hours. After completion of the reaction, a sodium carbonate aqueous solution was added to the mixture and extracted with DCM. The combined organic layer is washed with saturated brine solution, dried over anhydrous magnesium sulfate, filtered and concentrated under vacuum. The residue is purified with 100-200 silica gel, 100 g of hexane, and 10% of hexane to obtain compound **25**.

### Step 6-3. Benzylbenzenethiol synthesis

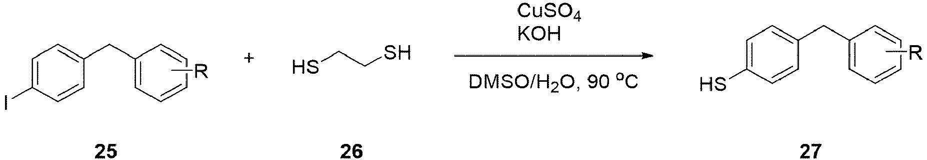

In the presence of argon, Compound **25** (9.4 mmol) was suspended in anhydrous DMSO (19 mL) in the presence of argon, CuSO₄ (0.47 mmol, 5 mol %) and KOH (47 mmol, 5.0 eq) were sequentially added thereto, and **26** was slowly added dropwise to the mixture, followed by stirring for 20 hours. After the reaction was completed, the mixture was cooled at room temperature, neutralized with a 5% HCl aqueous solution, and then extracted with EtOAc by adding a sodium carbonate aqueous solution. The combined organic layer is washed with saturated brine solution, dried over anhydrous magnesium sulfate, filtered and concentrated under vacuum. residue (100-200 silica gel, 100 g, hexane) to obtain compound **27.**

### Step 7-1. BENZYL DERIVATIVE SYNTHESIS

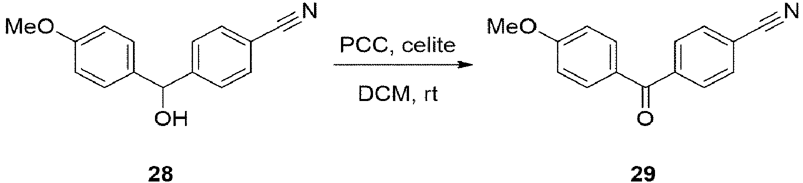

Compound **28** (0.715 mmol) and celite (71.5 g) were suspended in anhydrous DCM in the presence of argon and PCC (2 eq.) was added and the mixture was stirred at room temperature for 3 hours. the mixture is celite-filtered and the filtrate is concentrated under vacuum. The residue was purified by column chromatography (100-200 silica gel, 100 g, 20% etOAC in hexane) to obtain compound **29.**

### Step 7-2. Synthesis of 4-(4-hydroxybenzoyl)benzonitrile

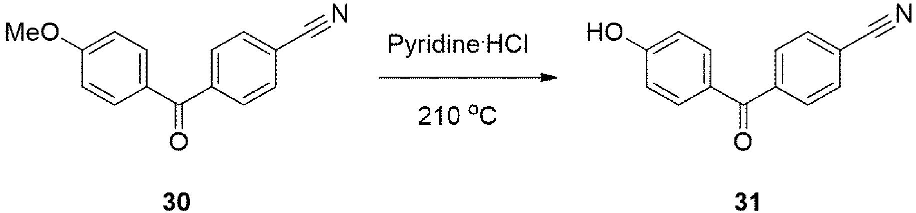

A mixture of compound **30** (0.611 mmol) and PyridineHCl (10 eq) was stirred at 210°C for 4 hours. The mixture is added with water and extracted with ethyl acetate. The combined organic layer is washed with saturated brine solution and dried with anhydrous sodium sulfate, filtered and concentrated under vacuum. The residue was purified by column chromatography (100-200 silica gel, 100 g, hexane: 20-33% etOAC) to obtain compound **31.**

### Step 8-1. amide reaction

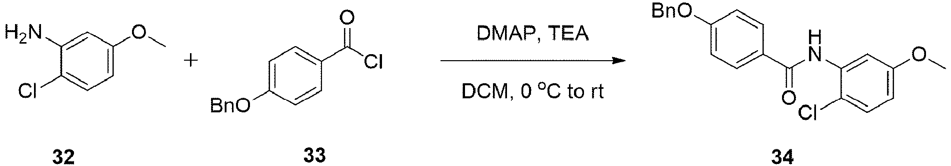

Compound **32** (8.76 mmol), DMAP (2.19 mmol, 0.2 equiv.), TEA (32.86 mmol, 3 equiv.) was added to THF (50 mL) and filled with argon gas. After cooling to 0° C, compound **33** (8.76 mmol, 1 equivalent) was added dropwise thereto, and the mixture was stirred at room temperature overnight. saturated solution of NaHCO3 was added to the mixture, and the mixture was extracted with DCM. The organic layer was washed with saturated brine solution and dried with anhydrous sodium sulfate, filtered and concentrated under reduced pressure. The residue was purified by column chromatography (EA: DCM: hexane = 1: 6: 12) to obtain Compound **34.**

### Step 8-2. cyclization reaction

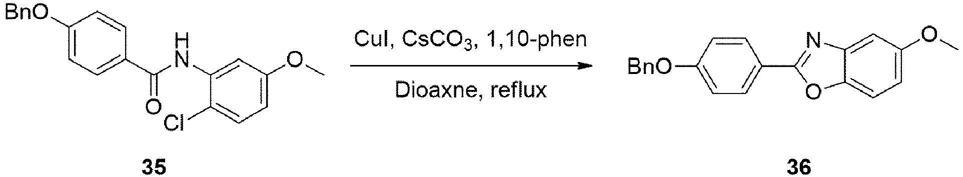

Compound **35** (4.1 mmol), CuI (1.0 mmol, 0.25 equiv.), 1,10-PHEn (3.08 mmol, 1.1 equiv.), and Cs₂CO₃ (12.3 mmol, 3 equiv.) were sequentially added to 1,4-dioxane (45 mL) and filled with argon gas. The mixture was refluxed at 105°C and stirred overnight. The mixture is concentrated under reduced pressure to remove dioXNAe, diluted in EA, and filtered and concentrated on Celite. The mixture was purified by column chromatography (100-200 silica gel, 5 g, EA: DCM = 1: 1) to obtain compound **36.**

### Step 8-3 debenzylation reaction

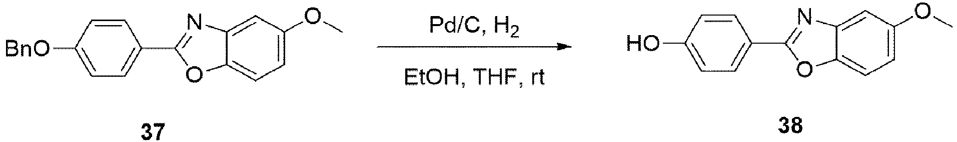

Compound 37 (3.95 mmol) was dissolved in EtOH:THF = 1:2 (80 mL), and then 10% Pd/C (wt 55%, 250 mg) was added. The solution was evacuated with hydrogen gas using a hydrogen balloon. After stirring overnight at room temperature, the solution was filtered through Celite and concentrated under reduced pressure to obtain Compound **38.**

### Step 9. Synthesis of 3-(7-isopropylbenzo[d]oxazole-2-yl)phenol

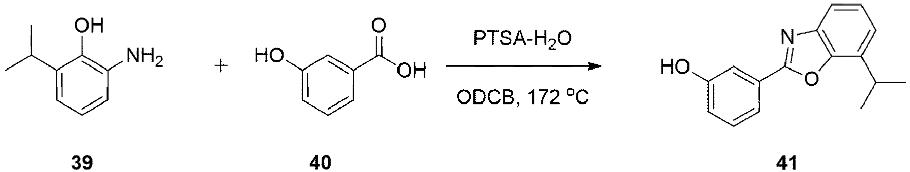

Compounds **39** (5.29 mmol) and **40** (5.29 mmol, 1 equivalent) were added to ODCB (1,2-Dichlorobenzene) (50 mL), and PTSA-H₂O (15.87 mmol, 3 equivalents) was added while stirring at room temperature. The reaction was carried out under reflux at 172 °C for 5 hours and then slowly cooled to 0 °C. A saturated solution of NaHCO3 was added to the mixture, and the mixture was stirred vigorously for 10 minutes. A small amount of hexane was mixed in, and the mixture was filtered. The solid was washed with a large amount of water and hexane and dried to obtain compound **41.**

### Step 10-1. Halogen substitution reaction

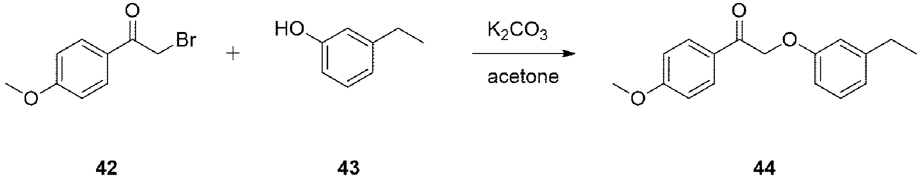

Compound **42** (8.73 mmol), compound **43** (10.48 mmol, 1.2 eq), and K2 CO3 (17.46 mmol, 2 eq) were added to acetone (30 mL), and reflux stirring was continued overnight. The mixture was filtered to separate the solid, concentrated under reduced pressure, and purified by column chromatography (Haxane:DCM=3:2) to obtain Compound **44.**

### Step 10-2. cyclization reaction

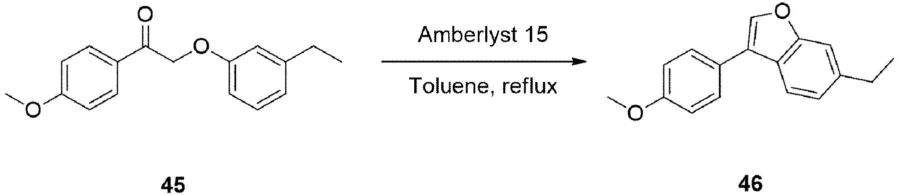

Compound **45** (4.44 mmol) and Amberlyst 15 (200 mg) were added to toluene (20 mL), and reflux stirring was continued for 4 hours. After cooling the mixture, the solid was filtered, concentrated under reduced pressure, and purified by column chromatography (Haxane:DCM=9:1) to obtain Compound **46.**

### Step 10-3. Synthesis of 3-(7-isopropylbenzo[d]oxazole-2-yl)phenol

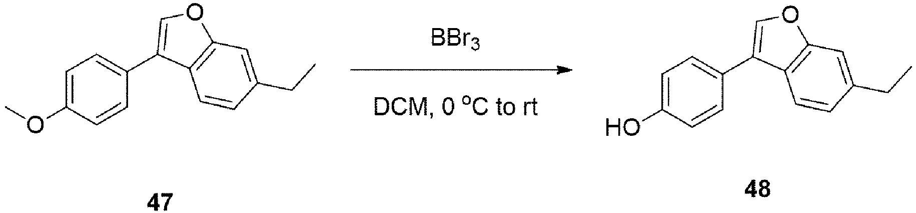

Compound **47** (2.5 mmol) was dissolved in DCM (15 mL) and substituted with argon gas. 1 M BBr₃ (6.2 mmol, 1.5 equiv.) was added dropwise at 0°C and stirred at room temperature for 1 hour. The mixture was cooled to 0° C. MeOH was added in 3 mL, and the mixture was stirred for 10 minutes. The solvent is concentrated under reduced pressure to remove the solvent and diluted in EA. saturated NaHCO₃ aqueous solution and extracted with EA. Brine, dried with anhydrous Na2SO4, filtered, and concentrated under reduced pressure. The mixture is purified by column chromatography (EA: DCM = 15-25%) to obtain compound **48.**

### Step 11-1. Synthesis of 4-(hydroxybis(4-methoxyphenyl)methyl)phenol

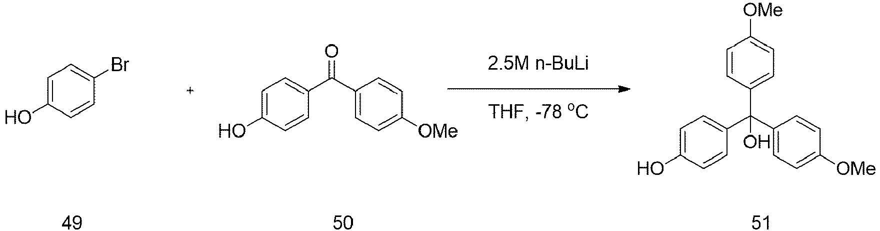

A mixed solution of anhydrous THF (50 ml) and compound **49** (30.0 mmol) was stirred at -78°C in the presence of argon, and 2.5 M n-BuLi (30.0 mmol) was slowly added dropwise thereto, and the mixture was stirred for 30 minutes. Compound **50** (10.0 mmol) was added dropwise, the temperature was slowly increased, and the mixture was stirred at room temperature for 6 hours. When it is confirmed that the reaction was completed, 50 ml of a saturated NaHCO3 aqueous solution was added thereto, and the mixture was extracted with EtOAC. anhydrous magnesium sulfate, filtered, and concentrated under vacuum. The residue was purified by column chromatography (100-200 silica gel, 100 g, EtOAC in hexane) to obtain Compound **51.**

### Step 11-2. Synthesis of 4-((4-methoxycyclohexa-2,4-diene-1-yl)(4-methoxyphenyl)methyl)phenol

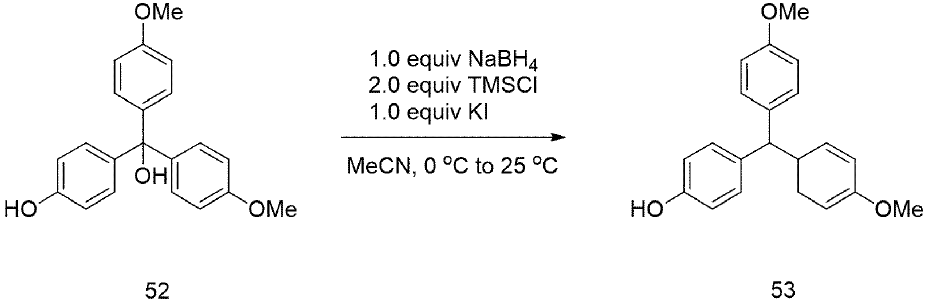

In the presence of argon, a mixed solution of anhydrous MeCN (45 ml) and Compound **52** (4.5 mmol) was stirred at 0 °C while slowly adding sodium borohydride (4.5 mmol) dropwise and stirring for 30 minutes. Subsequently, trimethylsilyl chloride (9.0 mmol) and potassium iodide (4.5 mmol) were added dropwise in sequence at 0 °C, after which the temperature was gradually increased and stirred at room temperature for 3 hours. Once the reaction was confirmed to be complete, 50 ml of a saturated aqueous NaHCO3 solution and 50 ml of a saturated aqueous Na₂S₂O₃ solution were added, and the mixture was extracted with EtOAc. After drying with anhydrous magnesium sulfate, the solution was filtered and concentrated under vacuum. The residue was purified by column chromatography (100-200 silica gel, 100 g, EtOAc 25% in hexane) to obtain Compound **53**.

### Step 12-1. Synthesis of 2-furoyl chloride

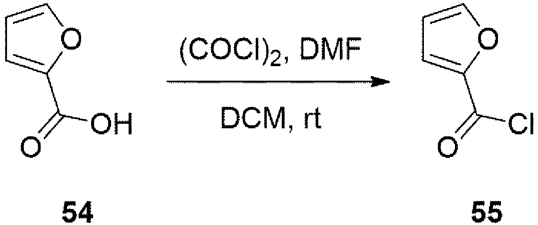

Compound **54** (40 mmol) and DMF (0.01 eq) were added to anhydrous DCM (120 mL) in the presence of argon, followed by dropwise addition of (COCl)₂ (1.2 eq) at 0°C and stirring overnight at room temperature. The residue is concentrated under vacuum to obtain compound **55.**

### Step 12-2. Synthesis of furan-2-yl(4-hydroxyphenyl)methanene

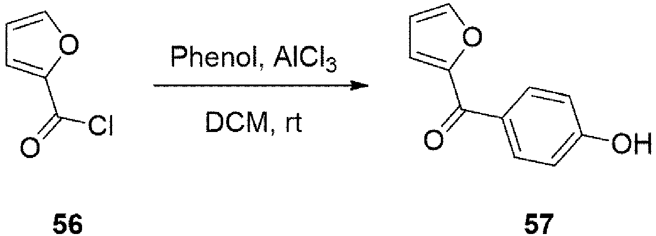

Compound **56** (3.83 mmol) and phenol (1.05 equiv.) were added to anhydrous DCM (4.3 mL) in the presence of argon, then AlCl₃ (1.4 equiv.) was added dropwise at 0°C and stirred overnight at room temperature. the ice water is added to the mixture and the organic layer is washed with 1N HCl and water. the organic layer is dried over anhydrous magnesium sulfate, filtered, and concentrated under vacuum. The residue was purified by column chromatography (100-200 silica gel, 100 g, hexane: 10% etOAC) to obtain compound **57.**

### Step 13. Synthesis of phenoxyphenol derivatives

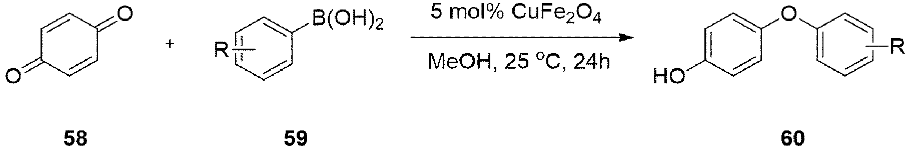

Compound **58** (1.0 mmol), compound **59** (1.2 mmol, 1.2 equivalent), and CuFe₂O₄ (0.05 mmol) were suspended in methanol (2 ml) and stirred for 24 hours. The reaction mixture is concentrated under vacuum and water is added to the residue and extracted with DCM. The combined organic layer is washed with saturated brine solution, dried over anhydrous sodium sulfate, filtered and concentrated under vacuum. The residue was purified by column chromatography (100-200 silica gel, 100 g, 25% etOAC in hexane) to obtain compound **60.**

### Step 14-1. Sonogashira reaction

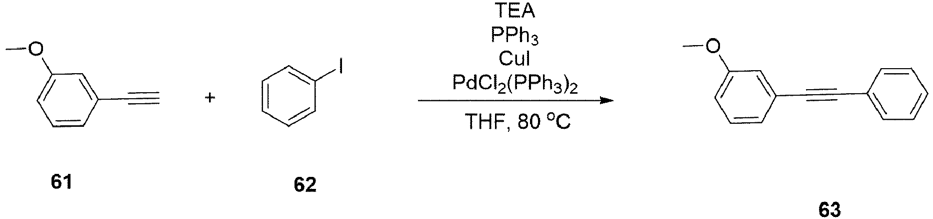

**61** (2 mmol) and **62** (1.5 mmol) were added at room temperature to anhydrous THF (80 mL) together with PPh₃ (0.02 eq), CuI (0.01 eq), PdCl₂(PPh₃)₂ (0.02 eq), and then TEA (80 mL) is added. The mixture was stirred at 80°C for 8 hours. After the reaction was completed, the mixture was cooled to room temperature, and distilled water was added thereto, followed by extraction with EA. The organic layer is washed with saturated brine solution, dried over anhydrous magnesium sulfate, filtered and concentrated under vacuum. The residue was purified by column chromatography (100-200 silica gel, 100 g, 50% EtOAc in hexane) to obtain compound **63.**

### Step 14-2. Demethylation reaction

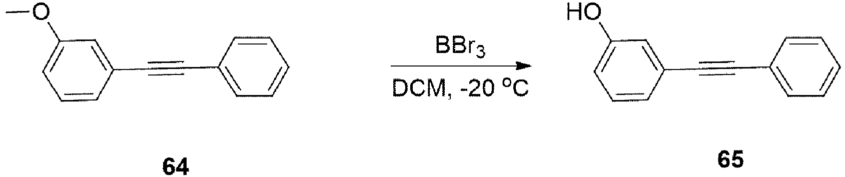

Compound **64** (0.6 mmol) was dissolved in anhydrous dichloromethane (10 mL) in the presence of argon, and the mixture was stirred for 1 hour while slowly adding dropwise biphenyl bromide (BBr₃, 1.2 equivalents) at -20°C. The mixture was then stirred for 12 hours at room temperature. When no reaction occurred, an aqueous sodium carbonate solution was added to the mixture, the pH was adjusted to 7, and the mixture was extracted with ethyl acetate (EA). The organic layer was collected, dried with anhydrous magnesium sulfate, filtered, and concentrated under reduced pressure. The residue was purified by column chromatography (100-200 silica gel, 100 g, 20% sodium ethylenediamine in hexane) to obtain compound **65.**

### Step 15-1. Wittig reaction

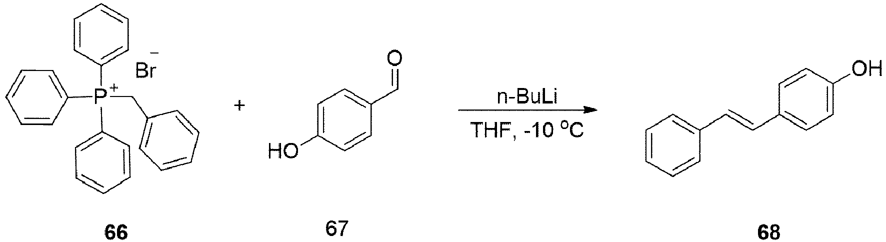

Compound **66** (1 mmol) is dissolved in anhydrous THF (2 mL) in the presence of argon, n-BuLi (2.5 M in hexane, 0.5 eq) is slowly added dropwise thereto at -10° C, and the mixture is stirred for 1 hour. **67** (0.5 equiv.) is dissolved in anhydrous THF (1 mL), slowly added dropwise, and stirred for 1 hour. The mixture is stirred at room temperature for 12 hours and extracted with DCM. The organic layer is collected, dried over anhydrous magnesium sulfate, filtered, and concentrated under vacuum. The residue was purified by column chromatography (100-200 silica gel, 100 g, 20% EtOAC in hexane). The obtained compound is dissolved in hexane (10 mL) and stirred at 70°C for 1 hour. DCM and Na2S2O3 (aq), and the organic layer is dried over anhydrous magnesium sulfate, filtered, and concentrated under vacuum to obtain compound **68.**

### Step 15-2. reduction reaction

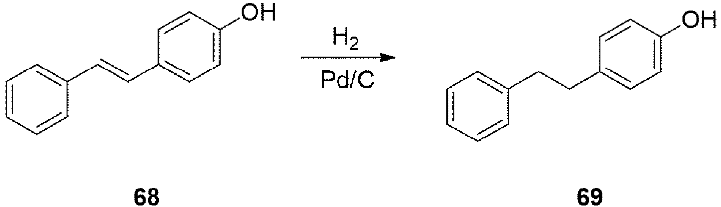

Compound **68** (1 mmol) is dissolved in anhydrous methanol (5 mL) in the presence of hydrogen, and then 10% Pd/C (5 mg) is added and stirred. Celite filter and the filtrate is concentrated under vacuum. The residue was purified by column chromatography (100-200 silica gel, 100 g, 20% etOAC in hexane) to obtain compound **69.**

### Preparation Example 2. Introduction of substituents in the α/β form to arabinose derivatives having protecting groups

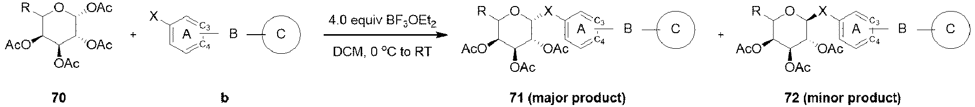

In the presence of argon, Compound **B** (2.4 mmol, 1.5 equiv.) that reacts with Compound **70** (1.6 mmol) was added to 16 ml of anhydrous DCM and mixed, and then boron trifluoride diethyl etherate (6.4 mmol, 4 equiv.) was slowly added while stirring at 0° C. and reacted at room temperature for 4 hours for 12 hours. after the completion of the reaction, 100 ml of a saturated aqueous potassium carbonate solution is added, an organic layer is separated, washed with water, and the organic layer is concentrated under reduced pressure and purified by column chromatography (100-200 silica gel, 100 g, 10-33% etOAC in hexane) to obtain **71** and **72** in the form of compound α/β (10: 1 to 1: 1).

* b was synthesized using the method from Step 1-1 to Step 15-2 of Preparation Example 1.

### Preparation Example 3. Introduction of a substituent in the α/β form to an arabinose derivative having a protecting group

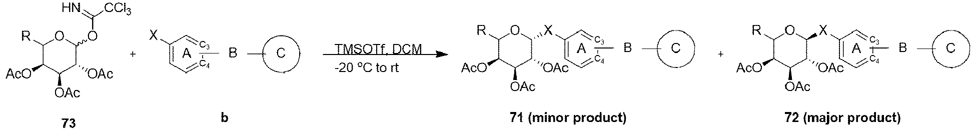

Compound **73** (0.181 mmol), **b** (2 eq.) was added to anhydrous DCM (3.6 mL) in the presence of argon, and then tMSOTf (0.2 eq.) was added dropwise at -20°C and stirred overnight at room temperature. et3N was added to the mixture and concentrated under vacuum. The residue was purified by column chromatography (100-200 silica gel, 100 g, hexane: 20-33% etOAC) to obtain compounds **71** and **72.**

### Preparation Example 4. Introduction of α-type substituents into arabinose derivatives having protecting groups

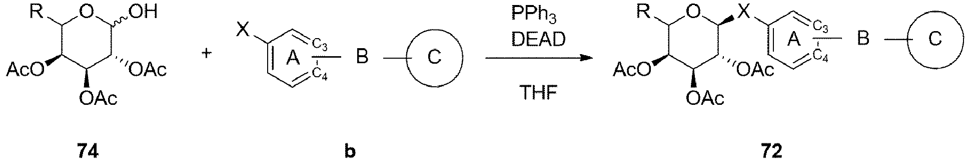

In a stirred solution of **b** (0.783 mmol) and compound **74** (1.174 mmol, 1.5 equivalents) to be used in the reaction in THF (7.8 mL), Triphenylphosphine (1.174 mmol, 1.5 equivalents) and DEAD (1.174 mmol, 1.5 equivalents) were added at 0°C and stirred at room temperature for 16 hours. The reaction mixture was concentrated under reduced pressure and purified by column chromatography (100-200 silica gel, 100 g, 25% EtOAc in Hexane) to obtain compound **72.**

### Preparation Example 5. Hydrolysis reaction by sodium methoxide

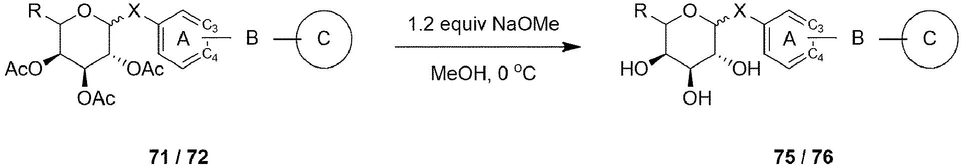

Under argon conditions, Compound **71** or **72** was mixed with 10 ml of methanol, and 1.2 equivalent of sodium methoxide (5.4 M in MeOH) was slowly added thereto while stirring at 0° C. followed by reaction at 25°C for 2 hours. After completion of the reaction, a 10% HCl-methanol solution is slowly added dropwise and neutralized, concentrated under reduced pressure, and purified by column chromatography (100-200 silica gel, 100 g, 5% MeOH in CH₂Cl₂) to obtain a hydrolyzed **75** or **76** compound.

### Preparation Example 6. Synthesis of (2R,3R,4S,SS)-2-(4-(5-methoxy-1H-indol-1-yl)phenoxy)tetrahyd ro-2H-pyran-3,4,5-triol

### step 1-1 Ullmann-type C-N bond formation reaction

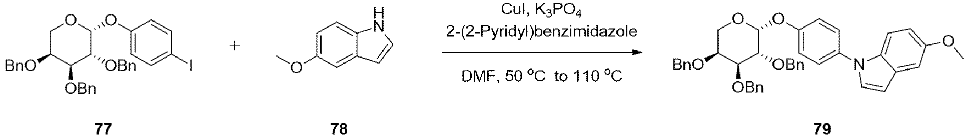

In DMF (10 mL), CuI (0.08 mmol, 0.1 eq), 2-(2-pyridyl) benzimidazole (0.08 mmol, 0.1 eq), and K₃PO₄ (1.6 mmol, 2 eq) were added thereto, and the resulting mixture was stirred at 50°C after argon gas substitution. After 30 minutes, Compound **77** (0.8 mmol) and Compound **78** (1.2 mmol, 1.5 equivalent) were added and stirred at 110°C overnight. After filtering the mixture, the filtrate was extracted with EA by adding NaHCO3 saturated solution. The organic layer is washed with brine and dried with anhydrous sodium sulfate, filtered, and concentrated under reduced pressure. The residue was purified by column chromatography (EA: hexane = 1: 6) to obtain compound **79.**

### step 1-2 Suzuki coupling reaction

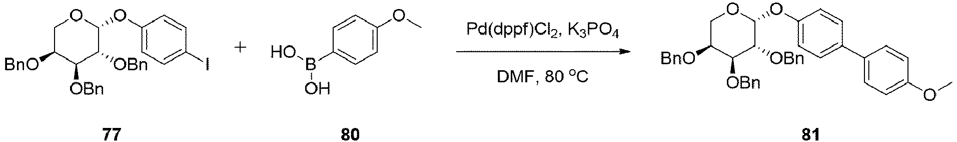

K₃PO₄ (1.93 mmol, 2 eq), Compound **77** (0.96 mmol), Compound **80** (1.16 mmol, 1.5 eq), and Pd(dppf)Cl₂ (0.067 mmol, 0.07 eq) were added to DMF (8 mL), and the mixture was stirred overnight at 80°C after purging with argon gas. Extraction was performed using a saturated aqueous solution of NaHCO3 and EA. The organic layer is washed with brine and dried with anhydrous sodium sulfate, filtered, and concentrated under reduced pressure. The residue is purified by column chromatography (EA: DCM = 20-30%) to obtain compound **81.**

### step 1-3 debenzylation reaction

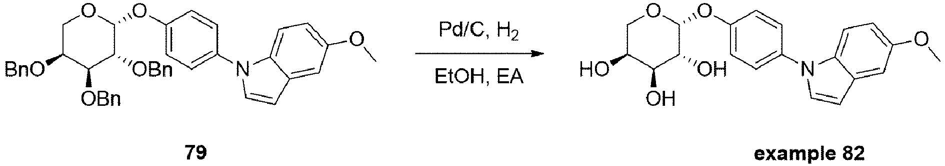

Compound **79** (0.53 mmol) was dissolved in EtOH: EA = 1: 2(15 mL), and 10% Pd/C (wt 55%, 100 mg) was added. The mixture was replaced with hydrogen gas using a hydrogen balloon. After stirring overnight at room temperature and filtering, the solution was concentrated under Celite and purified by column chromatography (MeOH:DCM = 5%) to obtain Example **82.**

### Preparation Example 7. Introduction of a substituent to the hydroxyl group at the 2nd carbon of compound 35

### step 1-1 Introduction of acetal protecting groups at positions 3 and 4 hydroxyl groups

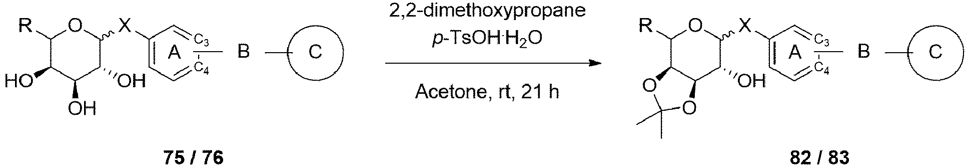

2,2-dimethoxypropane (2 mmol) and para-toluenesulfonic acid hydrate (0.01 mmol) were added to a stirring solution of Compound **75** or **76** in 5 mL of acetone and stirred at room temperature for 21 hours. Triethylamine (0.01 mmol) was added to the reaction mixture and stirred for 30 minutes.
Upon completion of the reaction, the reaction mixture was concentrated under reduced pressure and purified by column chromatography (100-200 silica gel, 20 g, hexane: 20% etOAC) to obtain compound **82** or **83** as a white solid.

### step 1-2 Alkylation reaction of the 2nd hydroxyl group

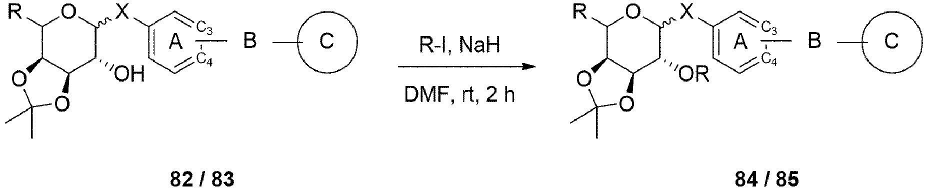

Sodium hydride (0.932 mmol, 2 equivalents) was added to a solution of compound **82** or **83** (0.466 mmol) dissolved in 4.7 mL of anhydrous DMF under an argon atmosphere at 0°C while stirring, and the mixture was stirred for 1 hour. An alkyl halide (0.932 mmol, 2 equivalents) was added dropwise, and the mixture was stirred at room temperature for 2 hours. Upon completion of the reaction, 1.8 mL of water was added to the reaction mixture at 0°C. The mixture was extracted with DCM (3.6 mL × 3) and washed with 3.6 mL of brine. Water was removed with Na₂SO₄, filtered, and concentrated under reduced pressure. The reaction mixture was purified by column chromatography (100-200 silica gel, 20 g, 20% EtOAc in hexane) to obtain compound **84** or **85** as a colorless liquid.

### step 1-3 Deacetalization reaction

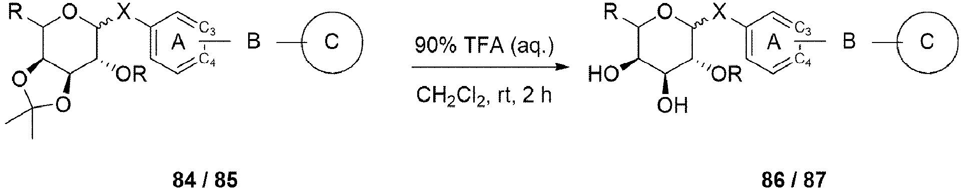

90% TFA (3.959 mmol, 10 equivalents) was added to a solution in which compound **84** or **85** (0.440 mmol) was dissolved in 4.4 mL of DCM, and the mixture was stirred at room temperature for 2 hours. After confirming that the reaction was complete, an aqueous solution of saturated sodium bicarbonate (4.0 mL) was added. DCM (3.4 mL × 2) was extracted and washed with 3.4 mL of brine. Water was removed with Na₂SO₄, filtered, and concentrated under reduced pressure. The reaction mixture was purified by column chromatography (100-200 silica gel, 20 g, 50% EtOAC in hexane) to obtain colorless liquid compound **86** or **87**.

### Preparation Example 8. Introduction of a substituent to the hydroxyl group at the 4th carbon of compound number

### step 1-1 Introduction of 3,4-protectors

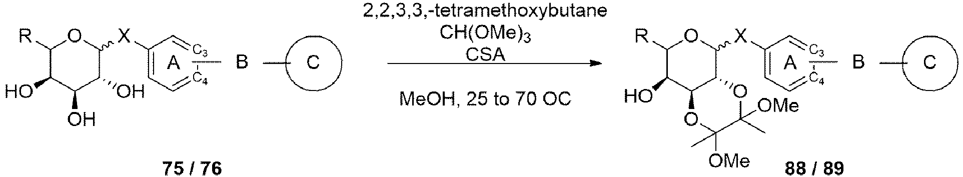

In an argon atmosphere, Compound **75** or **76** (0.5 mmol), 2,2,3,3,-tetramethoxybutane (1.3 eq), CH(OMe)3 (4 eq), and CSA (10 mol %) in MeOH (6 mL) were added and stirred at 70° C. When it was confirmed that the reaction was completed, the solvent was concentrated under reduced pressure. The reaction mixture was purified by column chromatography (100-200 silica gel, 100 g, 5% MeOH in DCM to obtain 88 or **89** compounds.

### step 1-2 Alkylation reaction of the 2nd hydroxyl group

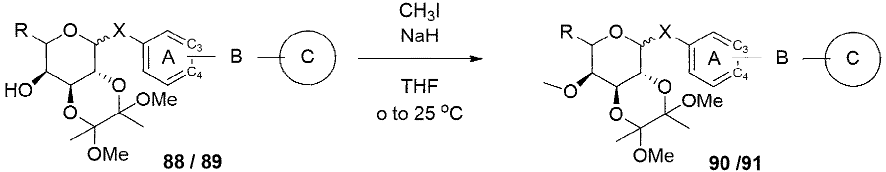

Under an argon atmosphere, 2.5 equivalents of NaH were added at 0 °C to a stirred solution of compound **88** or **89** (0.14 mmol) in 3 mL of anhydrous DCM and stirred for 10 minutes. Subsequently, 2.0 equivalents of CH₃I were added dropwise to the mixture, and the reaction was carried out at room temperature for 3 hours. Once the reaction was confirmed to be complete, the organic layer was washed with a saturated brine solution, dried with anhydrous magnesium sulfate, filtered, and concentrated under vacuum. The residue was purified with (100-200 silica gel, 100 g, 25% EtOAc in hexane) to obtain compound **90** or **91.**

### step 1-3 Compound deprotection reaction

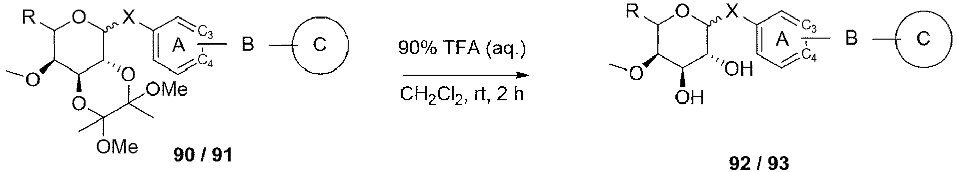

The process was carried out in the same manner as steps 1-3 of Manufacturing 7 above.

### Preparation Example 9. Introduction of a substituent to the hydroxyl group at the 3rd carbon of Compound No. 1

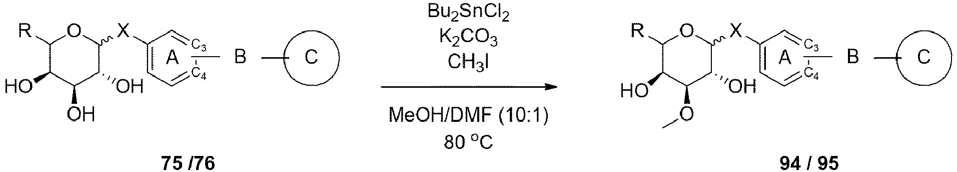

Under an argon atmosphere, Compound **75** or **76** (0.4 mmol) and Bu₂SnCl₂ (10 mol %) in MeOH/DMF (10: 1, 2.2 mL) were added, and then K₂CO₃ (1.5 eq) and CH₃I (2.0 eq.) were added thereto, followed by stirring at 80° C. When it was confirmed that the reaction was completed, the solvent was concentrated under reduced pressure. The reaction mixture is purified by column chromatography (100-200 silica gel, 100 g, 10% MeOH in DCM) to obtain compound **94** or **95.**

### Preparation Example 10. Synthesis of rabinose derivatives

### step 1-1 Synthesis of dihydropyran

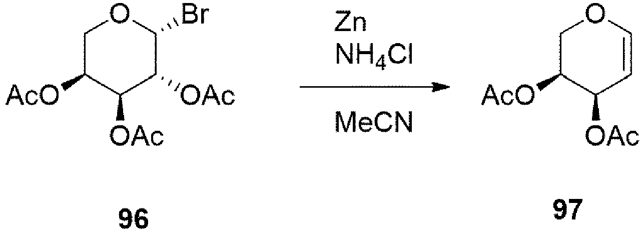

Zinc powder (102.9 mmol) and ammonium chloride (102.9 mmol) were sequentially added dropwise to a **96** (14.7 mmol) stirred solution in anhydrous MeCN (150 ml) in the presence of argon, and the mixture was stirred at room temperature for 2 hours. When it is confirmed that the reaction is completed, the inorganic salt, which is the reaction by-product, and the extra zinc powder were filtered and concentrated under vacuum. The residue was purified by column chromatography (100-200 silica gel, 100 g, 33% etOAC in hexane) to obtain compound **97.**

### step 1-2 Fluorinated synthesis of 2-OH

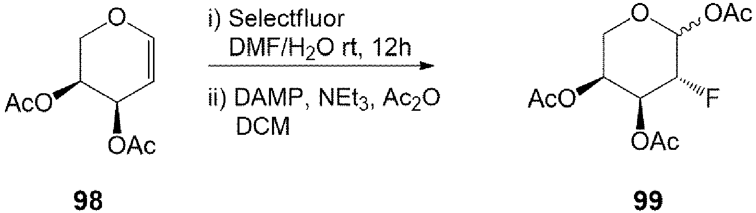

Compound **98** (0.7 mmol) was dissolved in DMF (5 mL) under an argon atmosphere. Selectfluor (1.7 equivalents) and H₂O (5 mL) were added, and the mixture was stirred at room temperature for 12 hours. The organic layer was separated by extraction with ethyl acetate (EA) and water, and the organic layer was collected, dried with anhydrous magnesium sulfate, filtered, and concentrated under reduced pressure. This mixture was redissolved in anhydrous dichloromethane (5 mL) by adding DMAP (0.9 equivalents) and Et₃N (2 equivalents). Ac₂O (1.5 equivalents) was added dropwise at 0 °C, and the mixture was stirred at room temperature. The reaction mixture was dissolved in EA and extracted by washing with 1N HCl. The organic layer was dried with anhydrous magnesium sulfate, filtered, and concentrated under reduced pressure. The residue was purified by column chromatography (100-200 silica gel, 100 g, 30% EtOAc in hexane) to obtain compound **99.**

### step 1-3 Synthesis of 2-Nitroxy Derivatives

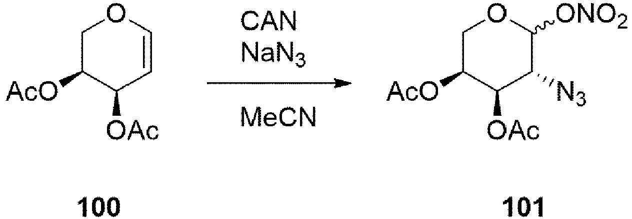

Compound **100** (18.2 mmol) was added to anhydrous MeCN (70 ml) in the presence of argon at -10 °C, then cerium ammonium nitrate (55 mmol) and sodium azide (36.4 mmol) were slowly added dropwise and stirred at -10 °C for 8 hours. Once it was confirmed that the reaction was complete, the solution was diluted with low-temperature EtOAc (50 ml) and distilled water (50 ml), filtered using Celite, and the filtrate was dried with anhydrous magnesium sulfate, filtered, and concentrated under vacuum. The residue was purified by column chromatography (100-200 silica gel, 100 g, 40% EtOAc in hexane) to obtain compound **101.**

### step 1-4 Synthesis of 2-acetyl derivatives

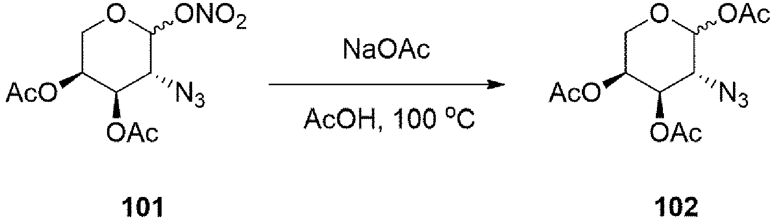

Compound **101** (4.5 mmol) and sodium acetate (8 mmol) were suspended in acetic acid (10 ml) and stirred at 100 °C for 1 hour. Once the reaction was confirmed to be complete, the mixture was diluted with DCM, washed with distilled water, an aqueous solution of saturated sodium bicarbonate, and a saturated brine solution, and the organic layer was extracted with DCM. After drying with anhydrous magnesium sulfate, the mixture was filtered and concentrated under vacuum. The residue was purified by column chromatography (100-200 silica gel, 100 g, EtOAc 40% in hexane) to obtain Compound **102.**

### Example 1. Preparation of (2R,3R,4S,5S)-2-(4-benzylphenoxy)tetrahydro-2H-pyran-3,4,5-triol

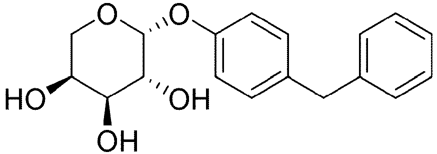

The above compounds were synthesized through Preparation Examples 2 and 3, and the target compounds were obtained by hydrolyzing according to Preparation Example 5 after introducing substituents in the α/β form.

¹H NMR (400 MHz, DMSO-d6) δ 7.27 (t, *J =* 7.4 Hz, 2H), 7.23 - 7.15 (m, 3H), 7.13 (d, *J =* 8.4 Hz, 2H), 6.95 (d, *J* = 8.5 Hz, 2H), 5.38 (s, 1H), 4.96 (s, 1H), 4.84 (s, 1H), 4.72 (s, 1H), 3.87 (s, 2H), 3.76 (s, 3H), 3.67 (d, *J =* 11.9 Hz, 1H), 3.48 (d, *J =* 10.8 Hz, 1H).

### Example 2. Preparation of (2R,3R,4S,5S)-2-(4-(4-methylbenzyl)phenoxy) tetrahydro-2H-pyran-3,4,5-triol

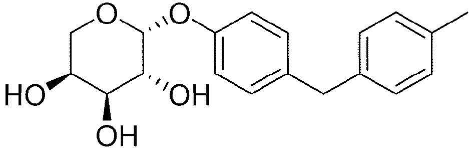

In Example 2, the target compound could be obtained using the same method as in Example 1.

¹H NMR (400 MHz, CD₃OD) δ 7.04 (dt, *J =* 21.1, 8.7 Hz, 8H), 5.46 (d, *J =* 3.2 Hz, 1H), 3.99 - 3.87 (m, 4H), 3.84 (s, 2H), 3.63 (d, *J =* 10.4 Hz, 1H), 2.27 (s, 3H).

### Example 3. Preparation of (2R,3R,4S,5S)-2-(4-(4-methoxybenzyl) phenoxy)tetrahydro-2H-pyran-3,4,5-triol

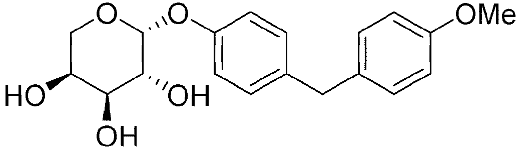

In Example 3, the target compound could be obtained using the same method as in Example 1.

¹H NMR (400 MHz, DMSO-d6) δ 7.11 (dd, *J* = 8.4, 3.4 Hz, 4H), 6.94 (d, *J* = 8.5 Hz, 2H), 6.83 (d, *J =* 8.5 Hz, 2H), 5.37 (s, 1H), 4.89 (d, *J =* 5.3 Hz, 1H), 4.71 (d, *J =* 4.2 Hz, 1H), 4.63 (d, *J* = 3.2 Hz, 1H), 3.80 (s, 2H), 3.75 (s, 2H), 3.70 (s, 3H), 3.67 (d, *J =* 12.2 Hz, 1H), 3.47 (d, *J* = 11.9 Hz, 1H).

### Example 4. Preparation of (2S,3R,4S,5S)-2-(4-(4-methoxybenzyl)phenoxy) tetrahydro-2H-pyran-3,4-diol

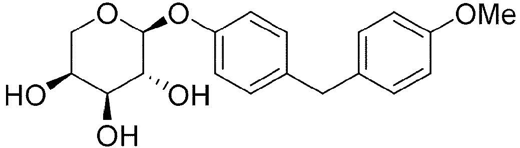

In the above Example 4, a target compound was obtained by introducing a substituent in the β form of peracetyl-substituted arabinose and compound b (synthesized using Step 1 of Preparation Example 1) as Preparation Example 4 and then hydrolyzing it according to Preparation Example 5.

¹H NMR (400 MHz, DMSO-d6) δ 7.10 (d, *J =* 7.2 Hz, 4H), 6.91 (d, *J =* 8.2 Hz, 2H), 6.83 (d, *J* = 8.2 Hz, 2H), 5.18 (d, *J* = 4.9 Hz, 1H), 4.81 (d, *J =* 5.5 Hz, 1H), 4.77 (d, *J =* 6.9 Hz, 1H), 4.63 (d, *J* = 4.0 Hz, 1H), 3.80 (s, 2H), 3.71 - 3.67 (m, 4H), 3.54 (d, *J =* 11.5 Hz, 1H).

### Example 5. Preparation of (3S,4S,5R,6R)-5-methoxy-6-(4-(4-methoxybenzyl) phenoxy)tetrahydro-2H-pyran-3,4-diol

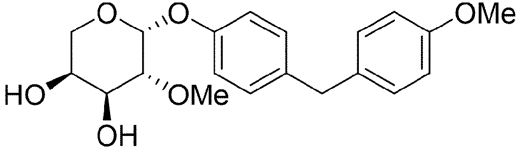

In the above Example 5, a compound having peracetyl-substituted arabinose and an alcohol substituent was prepared as Preparation Example 2, and after introducing the substituent in the α/β form, the target compound was obtained through steps 1-1 to 1-3 of Preparation Example 7.

¹H NMR (400 MHz, CD₃OD) δ 7.10 (dd, *J =* 8.5, 3.7 Hz, 4H), 7.02 (d, *J =* 8.6 Hz, 2H), 6.83 (d, *J* = 8.5 Hz, 2H), 5.69 (d, *J* = 3.2 Hz, 1H), 4.18 (d, *J* = 9.6 Hz, 1H), 4.07 (s, 1H), 3.93 - 3.86 (m, 3H), 3.81 - 3.75 (m, 4H), 3.69 (dd, *J =* 9.7, 3.3 Hz, 1H), 3.49 (s, 3H), 2.68 (s, 1H), 2.60 (s, 1H).

### Example 6. Preparation of (2R,3S,4R,5S,6S)-2-(4-(4-methoxybenzyl) phenoxy)-6-methyltetrahydro-2H-pyran-3,4,5-triol

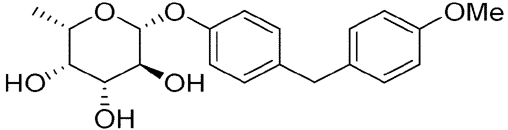

Example 6 was able to obtain the target compound using the same method as Example 1.

¹H NMR (400 MHz, CD₃OD) δ 7.08 (dd, *J* = 8.6, 3.4 Hz, 4H), 7.01 (d, *J* = 8.6 Hz, 2H), 6.81 (d, *J =* 8.6 Hz, 2H), 5.40 (d, *J* = 3.5 Hz, 1H), 3.96 - 3.91 (m, 1H), 3.87 (dd, *J =* 10.3, 3.8 Hz, 1H), 3.83 (s, 2H), 3.75 (s, 3H), 3.72 (s, 1H), 1.17 (d, *J* = 6.6 Hz, 3H).

### Example 7. (Preparation of (2R,3R,4S,5S)-2-(4-(4-isopropylbenzyl) phenoxy)tetrahydro-2H-pyran-3,4,5-triol

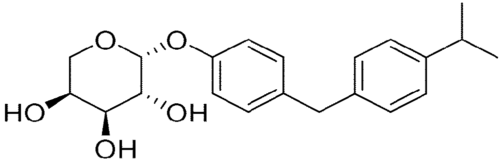

Example 7 was able to obtain the target compound using the same method as Example 1.

¹H NMR (400 MHz, DMSO-d6) δ 7.12 (d, *J* = 3.3 Hz, 6H), 6.94 (d, *J =* 8.6 Hz, 2H), 5.37 (s, 1H), 4.90 (d, *J* = 5.5 Hz, 1H), 4.73 (d, *J* = 3.8 Hz, 1H), 4.65 (d, *J* = 3.3 Hz, 1H), 3.82 (s, 2H), 3.75 (s, 3H), 3.67 (d, *J =* 11.6 Hz, 1H), 3.47 (d, *J =* 10.9 Hz, 1H), 2.85 - 2.80 (m, 1H), 1.16 (d, *J* = 6.9 Hz, 6H).

### Example 8. Preparation of (2R,3R,4S,5S)-2-(2-chloro-4-(4-isopropylbenzyl)phenoxy)tetrahydro-2H-pyran-3,4,5-triol

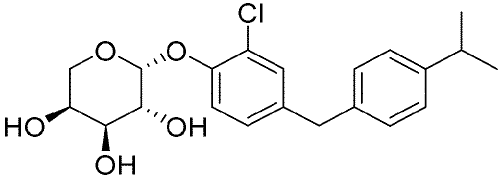

Example 8 was able to obtain the target compound using the same method as Example 1.

¹H NMR (400 MHz, DMSO-d6) δ 7.27 (d, *J =* 1.5 Hz, 1H), 7.20 - 7.11 (m, 6H), 5.58 (d, *J* = 1.6 Hz, 1H), 4.97 (d, *J =* 4.8 Hz, 1H), 4.83 (d, *J =* 4.7 Hz, 1H), 4.67 (d, *J* = 3.4 Hz, 1H), 3.83 (s, 4H), 3.78 (s, 1H), 3.69 (d, *J* = 12.0 Hz, 1H), 3.50 - 3.44 (m, 1H), 2.83 (dt, *J* = 13.7, 6.9 Hz, 1H), 1.17 (s, 3H), 1.16 (s, 3H).

### Example 9. Preparation of (2R,3R,4S,5S)-2-(4-(4-phenoxybenzyl)phenoxy) tetrahydro-2H-pyran-3,4,5-triol

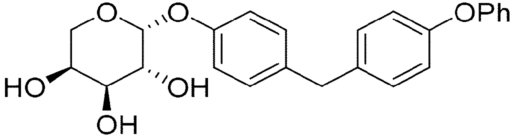

Example 9 was able to obtain the target compound using the same method as Example 1.

¹H NMR (400 MHz, DMSO-d6) δ 7.36 (dd, *J* = 8.3, 7.7 Hz, 2H), 7.22 (d, *J* = 8.4 Hz, 2H), 7.15 (d, *J =* 8.5 Hz, 2H), 7.10 (t, *J =* 7.4 Hz, 1H), 6.94 (dd, *J =* 14.4, 8.2 Hz, 6H), 5.39 (s, 1H), 4.89 (d, *J =* 5.3 Hz, 1H), 4.72 (d, *J* = 4.5 Hz, 1H), 4.64 (d, *J =* 3.3 Hz, 1H), 3.86 (s, 2H), 3.76 (s, 3H), 3.68 (d, *J=* 12.0 Hz, 1H), 3.48 (dd, *J =* 11.9, 1.9 Hz, 1H).

### Example 10. (2R,3R,4S,5S)-2-(4-(4-trifluoromethoxy)benzyl)phenoxy)tetrahydro-2H-pyran-3,4,5-triol

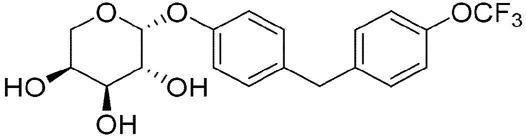

Example 10 was able to obtain the target compound using the same method as Example 1.

¹H NMR (400 MHz, DMSO-d6) δ 7.85 (d, *J =* 8.6 Hz, 2H), 7.76 (d, *J =* 8.7 Hz, 2H), 7.54 (d, *J* = 8.2 Hz, 2H), 7.20 (d, *J =* 8.7 Hz, 2H), 5.62 (s, 1H), 5.05 (d, *J =* 4.4 Hz, 1H), 4.81 (d, *J =* 0.9 Hz, 1H), 4.72 (d, *J =* 3.0 Hz, 1H), 3.82 (s, 2H), 3.67 (d, *J =* 11.7 Hz, 1H), 3.54 (dd, *J =* 11.8, 1.4 Hz, 1H).

**Example 11.** (2R,3R,4S,5S)-2-(4-(4-(methylthio)benzyl)phenoxy)tetrahydro-2*H-*pyran-3,4,5-triol

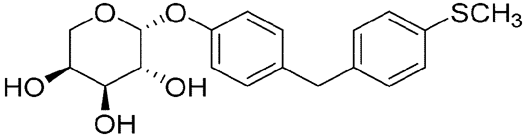

### Example 11 was able to obtain the target compound using the same method as Example 1

¹H NMR (400 MHz, DMSO-d6) δ 7.16 (q, *J =* 8.5 Hz, 4H), 7.12 (d, *J =* 8.4 Hz, 2H), 6.94 (d, *J* = 8.4 Hz, 2H), 5.38 (s, 1H), 4.90 (d, *J* = 3.5 Hz, 1H), 4.73 (s, 1H), 4.65 (s, 1H), 3.82 (s, 2H), 3.75 (s, 3H), 3.67 (d, *J=* 11.9 Hz, 1H), 3.47 (d, *J=* 11.0 Hz, 1H), 2.43 (s, 3H).

### Example 12. (2S,3R,4S,5S)-2-(4-(4-(dimethylamino)benzyl)phenoxy) tetrahydro-2H-pyran 3,4,5-triol

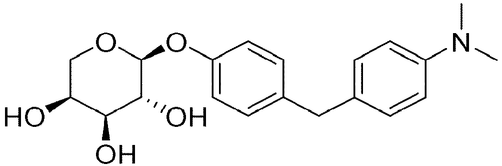

Example 12 was able to obtain the target compound using the same method as Example 4.

¹H NMR (400 MHz, CD₃OD) δ 7.03 (dd, *J* = 24.6, 8.5 Hz, 4H), 6.96 (d, *J* = 8.6 Hz, 2H), 6.72 (d, *J =* 8.6 Hz, 2H), 4.78 (d, *J* = 7.2 Hz, 1H), 3.90 (dd, *J* = 12.4, 2.5 Hz, 1H), 3.85 (s, 1H), 3.77 (d, *J =* 7.3 Hz, 1H), 3.66 (d, *J* = 12.3 Hz, 1H), 3.60 (dd, *J =* 9.1, 3.4 Hz, 1H), 2.86 (s, 6H).

### Example 13. (2R,3R,4S,5S)-2-(4-(4-fluorobenzyl)phenoxy)tetrahydro-2H-pyran-3,4,5-triol

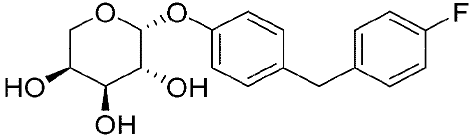

Example 13 was able to obtain the target compound using the same method as Example 1.

¹H NMR (400 MHz, CD₃OD) δ 7.16 (dd, *J* = 8.2, 5.6 Hz, 2H), 7.10 (d, *J* = 8.5 Hz, 2H), 7.02 (d, *J =* 8.6 Hz, 2H), 6.97 (t, *J =* 8.8 Hz, 2H), 5.46 (d, *J =* 3.1 Hz, 1H), 3.99 - 3.87 (m, 6H), 3.63 (d, *J =* 11.0 Hz, 1H).

### Example 14. (2R,3R,4S,5S)-2-(4-(4-chlorobenzyl)phenoxy)tetrahydro-2H-pyran-3,4,5-triol

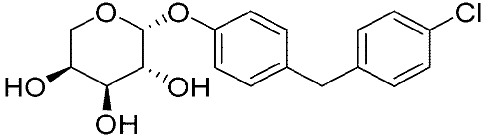

Example 14 was able to obtain the target compound using the same method as Example 1.

¹H NMR (400 MHz, DMSO-d6) δ 7.33 (d, *J =* 8.3 Hz, 2H), 7.23 (d, *J* = 8.3 Hz, 2H), 7.13 (d, *J* = 8.5 Hz, 2H), 6.95 (d, *J =* 8.5 Hz, 2H), 5.38 (s, 1H), 4.89 (d, *J =* 4.6 Hz, 1H), 4.72 (d, *J =* 2.8 Hz, 1H), 4.64 (d, *J =* 2.5 Hz, 1H), 3.87 (s, 2H), 3.76 (s, 3H), 3.67 (d, *J =* 11.9 Hz, 1H), 3.48 (dd, *J =* 11.8, 1.4 Hz, 1H).

### Example 15. (2R,3R,4S,5S)-2-(4-(3-methoxybenzyl)phenoxy)tetrahydro-2H-pyran-3,4,5-triol

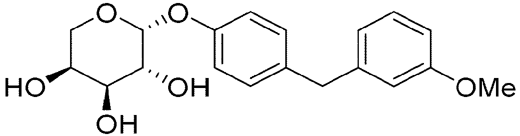

Example 15 was able to obtain the target compound using the same method as Example 1.

¹H NMR (400 MHz, CD₃OD) δ 7.18 - 7.13 (m, 1H), 7.10 (d, *J* = 8.6 Hz, 2H), 7.02 (d, *J* = 8.6 Hz, 2H), 6.77 - 6.67 (m, 3H), 5.46 (d, *J* = 3.2 Hz, 1H), 3.99 - 3.88 (m, 4H), 3.86 (s, 2H), 3.73 (s, 3H), 3.63 (d, *J =* 10.3 Hz, 1H).

### Example 16. (2R,3R,4S,5S)-2-(4-(3,5-bis(trifluoromethyl)benzyl)phenoxy) tetrahydro-2H-pyran-3,4,5-triol

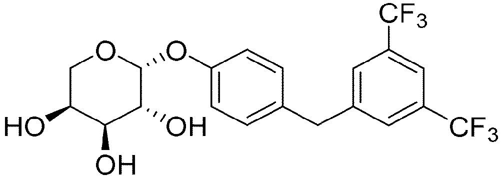

Example 16 was able to obtain the target compound using the same method as Example 1.

¹H NMR (400 MHz, DMSO-d6) δ 7.97 (s, 2H), 7.92 (s, 1H), 7.23 (d, *J =* 8.4 Hz, 2H), 6.99 (d, *J* = 8.5 Hz, 2H), 5.41 (s, 1H), 4.91 (s, 1H), 4.79 - 4.61 (m, 2H), 4.11 (s, 2H), 3.76 (s, 3H), 3.67 (d, *J* = 11.7 Hz, 1H), 3.48 (d, *J* = 11.4 Hz, 1H).

### Example 17. (2R,3R,4S,5S)-2-(4-(3,4-dimethoxybenzyl)phenoxy)tetrahydro-2H-pyran-3,4-triol

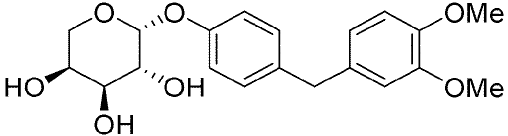

Example 17 was able to obtain the target compound using the same method as Example 1.

¹H NMR (400 MHz, CD₃OD) δ 7.10 (d, *J =* 7.9 Hz, 2H), 7.02 (d, *J =* 7.0 Hz, 2H), 6.84 (d, *J* = 7.8 Hz, 1H), 6.76 (s, 1H), 6.71 (d, *J =* 7.6 Hz, 1H), 5.46 (s, 1H), 3.96 - 3.87 (m, *J =* 17.5, 9.9 Hz, 4H), 3.84 (s, 2H), 3.77 (d, *J* = 10.5 Hz, 6H), 3.63 (d, *J=* 11.9 Hz, 1H).

### Example 18. (2R,3R,4S,5S)-2-(4-(3,4,5-trimethoxybenzyl)phenoxy) tetrahydro-2H-pyran-3,4,5-triol

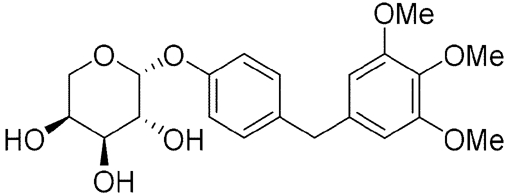

Example 18 was able to obtain the target compound using the same method as Example 1.

*447¹H NMR (400 MHz, CD₃OD) δ 7.13 (d, *J =* 8.4 Hz, 2H), 7.03 (d, *J =* 8.5 Hz, 2H), 6.48 (s, 2H), 5.47 (d, *J =* 2.9 Hz, 1H), 3.96 - 3.88 (m, 4H), 3.85 (s, 2H), 3.77 (s, 6H), 3.72 (s, 3H), 3.63 (d, *J =* 10.9 Hz, 1H).

### Example 19. (2R,3R,4S,5S)-2-(4-(naphthalen-2-ylmethyl)phenoxy) tetrahydro-2H-pyran-3,4,5-triol

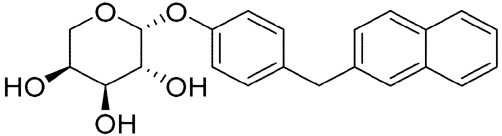

Example 19 was able to obtain the target compound using the same method as Example 1.

¹H NMR (400 MHz, DMSO-d6) δ 7.84 (dd, *J =* 12.7, 7.7 Hz, 3H), 7.72 (s, 1H), 7.46 (dt, *J =* 12.7, 6.2 Hz, 2H), 7.37 (d, *J =* 8.4 Hz, 1H), 7.19 (d, *J* = 8.5 Hz, 2H), 6.96 (d, *J* = 8.4 Hz, 2H), 5.38 (s, 1H), 4.92 (s, 1H), 4.77 (s, 1H), 4.67 (s, 1H), 4.04 (s, 2H), 3.76 (s, 3H), 3.67 (d, *J =* 12.0 Hz, 1H), 3.47 (d, *J =* 9.9 Hz, 1H).

### Example 20. ((2R,3R,4S,5S)-2-(4-(6-methoxynaphthalen-2-yl)methyl) phenoxy)tetrahydro-2H-pyran-3,4,5-triol

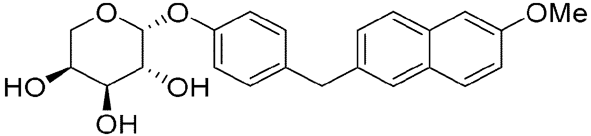

Example 20 was able to obtain the target compound using the same method as Example 1.

¹H NMR (400 MHz, DMSO-d6) δ 7.75 (t, *J =* 7.9 Hz, 2H), 7.65 (s, 1H), 7.32 (d, *J =* 8.6 Hz, 1H), 7.26 (s, 1H), 7.20 (d, *J =* 8.4 Hz, 2H), 7.13 (dd, *J* = 8.9, 2.1 Hz, 1H), 6.97 (d, *J =* 8.4 Hz, 2H), 5.40 (d, *J =* 2.4 Hz, 1H), 4.00 (s, 2H), 3.85 (s, 3H), 3.82 - 3.77 (m, 6H), 3.69 (d, *J =* 11.9 Hz, 1H), 3.49 (d, *J =* 10.6 Hz, 1H).

### Example 21. (2R,3R,4S,5S)-2-(4-(cyclopentylmethyl)phenoxy)tetrahydro-2H-pyran-3,4,5-triol

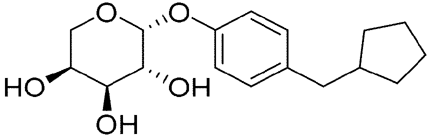

Example 21 was able to obtain the target compound using the same method as Example 1.

¹H NMR (400 MHz, DMSO-d6) δ 7.08 (d, *J =* 8.4 Hz, 2H), 6.92 (d, *J =* 8.5 Hz, 2H), 5.37 (d, *J =* 1.7 Hz, 1H), 4.90 (d, *J* = 4.1 Hz, 1H), 4.73 (s, 1H), 4.65 (s, 1H), 3.76 (s, 3H), 3.69 (d, *J* = 12.0 Hz, 1H), 3.49 (dd, *J =* 11.6, 1.7 Hz, 1H), 2.48 (s, 2H), 2.05 - 1.98 (m, 1H), 1.65 - 1.55 (m, 4H), 1.51 - 1.43 (m, 2H), 1.18 - 1.10 (m, 2H).

### Example 22. (2R,3R,4S,5S)-2-(4-(cyclohexylmethyl)phenoxy)tetrahydro-2H-pyran-3,4,5-triol

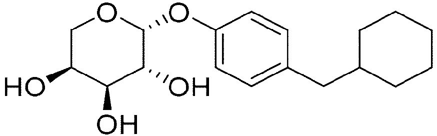

Example 22 was able to obtain the target compound using the same method as Example 1.

¹H NMR (400 MHz, CD₃OD) δ 7.02 (q, *J =* 8.5 Hz, 4H), 5.46 (d, *J =* 3.2 Hz, 1H), 3.99 - 3.90 (m, 4H), 3.64 (d, *J=* 10.6 Hz, 1H), 2.42 (d, *J* = 7.1 Hz, 2H), 1.72 - 1.61 (m, *J* = 12.1 Hz, 5H), 1.50 - 1.44 (m, 1H), 1.25 - 1.16 (m, 3H), 0.99 - 0.87 (m, *J* = 11.5 Hz, 2H).

### Example 23. (2R,3R,4S,5S)-2-(4-(thiophen-3-ylmethyl)phenoxy)tetrahydro-2H-pyran-3,4,5-triol

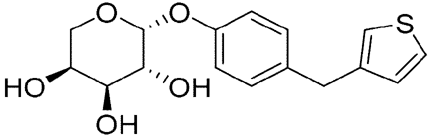

Example 23 was able to obtain the target compound using the same method as Example 1.

¹H NMR (400 MHz, DMSO-d6) δ 7.31 (dd, *J =* 5.1, 1.0 Hz, 1H), 7.17 (d, *J* = 8.4 Hz, 2H), 6.97 (d, *J =* 8.5 Hz, 2H), 6.93 (dd, *J* = 5.0, 3.5 Hz, 1H), 6.87 (d, *J =* 2.3 Hz, 1H), 5.40 (s, 1H), 4.91 (s, 1H), 4.69 (d, *J* = 27.1 Hz, 2H), 4.07 (s, 2H), 3.76 (s, 3H), 3.68 (d, *J* = 11.8 Hz, 1H), 3.48 (dd, *J =* 11.8, 1.3 Hz, 1H).

### Example 24. (2S,3R,4S,5S)-2-(4-(pyridin-3-ylmethyl)phenoxy)tetrahydro-2H-pyran-3,4,5-triol

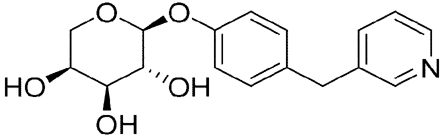

Example 24 was able to obtain the target compound using the same method as Example 4.

¹H NMR (400 MHz, CD₃OD) δ 8.40 (s, 1H), 8.35 (d, *J =* 4.3 Hz, 1H), 7.65 (d, *J =* 7.8 Hz, 1H), 7.34 (dd, *J =* 7.7, 5.0 Hz, 1H), 7.14 (d, *J* = 8.4 Hz, 2H), 7.02 (d, *J* = 8.5 Hz, 2H), 4.83 - 4.81 (d, *J* = 7.1 Hz, 1H), 3.97 (s, 2H), 3.94 - 3.86 (m, *J* = 17.6, 5.2 Hz, 2H), 3.83 - 3.77 (m, 1H), 3.68 (d, *J =* 11.8 Hz, 1H), 3.62 (dd, *J =* 9.0, 3.3 Hz, 1H).

### Example 25. (2R,3R,4S,5S)-2-(3-(4-methoxybenzyl)phenoxy)tetrahydro-2H-pyran-3,4,5-triol

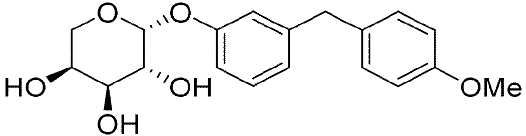

Example 25 was able to obtain the target compound using the same method as Example 1.

¹H NMR (400 MHz, DMSO-d6) δ 7.18 (dd, *J* = 8.8, 7.5 Hz, 1H), 7.13 (d, *J* = 8.6 Hz, 2H), 6.84 (m, 5H), 5.39 (s, 1H), 4.90 (d, *J =* 4.9 Hz, 1H), 4.72 (d, *J =* 3.5 Hz, 1H), 4.64 (d, *J =* 3.3 Hz, 1H), 3.83 (s, 2H), 3.75 (s, 3H), 3.71 (s, 3H), 3.66 (d, *J =* 12.1 Hz, 1H), 3.48 (dd, *J =* 12.0, 1.9 Hz, 1H).

### Example 26. (2R,3R,4S,5S)-2-((4-benzylphenyl)thio)tetrahydro-2H-pyran-3,4,5-thiol

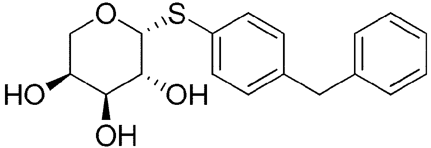

Example 26 was able to obtain the target compound using the same method as Example 1.

¹H NMR (400 MHz, DMSO-d6) δ 7.29 (dd, *J =* 14.1, 7.7 Hz, 4H), 7.20 (dd, *J =* 18.0, 7.6 Hz, 5H), 5.34 (d, *J =* 5.5 Hz, 1H), 5.26 (d, *J =* 3.1 Hz, 1H), 4.88 (d, *J =* 4.5 Hz, 1H), 4.62 (d, *J= 5.2* Hz, 1H), 3.90 (s, 2H), 3.78 (dd, *J =* 22.4, 10.2 Hz, 3H), 3.59 (s, 1H), 3.46 (dd, *J =* 10.8, 6.4 Hz, 1H).

### Example 27. (2R,4S,5S)-2-((4-(4-isopropylbenzyl)phenyl)thio) tetrahydro-2H-pyran-3,4,5-triol

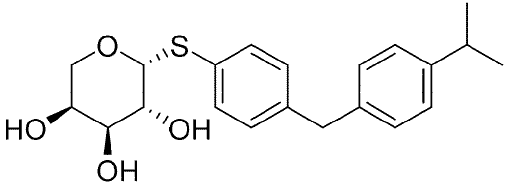

Example 27 was able to obtain the target compound using the same method as Example 1.

¹H NMR (400 MHz, DMSO-d6) δ 7.28 (d, *J =* 7.9 Hz, 2H), 7.17 - 7.07 (m, 6H), 5.31 (d, *J =* 5.5 Hz, 1H), 5.23 (d, *J =* 3.2 Hz, 1H), 4.86 (d, *J= 4.5* Hz, 1H), 4.60 (d, *J =* 5.1 Hz, 1H), 3.85 - 3.70 (m, 5H), 3.57 (s, 1H), 3.44 (dd, *J =* 11.0, 6.4 Hz, 1H), 2.84 - 2.77 (m, 1H), 1.14 (d, *J* = 6.9 Hz, 6H).

### Example 28. (2S,3R,4S,5S)-2-((4-(4-isopropylbenzyl)phenyl)thio) tetrahydro-2H-pyran-3,4,5-triol

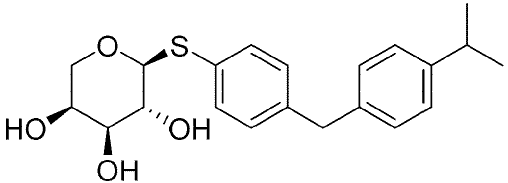

Example 28 was able to obtain the target compound using the same method as Example 1.

¹H NMR (400 MHz, DMSO-d6) δ 7.30 (d, *J =* 8.0 Hz, 2H), 7.15 - 7.09 (m, 6H), 5.18 (d, *J =* 5.2 Hz, 1H), 4.85 (d, *J =* 4.8 Hz, 1H), 4.72 (d, *J =* 6.1 Hz, 1H), 4.58 (d, *J =* 5.0 Hz, 1H), 3.87 - 3.76 (m, 3H), 3.70 - 3.68 (m, 1H), 3.57 (q, *J =* 6.1 Hz, 1H), 3.48 - 3.44 (m, 1H), 3.39 (dd, *J =* 11.4, 2.7 Hz, 1H), 2.84 - 2.77 (m, 1H), 1.14 (d, *J =* 6.9 Hz, 6H).

### Example 29. (2R,3R,4S,5S)-2-((4-(4-methoxybenzyl)phenyl)thio) tetrahydro-2H-pyran-3,4,5-thiol

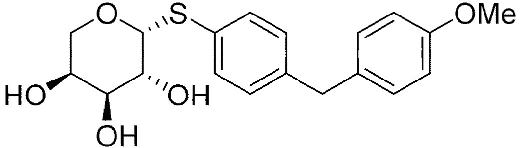

Example 29 was able to obtain the target compound using the same method as Example 1.

¹H NMR (400 MHz, DMSO-d6) δ 7.32 - 7.25 (m, 2H), 7.11 (dd, *J* = 11.1, 4.7 Hz, 4H), 6.87 - 6.78 (m, 2H), 5.34 (d, *J =* 5.5 Hz, 1H), 5.23 (d, *J =* 3.2 Hz, 1H), 4.88 (d, *J* = 4.5 Hz, 1H), 4.62 (d, *J* = 5.2 Hz, 1H), 3.83 - 3.71 (m, 5H), 3.68 (s, 3H), 3.57 (dt, *J* = 6.8, 3.6 Hz, 1H), 3.46 - 3.42 (m, 1H).

### Example 30. Preparation of (2R,3R,4S,5S)-2-(4-(2-(4-methoxyphenyl) propan-2-yl)phenoxy)tetrahydro-2H-pyran-3,4,5-triol

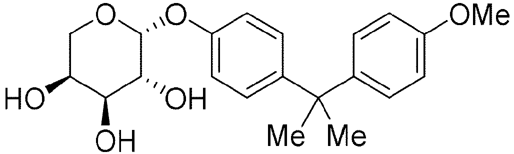

Example 30 was able to obtain the target compound using the same method as Example 1.

¹H NMR (400 MHz, CD₃OD) δ 7.12(dd, *J =* 8.8, 4.6 Hz, 4H), 6.99 (d, *J =* 8.8 Hz, 2H), 6.79 (d, *J =* 8.8 Hz, 2H), 5.47 (d, *J* = 3.1 Hz, 1H), 3.94 (m, *J =* 20.7, 13.8, 7.5 Hz, 4H), 3.75 (s, 3H), 3.64 (s, 1H), 1.61 (s, 6H).

### Example 31. Preparation of (2R,3R,4S,5S)-2-(4-(2-(4-ethoxyphenyl) propan-2-yl)phenoxy)tetrahydro-2H-pyran-3,4,5-triol

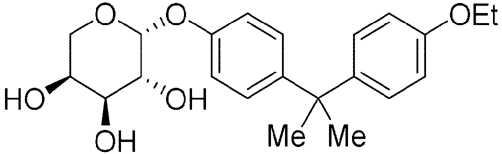

Example 31 was able to obtain the target compound using the same method as Example 1.

¹H NMR (400 MHz, CD₃OD) δ 7.11(dd, *J =* 10.9, 8.9 Hz, 4H), 6.98 (d, *J =* 8.8 Hz, 2H), 6.77 (d, *J =* 8.8 Hz, 2H), 5.47 (d, *J =* 3.2 Hz, 1H), 4.03 - 3.87 (m, 6H), 3.63 (d, *J* = 10.5 Hz, 1H), 1.61 (s, 6H), 1.35 (t, *J* = 7.0 Hz, 3H).

### Example 32. Preparation of (2R,3R,4S,5S)-2-(4-(1,1,1,3,3,3-hexafluoro-2-(4-methoxyphenyl)propan-2-yl)phenoxy)tetrahydro-2H-pyran-3,4,5-triol

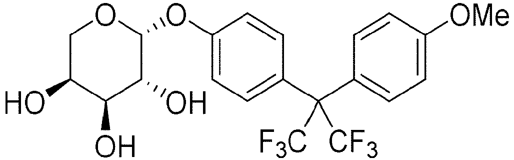

Example 32 was able to obtain the target compound using the same method as Example 1.

¹H NMR (400 MHz, DMSO-d6) δ 7.25 (d, *J* = 7.6 Hz, 4H), 7.13 (d, *J* = 9.0 Hz, 2H), 7.04 (d, *J =* 9.0 Hz, 2H), 5.52 (s, 1H), 4.98 (d, *J* = 4.3 Hz, 1H), 4.78 (d, *J =* 2.5 Hz, 1H), 4.70 (d, *J* = 3.4 Hz, 1H), 3.79 (d, *J* = 4.4 Hz, 6H), 3.68 (d, *J* = 11.8 Hz, 1H), 3.53 (dd, *J* = 11.9, 1.3 Hz, 1H).

### Example 33. Preparation of (2R,3R,4S,5S)-2-(4-(para-tolyloxy)phenoxy) tetrahydro-2H-pyran-3,4,5-triol

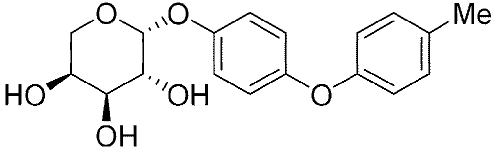

Example 33 was able to obtain the target compound using the same method as Example 1.

¹H NMR (400 MHz, DMSO-d6) δ 7.15 (d, *J =* 8.1 Hz, 2H), 7.04 (d, *J =* 9.0 Hz, 2H), 6.93 (d, *J* = 9.0 Hz, 2H), 6.85 (d, *J* = 8.5 Hz, 2H), 5.37 (s, 1H), 4.92 (d, *J =* 5.6 Hz, 1H), 4.74 (d, *J= 4.1* Hz, 1H), 4.66 (d, *J =* 3.4 Hz, 1H), 3.77 (s, 3H), 3.72 (d, *J =* 12.1 Hz, 1H), 3.51 (dd, *J=* 12.1, 1.9 Hz, 1H), 2.27 (s, 3H).

### Example 34. Preparation of (2R,3R,4S,5S)-2-(4-(3,5-dimethylphenoxy) phenoxy)tetrahydro-2H-pyran-3,4,5-triol

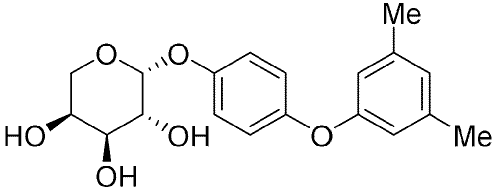

Example 34 was able to obtain the target compound using the same method as Example 1.

¹H NMR (400 MHz, DMSO-d6) δ 7.05 (d, *J* = 8.9 Hz, 2H), 6.94 (d, *J* = 8.9 Hz, 2H), 6.71 (s, 1H), 6.54 (s, 2H), 5.38 (s, 1H), 4.93 (d, *J =* 5.0 Hz, 1H), 4.75 (d, *J =* 3.1 Hz, 1H), 4.67 (d, *J =* 3.0 Hz, 1H), 3.77 (s, 3H), 3.72 (d, *J* = 11.7 Hz, 1H), 3.52 (dd, *J* = 11.0, 0.7 Hz, 1H), 2.22 (s, 6H).

### Example 35. Preparation of (2R,3R,4S,5S)-2-(4-(4-isopropylphenoxy) phenoxy)tetrahydro-2H-pyran-3,4,5-triol

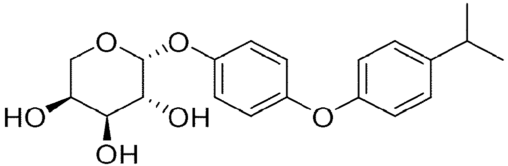

Example 35 was able to obtain the target compound using the same method as Example 1.

¹H NMR (400 MHz, DMSO-d6) δ 7.21 (d, *J =* 8.4 Hz, 2H), 7.05 (d, *J =* 8.9 Hz, 2H), 6.95 (d, *J* = 9.0 Hz, 2H), 6.87 (d, *J =* 8.5 Hz, 2H), 5.37 (s, 1H), 4.92 (d, *J =* 5.3 Hz, 1H), 4.74 (d, *J =* 3.4 Hz, 1H), 4.66 (d, *J* = 2.8 Hz, 1H), 3.77 (s, 3H), 3.72 (d, *J =* 11.9 Hz, 1H), 3.51 (dd, *J* = 11.9, 1.4 Hz, 1H), 2.89 - 2.84 (m, 1H), 1.19 (s, 3H), 1.17 (s, 3H).

### Example 36. Preparation of (2R,3R,4S,5S)-2-(4-(4-(tert-butyl)phenoxy)tetrahydro-2H-pyran-3,4,5-triol

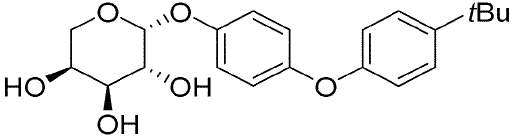

Example 36 was able to obtain the target compound using the same method as Example 1.

¹H NMR (400 MHz, DMSO-d6) δ 7.36 (d, *J* = 8.8 Hz, 2H), 7.05 (d, *J =* 9.1 Hz, 2H), 6.95 (d, *J* = 9.0 Hz, 2H), 6.87 (d, *J* = 8.8 Hz, 2H), 5.38 (s, 1H), 4.93 (d, *J =* 5.5 Hz, 1H), 4.75 (d, *J* = 4.3 Hz, 1H), 4.67 (d, *J =* 3.2 Hz, 1H), 3.77 (s, 3H), 3.72 (d, *J =* 11.7 Hz, 1H), 3.51 (dd, *J =* 11.8, 2.1 Hz, 1H), 1.27 (s, 9H).

### Example 37. Preparation of (2R,3R,4S,5S)-2-(4-(3-chloro-4-methoxybenzyl) phenoxy)tetrahydro-2H-pyran-3,4,5-triol

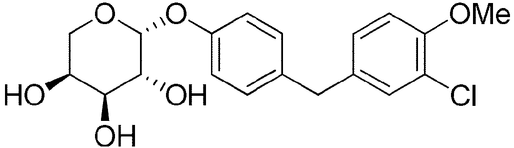

Example 37 was able to obtain the target compound using the same method as Example 1.

¹H NMR (400 MHz, DMSO-d6) δ 7.25 (d, *J =* 1.7 Hz, 1H), 7.14 (m, 3H), 7.05 (d, *J =* 8.4 Hz, 1H), 6.95 (d, *J =* 8.5 Hz, 2H), 5.38 (s, 1H), 4.91 (d, *J* = 4.7 Hz, 1H), 4.74 (s, 1H), 4.66 (d, *J* = 3.0 Hz, 1H), 3.80 (s, 3H), 3.75 (s, 2H), 3.67 (d, *J =* 11.7 Hz, 1H), 3.47 (dd, *J* = 12.2, 1.7 Hz, 1H).

### Example 38. Preparation of (2R,3R,4S,5S)-2-(3-chloro-4-(4-methoxybenzyl) phenoxy)tetrahydro-2H-pyran-3,4,5-triol

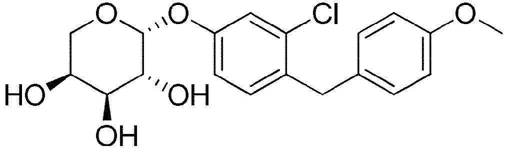

Example 38 was able to obtain the target compound using the same method as Example 1.

¹H NMR (400 MHz DMSO-d6) δ7.22 (d, *J =* 2.0 Hz, 1H), 7.18 - 7.05 (m, 4H), 6.87 - 6.77 (m, 2H), 5.57 (d, *J* = 2.5 Hz, 1H), 4.97 (d, *J* = 4.8 Hz, 1H), 4.83 (d, *J* = 4.7 Hz, 1H), 4.67 (d, *J =* 3.6 Hz, 1H), 3.84 - 3.60 (m, 9H), 3.45 (dd, *J =* 12.0, 2.2 Hz, 1H).

### Example 39. Preparation of (2R,3R,4S,5S)-2-(4-(4-ethoxybenzyl)phenoxy) tetrahydro-2H-pyran-3,4,5-triol

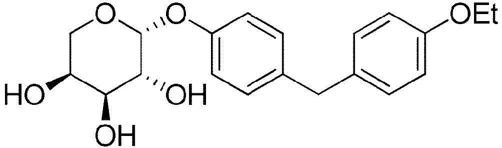

Example 39 was able to obtain the target compound using the same method as Example 1.

¹H NMR (400 MHz DMSO-d6) δ7.08 (dd, *J =* 8.5, 3.5 Hz, 4H), 6.92 (d, *J =* 8.5 Hz, 2H), 6.79 (d, *J* = 8.5 Hz, 2H), 5.35 (s, 1H), 4.89 (d, *J* = 5.3 Hz, 1H), 4.71 (d, *J* = 4.3 Hz, 1H), 4.63 (d, *J= 3.3* Hz, 1H), 3.94 (q, *J =* 7.0 Hz, 2H), 3.77 (s, 2H), 3.73 (s, 3H), 3.65 (d, *J=* 11.9 Hz, 1H), 3.45 (d, *J =* 10.2 Hz, 1H), 1.27 (t, *J =* 7.0 Hz, 3H).

### Example 40. Preparation of (2R,3R,4S,5S)-2-(3-chloro-4-(4-isopropylbenzyl)phenoxy)tetrahydro-2H-pyran-3,4,5-triol

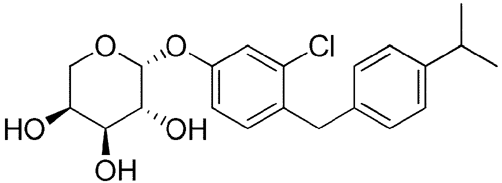

Example 40 was able to obtain the target compound using the same method as Example 1.

¹H NMR (400 MHz DMSO-d6) δ7.08 (dd, *J =* 8.5, 3.5 Hz, 4H), 6.92 (d, *J =* 8.5 Hz, 2H), 6.79 (d, *J =* 8.5 Hz, 2H), 5.35 (s, 1H), 4.89 (d, *J =* 5.3 Hz, 1H), 4.71 (d, *J* = 4.3 Hz, 1H), 4.63 (d, *J= 3.3* Hz, 1H), 3.94 (q, *J =* 7.0 Hz, 2H), 3.77 (s, 2H), 3.73 (s, 3H), 3.65 (d, *J=* 11.9 Hz, 1H), 3.45 (d, *J =* 10.2 Hz, 1H), 1.27 (t, *J=* 7.0 Hz, 3H).

### Example 41. Preparation of (2R,3R,4S,5S)-2-(2-fluoro-4-(4-isopropylbenzyl)phenoxy)tetrahydro-2H-pyran-3,4,5-triol

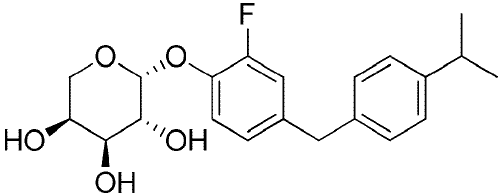

Example 41 was able to obtain the target compound using the same method as Example 1.

¹H NMR (400 MHz DMSO-d6) δ7.17 - 7.10 (m, 5H), 7.06 (dd, *J* = 12.2, 2.0 Hz, 1H), 6.94 (dd, *J =* 8.2, 2.0 Hz, 1H), 5.42 (d, *J =* 2.3 Hz, 1H), 4.98 (d, *J* = 4.6 Hz, 1H), 4.78 (d, *J* = 4.0 Hz, 1H), 4.66 (d, *J* = 3.4 Hz, 1H), 3.87 - 3.66 (m, 6H), 3.47 (dd, *J* = 12.0, 2.1 Hz, 1H), 2.84 - 2.77 (m, 1H), 1.14 (d, *J =* 6.9 Hz, 6H).

### Example 42. Preparation of (2R,3R,4S,5R)-2-(4-(4-methoxybenzyl)phenoxy) tetrahydro-2H-pyran-3,4,5-triol

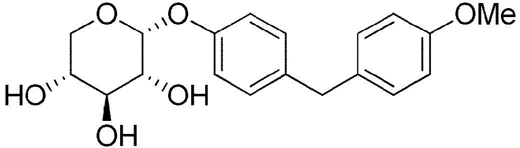

Example 42 was able to obtain the target compound using the same method as Example 1.

¹H NMR (400 MHz DMSO-d6) δ7.11 (d, *J =* 7.0 Hz, 4H), 6.94 (d, *J =* 8.5 Hz, 2H), 6.83 (d, *J =* 8.6 Hz, 2H), 5.34 (d, *J* = 3.5 Hz, 1H), 5.03 (dd, *J =* 9.9, 5.4 Hz, 2H), 4.96 (d, *J =* 4.8 Hz, 1H), 3.80 (s, 2H), 3.70 (s, 3H), 3.57 - 3.51 (m, 1H), 3.46 (d, *J= 4.8* Hz, 1H).

### Example 43. Preparation of (2R,3R,4S,5R)-2-(4-(4-isopropylbenzyl) phenoxy)tetrahydro-2H-pyran-3,4,5-triol

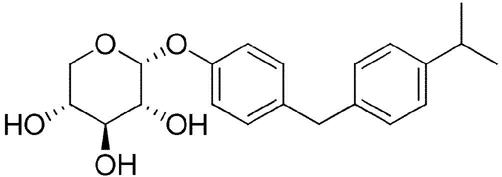

Example 43 was able to obtain the target compound using the same method as Example 1.

¹H NMR (400 MHz DMSO-d6) δ7.13 (m, 6H), 6.95 (d, *J* = 8.6 Hz, 2H), 5.34 (d, *J =* 3.5 Hz, 1H), 5.02 (dd, *J =* 10.2, 5.5 Hz, 2H), 4.95 (d, *J =* 4.5 Hz, 1H), 3.82 (s, 2H), 3.53 (s, 1H), 3.46 (d, *J* = 4.9 Hz, 1H), 2.85 - 2.79 (m, 1H), 1.17 (s, 3H), 1.15 (s, 3H).

### Example 44. Preparation of (2R,3R,4S,5S)-2-(4-(4-isopropylbenzyl) phenoxy)tetrahydro-2H-pyran-3,4,5-triol

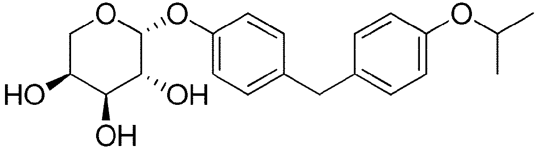

Example 44 was able to obtain the target compound using the same method as Example 1.

¹H NMR (400 MHz DMSO-d6) δ7.08 (dd, *J =* 10.8, 8.6 Hz, 4H), 6.92 (d, *J* = 8.5 Hz, 2H), 6.78 (d, *J =* 8.5 Hz, 2H), 5.35 (s, 1H), 4.88 (d, *J =* 5.3 Hz, 1H), 4.71 (d, *J =* 4.3 Hz, 1H), 4.63 (d, *J* = 3.3 Hz, 1H), 4.51 (dt, *J =* 12.0, 6.0 Hz, 1H), 3.76 (s, 2H), 3.73 (s, 3H), 3.65 (d, *J =* 11.7 Hz, 1H), 3.45 (d, *J =* 10.7 Hz, 1H), 1.20 (d, *J =* 6.0 Hz, 6H).

### Example 45. Preparation of (2R,3R,4S,5S)-2-(4-(4-(cyclopentyloxy) benzyl)phenoxy)tetrahydro-2H-pyran-3,4,5-triol

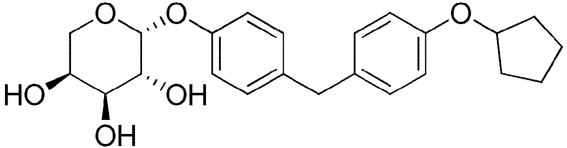

Example 45 was able to obtain the target compound using the same method as Example 1.

¹H NMR (400 MHz DMSO-d6) δ7.07 (dd, *J =* 11.5, 8.6 Hz, 4H), 6.92 (d, *J =* 8.5 Hz, 2H), 6.77 (d, *J* = 8.5 Hz, 2H), 5.35 (s, 1H), 4.88 (d, *J =* 5.3 Hz, 1H), 4.71 (d, *J =* 4.0 Hz, 1H), 4.63 (d, *J =* 3.2 Hz, 1H), 3.76 (s, 2H), 3.73 (s, 3H), 3.65 (d, *J =* 11.9 Hz, 1H), 3.45 (d, *J =* 10.0 Hz, 1H), 1.94 - 1.77 (m, 3H), 1.71 - 1.59 (m, 4H), 1.58 - 1.47 (m, 2H).

### Example 46. Preparation of (2R,3R,4S,5S)-2-(2-fluoro-4-(4-methoxybenzyl) phenoxy)tetrahydro-2H-pyran-3,4,5-triol

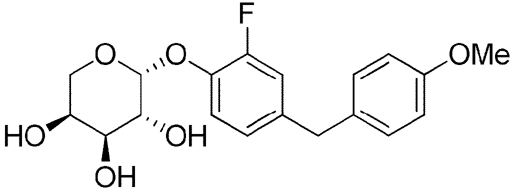

Example 46 was able to obtain the target compound using the same method as Example 1.

¹H NMR (400 MHz, DMSO-d6) δ7.19 - 7.07 (m, 3H), 7.02 (dd, *J =* 12.3, 2.1 Hz, 1H), 6.92 (dd, *J =* 8.5, 2.1 Hz, 1H), 6.87 - 6.76 (m, 2H), 5.42 (d, *J* = 2.3 Hz, 1H), 4.99 (d, *J =* 4.9 Hz, 1H), 4.78 (d, *J =* 4.4 Hz, 1H), 4.66 (d, *J =* 3.4 Hz, 1H), 3.82 - 3.66 (m, 9H), 3.47 (dd, *J =* 12.0, 2.2 Hz, 1H).

### Example 47. Preparation of (2R,3R,4S,5S)-2-(3-fluoro-4-(4-methoxybenzyl) phenoxy)tetrahydro-2H-pyran-3,4,5-trio

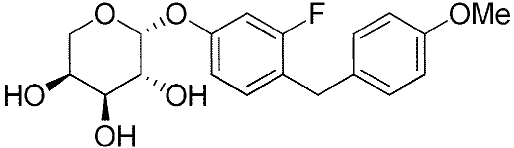

Example 47 was able to obtain the target compound using the same method as Example 1.

¹H NMR (400 MHz, DMSO-d6) δ 7.15 (t, *J =* 8.7 Hz, 1H), 7.08 (d, *J =* 8.5 Hz, 2H), 6.88 - 6.76 (m, 4H), 5.42 (d, *J =* 2.1 Hz, 1H), 4.92 (dd, *J =* 4.3*,* 1.8 Hz, 1H), 4.73 (d, *J =* 4.5 Hz, 1H), 4.65 (d, *J* = 3.5 Hz, 1H), 3.81 - 3.62 (m, 9H), 3.47 (dd, *J =* 12.1, 2.2 Hz, 1H).

### Example 48. Preparation of (2R,3R,4S,5S)-2-(3-fluoro-4-(4-isopropylbenzyl)phenoxy)tetrahydro-2H-pyran-3,4,5-triol

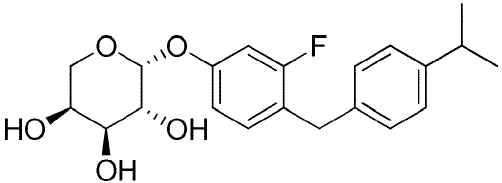

Example 48 was able to obtain the target compound using the same method as Example 1.

¹H NMR (400 MHz, DMSO-d6) δ 7.18 (t, *J =* 8.7 Hz, 1H), 7.10 (q, *J =* 8.1 Hz, 4H), 6.85 - 6.79 (m, 2H), 5.42 (d, *J =* 2.1 Hz, 1H), 4.94 - 4.92 (m, 1H), 4.73 (d, *J* = 4.4 Hz, 1H), 4.66 (d, *J* = 3.5 Hz, 1H), 3.82 (s, 2H), 3.73 (dd, *J =* 4.1, 2.0 Hz, 3H), 3.63 (d, *J =* 12.0 Hz, 1H), 3.49 - 3.45 (m, 1H), 2.79 (h, *J* = 6.9 Hz, 1H), 1.14 (d, *J* = 6.9 Hz, 6H).

### Example 49. Preparation of (2R,3R,4S,5S)-2-(4-(2-chloro-4-methoxybenzyl) phenoxy)tetrahydro-2H-pyran-3,4,5-triol

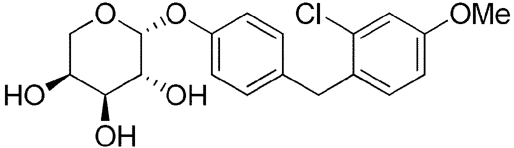

Example 49 was able to obtain the target compound using the same method as Example 1.

¹H NMR (400 MHz, DMSO-d6) δ 7.25 (d, *J =* 1.8 Hz, 1H), 7.14 (m, 3H), 7.05 (d, *J =* 8.4 Hz, 1H), 6.95 (d, *J =* 8.5 Hz, 2H), 5.38 (d, *J =* 1.5 Hz, 1H), 4.89 (d, *J =* 5.5 Hz, 1H), 4.72 (d, *J* = 4.3 Hz, 1H), 4.64 (d, *J* = 3.4 Hz, 1H), 3.80 (s, 3H), 3.75 (s, 2H), 3.67 (d, *J =* 11.9 Hz, 1H), 3.47 (dd, *J =* 11.9, 1.8 Hz, 1H).

### Example 50. Preparation of (2R,3R,4S,5S)-2-(4-(2-fluoro-4-methoxybenzyl) phenoxy)tetrahydro-2H-pyran-3,4,5-triol

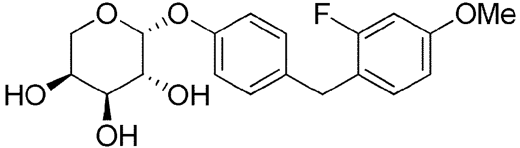

Example 50 was able to obtain the target compound using the same method as Example 1.

¹H NMR (400 MHz, DMSO-d6) δ 7.17 (t, *J =* 8.8 Hz, 1H), 7.09 (d, *J =* 8.4 Hz, 2H), 6.94 (d, *J* = 8.6 Hz, 2H), 6.78 (dd, *J =* 12.1, 2.4 Hz, 1H), 6.71 (dd, *J =* 8.4, 2.2 Hz, 1H), 5.37 (d, *J =* 1.5 Hz, 1H), 4.90 (d, *J* = 5.4 Hz, 1H), 4.73 (d, *J =* 4.2 Hz, 1H), 4.64 (d, *J* = 3.3 Hz, 1H), 3.81 (s, 2H), 3.75 (s, 3H), 3.66 (d, *J =* 12.0 Hz, 1H), 3.47 (dd, *J =* 11.8, 1.8 Hz, 1H).

### Example 51. Preparation of (2R,3R,4S,5S)-2-(2-chloro-4-(4-methoxybenzyl) phenoxy)tetrahydro-2H-pyran-3,4,5-triol

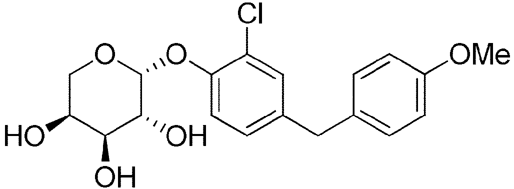

Example 51 was able to obtain the target compound using the same method as Example 1.

¹H NMR (400 MHz, DMSO-d6) δ 7.24 (d, *J* = 2.0 Hz, 1H), 7.20 - 7.07 (m, 4H), 6.87 - 6.82 (m, 2H), 5.58 (d, *J =* 2.5 Hz, 1H), 4.98 (d, *J =* 4.7 Hz, 1H), 4.84 (d, *J =* 4.4 Hz, 1H), 4.68 (d, *J* = 3.7 Hz, 1H), 3.87 - 3.75 (m, 5H), 3.70 - 3.67 (m, 4H), 3.47 (dd, *J* = 12.1, 2.2 Hz, 1H).

### Example 52. Preparation of (2R,3R,4S,5S)-2-(4-((4'-isopropyl-[1,1'-biphenyl]-4-yl)methyl)phenoxy)tetrahydro-2H-pyran-3,4,5-triol

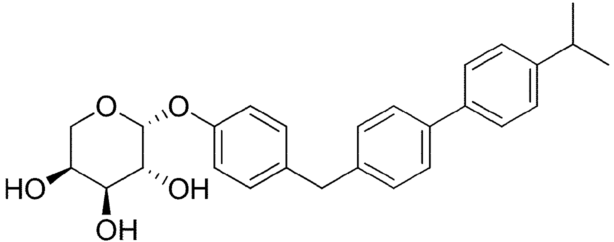

Example 52 was able to obtain the target compound using the same method as Example 1.

¹H NMR (400 MHz, DMSO-d6) δ 7.52 (d, *J* = 7.0 Hz, 4H), 7.32 - 7.25 (m, 4H), 7.16 (d, *J* = 8.5 Hz, 2H), 6.95 (d, *J =* 8.6 Hz, 2H), 5.37 (s, 1H), 4.90 (d, *J =* 5.0 Hz, 1H), 4.73 (s, 1H), 4.65 (s, 1H), 3.89 (s, 2H), 3.74 (s, 3H), 3.66 (d, *J* = 11.5 Hz, 1H), 3.46 (d, *J* = 11.1 Hz, 1H), 2.95 - 2.85 (m, 1H), 1.21 (d, *J* = 6.9 Hz, 6H).

### Example 53. Preparation of (2R,3R,4S,5S)-2-(4-((5-methoxybenzo[d] oxazol-2-yl)methyl)phenoxy)tetrahydro-2H-pyran-3,4,5-triol

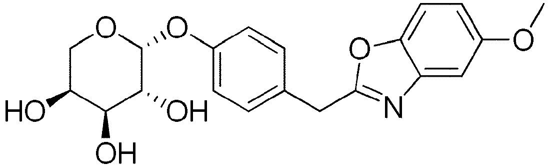

Example 53 was able to obtain the target compound using the same method as Example 1.

MS(m/z): 388.1 [M+H]⁺

### Example 54. Preparation of (2R,3R,4S,5S)-2-(3-(4-isopropylphenoxy) phenoxy)tetrahydro-2H-pyran-3,4,5-triol

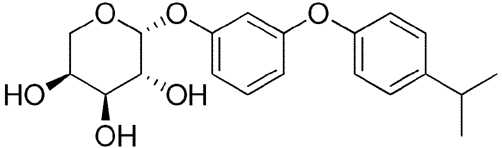

Example 54 was able to obtain the target compound using the same method as Example 1.

¹H NMR (400 MHz, DMSO-d6) δ 7.28 - 7.23 (m, 3H), 6.96 (d, *J =* 8.6 Hz, 2H), 6.79 (dd, *J* = 8.4, 1.8 Hz, 1H), 6.61 (d, *J* = 2.2 Hz, 1H), 6.58 (dd, *J =* 8.2, 1.8 Hz, 1H), 5.40 (s, 1H), 4.93 (s, 1H), 4.75 (s, 1H), 4.66 (s, 1H), 3.74 (s, 3H), 3.66 (d, *J* = 11.8 Hz, 1H), 3.49 (dd, *J =* 12.0, 2.1 Hz, 1H), 2.92 - 2.87 (m, 1H), 1.21 (s, 3H), 1.19 (s, 3H).

### Example 55. Preparation of (2R,3R,4S,5S)-2-(4-(4-methoxyphenoxy) phenoxy)tetrahydro-2H-pyran-3,4,5-triol

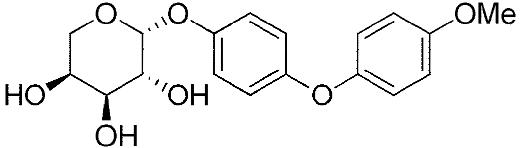

Example 55 was able to obtain the target compound using the same method as Example 1.

¹H NMR (400 MHz, DMSO-d6) δ 7.00 (d, *J =* 9.1 Hz, 2H), 6.93 - 6.83 (m, 6H), 5.33 (s, 1H), 4.91 (d, *J =* 5.5 Hz, 1H), 4.73 (d, *J* = 3.8 Hz, 1H), 4.65 (d, *J* = 3.2 Hz, 1H), 3.74 (d, *J* = 1.6 Hz, 3H), 3.71-3.68 (m, 4H), 3.48 (d, *J =* 11.8 Hz, 1H).

### Example 56. Preparation of (2R,3R,4S,5R)-2-(4-(4-isopropylphenoxy) phenoxy)tetrahydro-2H-pyran-3,4,5-triol

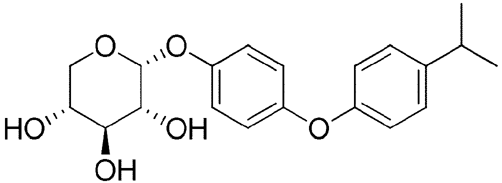

Example 56 was able to obtain the target compound using the same method as Example 1.

¹H NMR (400 MHz, DMSO-d6) δ 7.22 (d, *J =* 8.4 Hz, 2H), 7.05 (d, *J =* 9.1 Hz, 2H), 6.95 (d, *J= 9.0* Hz, 2H), 6.87 (d, *J* = 8.6 Hz, 2H), 5.34 (d, *J =* 3.5 Hz, 1H), 5.07 (d, *J* = 6.4 Hz, 1H), 5.03 (d, *J =* 4.6 Hz, 1H), 4.97 (d, *J=* 5.0 Hz, 1H), 3.55 (dd, *J=* 8.4, 5.2 Hz, 1H), 3.50 (d, *J* = 5.0 Hz, 1H), 3.37 (d, *J =* 6.1 Hz, 3H), 2.85 (dd, *J =* 13.4, 6.6 Hz, 1H), 1.19 (s, 3H), 1.17 (s, 3H).

### Example 57. Preparation of (2R,3R,4S,5S)-2-(2-chloro-4-(4-isopropylphenoxy)phenoxy)tetrahydro-2H-pyran-3,4,5-triol

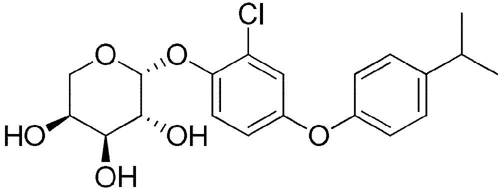

Example 57 was able to obtain the target compound using the same method as Example 1.

¹H NMR (400 MHz, DMSO-d6) δ 7.31 - 7.19 (m, 3H), 7.08 (d, *J =* 2.9 Hz, 1H), 6.94 - 6.89 (m, 3H), 5.55 (d, *J* = 2.3 Hz, 1H), 5.00 (d, *J* = 4.6 Hz, 1H), 4.85 (d, *J* = 4.4 Hz, 1H), 4.69 (d, *J* = 3.6 Hz, 1H), 3.85 - 3.69 (m, 4H), 3.49 (dd, *J =* 12.0, 2.2 Hz, 1H), 2.86 (hept, *J =* 6.9 Hz, 1H), 1.17 (d, *J* = 6.9 Hz, 6H).

### Example 58. Preparation of (2R,3R,4R,5S)-5-fluoro-2-(4-(4-isopropylphenoxy)phenoxy)tetrahydro-2H-pyran-3,4-diol

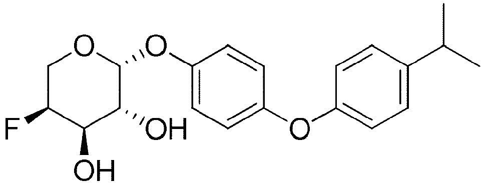

In Example 58, the target compound could be obtained by further synthesizing a compound prepared by the same method as in Example 1 using the method of Preparation Example 8.

¹H NMR (400 MHz, DMSO-d6) δ 7.22 (d, *J =* 8.4 Hz, 2H), 7.07 (d, *J =* 9.0 Hz, 2H), 6.96 (d, *J* = 9.0 Hz, 2H), 6.87 (d, *J* = 8.6 Hz, 2H), 5.46 (d, *J* = 3.3 Hz, 1H), 5.25 (dd, *J* = 27.8, 5.7 Hz, 2H), 4.83 - 4.71 (d, *J =* 49.8 Hz, 1H), 3.96 - 3.70 (m, 5H), 2.86 (dt, *J=* 14.0, 7.1 Hz, 1H), 1.19 (s, 3H), 1.18 (s, 3H).

### Example 59. Preparation of (2R,3R,4S,5R,6R)-2-(hydroxymethyl)-6-(4-(4-isopropylphenoxy)phenoxy)tetrahydro-2H-pyran-3,4,5-triol

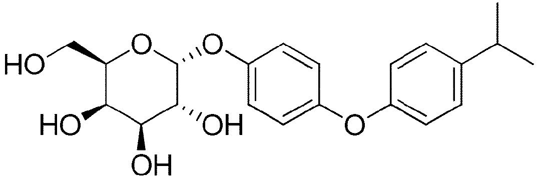

Example 59 was able to obtain the target compound using the same method as Example 1.

¹H NMR (400 MHz, DMSO-d6) δ 7.21 (d, *J =* 8.4 Hz, 2H), 7.09 (d, *J =* 9.0 Hz, 2H), 6.94 (d, *J =* 9.0 Hz, 2H), 6.87 (d, *J =* 8.5 Hz, 2H), 5.31 (d, *J* = 2.1 Hz, 1H), 4.86 (d, *J* = 5.9 Hz, 1H), 4.73 (d, *J =* 4.8 Hz, 1H), 4.54 (t, *J* = 4.3 Hz, 2H), 3.76 (dd, *J =* 16.3, 4.6 Hz, 4H), 3.56 - 3.50 (m, 1H), 3.43 - 3.37 (m, 1H), 2.86 (dt, *J=* 13.9, 6.9 Hz, 1H), 1.19 (s, 3H), 1.17 (s, 3H).

### Example 60. Preparation of (2R,3R,4S,5S)-2-(4-(4-isopropylphenoxy)phenoxy)-4-methoxytetrahydro-2H-pyran-3,5-diol

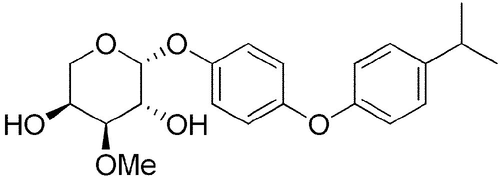

In Example 60, the target compound could be obtained by further synthesizing a compound prepared by the same method as in Example 1 using the method of Preparation Example 9.

¹H NMR (400 MHz, DMSO-d6) δ 7.23 - 7.16 (m, 2H), 7.03 (td, *J =* 6.4, 2.2 Hz, 2H), 6.96 - 6.90 (m, 2H), 6.88 - 6.82 (m, 2H), 5.36 (d, *J =* 3.5 Hz, 1H), 5.01 (d, *J =* 6.8 Hz, 1H), 4.70 (d, *J* = 4.1 Hz, 1H), 3.99 (d, *J =* 4.5 Hz, 1H), 3.85 (ddd, *J* = 10.1, 6.7, 3.5 Hz, 1H), 3.70 (d, *J =* 12.0 Hz, 1H), 3.52 (dd, *J =* 12.1, 2.3 Hz, 1H), 3.36 (s, 3H), 3.44 (dd, *J =* 9.8, 3.2 Hz, 1H), 2.83 (h, *J =* 6.9 Hz, 1H), 1.16 (d, *J* = 6.9 Hz, 6H).

### Example 61. Preparation of (2R,3R,4R,5S)-2-(4-(4-isopropylphenoxy) phenoxy)-5-methoxytetrahydro-2H-pyran-3,4-diol

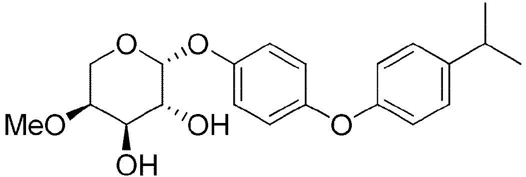

In Example 61, the target compound was obtained by further synthesizing the compound prepared in the same manner as in Example 1 as in Preparation Example 7.

¹H NMR (400 MHz, DMSO-d6) δ 7.20 (d, *J =* 8.5 Hz, 2H), 7.05 - 7.01 (m, 2H), 6.95 - 6.90 (m, 2H), 6.87 - 6.82 (m, 2H), 5.35 (d, *J =* 3.4 Hz, 1H), 4.95 (d, *J =* 6.5 Hz, 1H), 4.75 (d, *J =* 6.1 Hz, 1H), 3.84 (ddd, *J =* 9.6*,* 6.2, 3.5 Hz, 1H), 3.74 - 3.67 (m, 2H), 3.61 (d, *J =* 12.2 Hz, 1H), 3.44 (s, 1H), 3.31 (s, 3H), 2.84 (p, *J =* 7.0 Hz, 1H), 1.16 (d, *J =* 6.9 Hz, 6H).

### Example 62. Preparation of (3S,4S,5R,6R)-6-(4-(4-isopropylphenoxy) phenoxy)-5-methoxytetrahydro-2H-pyran 3,4-diol

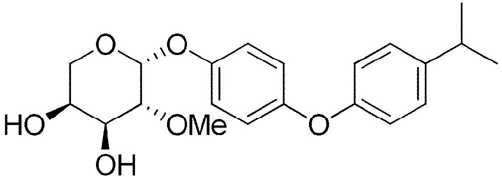

In Example 62, the target compound was obtained by further synthesizing the compound prepared in the same manner as in Example 1 as in Preparation Example 7.

¹H NMR (400 MHz, DMSO-d6) δ 7.22 (d, *J =* 8.5 Hz, 2H), 7.05 (d, *J =* 9.0 Hz, 2H), 6.95 (d, *J* = 9.0 Hz, 2H), 6.87 (d, *J* = 8.5 Hz, 2H), 5.66 (d, *J =* 3.2 Hz, 1H), 4.94 (d, *J* = 6.1 Hz, 1H), 4.78 (d, *J* = 3.6 Hz, 1H), 3.86 (ddd, *J =* 9.6*,* 6.2, 3.4 Hz, 1H), 3.76 (s, 1H), 3.70 (d, *J* = 11.9 Hz, 1H), 3.50 (d, *J =* 3.6 Hz, 2H), 3.41 (s, 3H), 2.86 (dt, *J =* 13.7, 6.9 Hz, 1H), 1.18 (d, *J* = 6.9 Hz, 6H).

### Example 63. Preparation of (3S,4S,5R,6R)-5-fluoro-6-(4-(4-isopropylphenoxy)phenoxy)tetrahydro-2H-pyran-3,4-diol

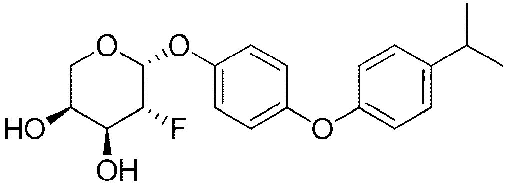

Example 63 was able to obtain the target compound using the same method as Example 1 and Compound No. 99 of Example 10.

¹H NMR (400 MHz, DMSO-d6) δ 7.22 (d, *J =* 8.5 Hz, 2H), 7.07 (t, *J =* 8.6 Hz, 2H), 6.97 (d, *J* = 8.8 Hz, 2H), 6.92 - 6.83 (m, 2H), 5.75 (s, 1H), 5.35 (dd, *J =* 23.1, 6.2 Hz, 1H), 5.03 - 4.96 (m, 1H), 4.77 - 4.58 (m, 1H), 4.57 - 4.33 (m, 1H), 3.75 (d, *J=* 6.3 Hz, 1H), 3.69 (d, *J=* 12.3 Hz, 1H), 3.55 (d, *J =* 12.3 Hz, 1H), 2.87 (dt, *J =* 13.6, 6.7 Hz, 1H), 1.19 (d, *J =* 6.9 Hz, 6H)
MS(m/z): 385.1 [M+Na]⁺

### Example 64. Preparation of (2R,3R,4R,5R)-5-fluoro-2-(4-(4-isopropylphenoxy)phenoxy)tetrahydro-2H-pyran-3,4-diol

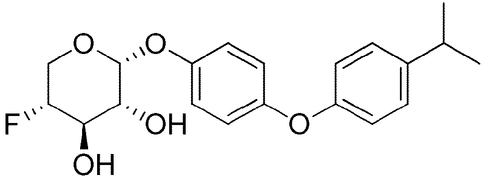

Example 64 was able to obtain the target compound using the same method as in Example 58.

¹H NMR (400 MHz, DMSO-d6) δ 7.22 (d, *J* = 8.4 Hz, 2H), 7.09 (d, *J* = 9.0 Hz, 2H), 6.96 (d, *J =* 9.0 Hz, 2H), 6.88 (d, *J =* 8.6 Hz, 2H), 5.54 (d, *J =* 4.8 Hz, 1H), 5.40 (d, *J =* 3.6 Hz, 2H), 4.50 - 4.31 (m, 1H), 3.82 (dd, *J =* 10.7, 5.8 Hz, 2H), 3.58 (td, *J =* 10.7, 5.0 Hz, 1H), 3.46 - 3.41 (m, 1H), 2.89 - 2.84 (m, 1H), 1.19 (s, 3H), 1.18 (s, 3H).

### Example 65. Preparation of (3S,4S,5S,6R)-5-fluoro-6-(4-(4-isopropylphenoxy)phenoxy)tetrahydro-2H-pyran-3,4-diol

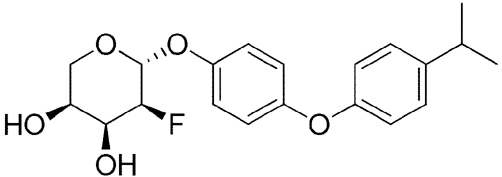

In Example 65, the target compound could be obtained using the same method as in Example 63.

¹H NMR (400 MHz, DMSO-d6) δ 7.22 (d, *J* = 8.5 Hz, 2H), 7.05 (d, *J* = 9.0 Hz, 2H), 6.96 (d, *J =* 9.1 Hz, 2H), 6.88 (d, *J =* 8.6 Hz, 2H), 5.41 - 5.33 (m, 2H), 4.90 (d, *J=* 5.3 Hz, 1H), 4.49 (ddd, *J =* 48.1, 6.7, 2.9 Hz, 1H), 4.11 (dd, *J =* 14.7, 3.5 Hz, 1H), 3.72 - 3.60 (m, 3H), 2.87 (dt, *J* = 14.1, 6.9 Hz, 1H), 1.19 (s, 3H), 1.18 (s, 3H).

### Example 66. Preparation of (3S,4R,6R)-6-(4-(4-isopropylphenoxy) phenoxy)tetrahydro-2H-pyran-3,4-diol

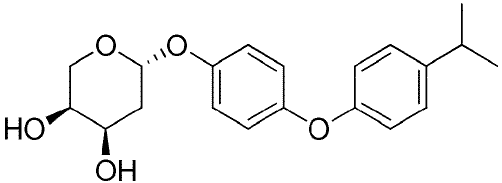

In Example 66, the target compound was obtained using the same method as in Example 1 with compound 100 of Example 10.

¹H NMR (400 MHz, DMSO-d6) δ 7.21 (d, *J =* 8.5 Hz, 2H), 7.03 (d, *J =* 9.0 Hz, 2H), 6.94 (d, *J=* 9.0 Hz, 2H), 6.86 (d, *J =* 8.6 Hz, 2H), 5.58 (t, *J =* 6.1 Hz, 1H), 4.74 (d, *J* = 5.5 Hz, 1H), 4.65 (d, *J =* 4.0 Hz, 1H), 3.97 (dd, *J =* 8.8, 5.0 Hz, 1H), 3.72 - 3.62 (m, 2H), 3.56 (dd, *J =* 11.6, 3.6 Hz, 1H), 2.86 (dt, *J =* 13.8, 7.0 Hz, 1H), 2.06 - 1.98 (m, 1H), 1.84 - 1.72 (m, 1H), 1.18 (d, *J* = 6.9 Hz, 6H).

MS(m/z): 367.2 [M+Na]⁺

### Example 67. Preparation of (2R,3R,4S,5R)-2-(2-chloro-4-(4-isopropylphenoxy) henoxy)tetrahydro-2H-pyran-3,4,5-triol

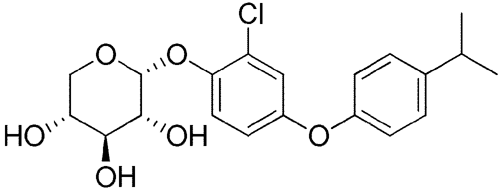

Example 67 was able to obtain the target compound using the same method as in Example 1.

¹H NMR (400 MHz, DMSO-d6) δ 7.26 (t, *J* = 9.0 Hz, 3H), 7.11 (d, *J=* 2.9 Hz, 1H), 6.98 - 6.91 (m, 3H), 5.53 (d, *J* = 3.5 Hz, 1H), 5.17 (d, *J =* 5.6 Hz, 1H), 5.10 (d, *J =* 4.4 Hz, 1H), 5.07 (d, *J=* 4.9 Hz, 1H), 3.68 - 3.59 (m, 1H), 3.54 - 3.48 (m, 1H), 3.46 - 3.37 (m, 3H), 2.88 (p, *J =* 6.9 Hz, 1H), 1.19 (d, *J =* 6.9 Hz, 6H).

### Example 68. Preparation of (2R,3R,4S,5S)-2-(2-fluoro-4-(4-isopropylphenoxy)phenoxy)tetrahydro-2H-pyran-3,4,5-triol

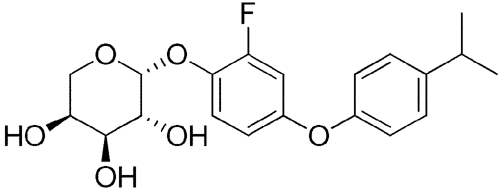

Example 68 was able to obtain the target compound using the same method as Example 1.

¹H NMR (400 MHz, DMSO-d6) δ 7.28 - 7.17 (m, 3H), 6.95 - 6.89 (m, 2H), 6.80 - 6.67 (m, 2H), 5.46 (d, *J* = 3.0 Hz, 1H), 4.09 - 3.92 (m, 4H), 3.67 (dd, *J =* 12.4, 2.1 Hz, 1H), 2.90 (hept, *J* = 6.9 Hz, 1H), 1.24 (d, *J =* 6.9 Hz, 6H).

### Example 69. Preparation of (3S,4R,5R,6R)-5-azido-6-(4-(4-isopropylphenoxy)phenoxy) tetrahydro-2H-pyra-3,4-diol

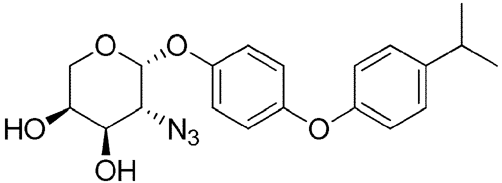

Example 69 was able to obtain the target compound using the same method as Example 1 and Compound No. 102 of Example 10.

¹H NMR (400 MHz, DMSO-d6) δ 7.18 (d, *J =* 8.5 Hz, 2H), 7.07 (d, *J =* 9.0 Hz, 2H), 6.92 (d, *J* = 9.0 Hz, 2H), 6.85 (d, *J =* 8.6 Hz, 2H), 5.53 (d, *J =* 3.3 Hz, 1H), 4.19 (dd, *J =* 10.5, 3.2 Hz, 1H), 3.97 (d, *J =* 12.2 Hz, 2H), 3.73 - 3.61 (m, 2H), 2.86 (dq, *J =* 14.0, 6.9 Hz, 1H), 1.23 (d, *J =* 6.9 Hz, 6H).

### Example 70. Preparation of (3S,4R,5R,6R)-5-amino-6-(4-(4-isopropylphenoxy)phenoxy)tetrahydro-2H-pyran-3,4-diol

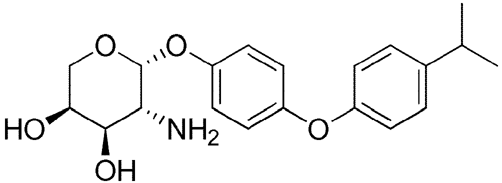

For Example 70, Pd/C (10%) was carefully added to a mixed solution of anhydrous EtOAc (10 ml) and Example 69 (1.0 mmol) in the presence of argon, stirred, and the inside of the reaction vessel was purged with hydrogen gas. The reaction mixture was then stirred at 25 °C for 1 hour. After the reaction was complete, the Pd/C catalyst was removed using a Celite filter and concentrated under reduced pressure. The reaction mixture was solidified with DCM/Hexane to obtain the compound of Example 70.

¹H NMR (400 MHz, DMSO-d6) δ 7.18 (d, *J =* 8.5 Hz, 2H), 7.10 (d, *J =* 9.0 Hz, 2H), 6.91 (d, *J =* 9.0 Hz, 2H), 6.84 (d, *J =* 8.6 Hz, 2H), 5.46 (d, *J* = 3.3 Hz, 1H), 3.96 (d, *J* = 12.5 Hz, 1H), 3.88 (s, 1H), 3.80 (dd, *J* = 10.1, 3.2 Hz, 1H), 3.69 (d, *J* = 12.5 Hz, 1H), 3.15 (dd, *J* = 10.2, 3.4 Hz, 1H), 2.87 (dt, *J* = 13.9, 6.8 Hz, 1H), 1.23 (d, *J =* 6.9 Hz, 6H).

### Example 71. Preparation of (4-methoxyphenyl)(4-(((2R,3R,4S,5S)-3,4,5-trihydroxytetrahydro-2H-pyran-2-yl)oxy)phenyl)methanone

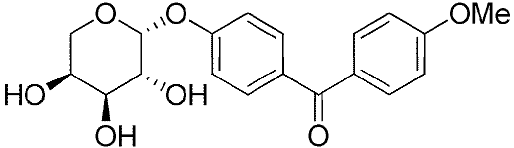

Example 71 was able to obtain the target compound using the same method as in Example 1.

¹H NMR (400 MHz, DMSO-d6) δ 7.79 - 7.63 (m, 4H), 7.09 (dd, *J =* 23.2, 8.7 Hz, 4H), 5.27 (d, *J =* 5.1 Hz, 1H), 4.97 (d, *J* = 6.8 Hz, 1H), 4.86 (s, 1H), 4.67 (d, *J =* 4.1 Hz, 1H), 3.84 (s, 3H), 3.77 - 3.68 (m, 2H), 3.63 (d, *J =* 10.9 Hz, 2H), 3.50 - 3.43 (m, 1H).

### Example 72. Preparation of 4-(4-(((2S,3R,4S,5S)-3,4,5-trihydroxytetrahydro-2H-pyran-2-yl)oxy)benzoyl)benzonitrile

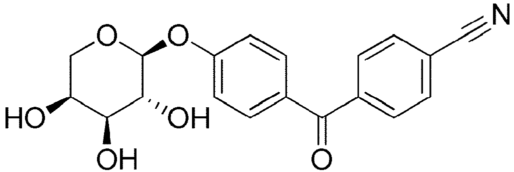

Example 72 was synthesized through Preparation Example 3, and the target compound was obtained by hydrolyzing according to Preparation Example 5 after introducing a substituent in the α/β form.

¹H NMR (400 MHz, DMSO-d6) δ 8.03 (d, *J* = 8.3 Hz, 2H), 7.84 (d, *J* = 8.3 Hz, 2H), 7.76 (d, *J =* 8.8 Hz, 2H), 7.17 (d, *J =* 8.9 Hz, 2H), 5.31 (d, *J* = 5.1 Hz, 1H), 5.02 (d, *J* = 6.7 Hz, 1H), 4.91 (d, *J* = 5.5 Hz, 1H), 4.71 (d, *J* = 3.8 Hz, 1H), 3.73 (dd, *J =* 11.6, 8.0 Hz, 2H), 3.65 (d, *J= 10.9* Hz, 2H), 3.51 - 3.47 (m, 1H).

### Example 73. Preparation of (2R,3R,4S,5S)-2-(4-(((1R,4R)-4-ethylcyclohexyl)methyl)phenoxy)tetrahydro-2H-pyran-3,4,5-triol

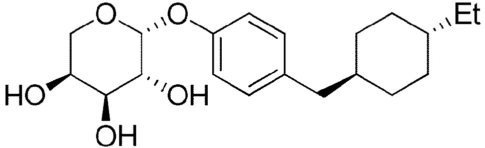

Example 73 was able to obtain the target compound using the same method as in Example 1.

¹H NMR (400 MHz, DMSO-d6) δ 7.02 (d, *J* = 8.4 Hz, 2H), 6.90 (d, *J* = 8.5 Hz, 2H), 5.35 (s, 1H), 4.89 (d, *J =* 5.1 Hz, 1H), 4.72 (d, *J =* 4.0 Hz, 1H), 4.64 (d, *J =* 3.0 Hz, 1H), 3.74 (s, 3H), 3.67 (d, *J =* 11.8 Hz, 1H), 3.47 (d, *J =* 10.5 Hz, 1H), 2.36 (d, *J* = 6.9 Hz, 2H), 1.63 (dd, *J =* 24.2, 12.3 Hz, 4H), 1.39 - 1.30 (m, 1H), 1.20 - 1.08 (m, 2H), 1.02 (s, 1H), 0.95 - 0.68 (m, 7H).

### Example 74. Preparation of (2R,3R,4S,5S)-2-(4-(((1R,4R)-4-isopropylcyclohexyl)methyl)phenoxy)tetrahydro-2H-pyran-3,4,5-triol

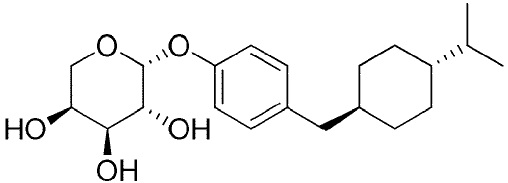

Example 74 was able to obtain the target compound using the same method as Example 1.

¹H NMR (400 MHz, DMSO-d6) δ 7.02 (d, *J =* 8.4 Hz, 2H), 6.90 (d, *J =* 8.5 Hz, 2H), 5.35 (s, 1H), 4.89 (d, *J* = 5.4 Hz, 1H), 4.72 (d, *J* = 4.4 Hz, 1H), 4.64 (d, *J =* 3.2 Hz, 1H), 3.72 (d, *J= 18.5* Hz, 3H), 3.67 (d, *J =* 12.0 Hz, 1H), 3.47 (d, *J =* 10.6 Hz, 1H), 2.35 (d, *J =* 7.0 Hz, 2H), 1.62 (s, 4H), 1.34 (dd, *J =* 12.4, 6.5 Hz, 2H), 0.87 (dd, *J =* 20.1, 11.2 Hz, 5H), 0.79 (d, *J =* 6.8 Hz, 6H).

### Example 75. Preparation of (2S,3R,4S,5S)-2-(4-(pyrrolidin-1-ylmethyl) phenoxy)tetrahydro-2H-pyran-3,4,5-triol

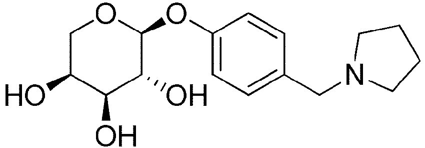

Example 75 was able to obtain the target compound using the same method as Example 1.

¹H NMR (400 MHz, DMSO-d6) δ 7.26 (d, *J =* 8.5 Hz, 2H), 7.03 (d, *J =* 8.6 Hz, 2H), 4.83 (s, 1H), 3.92 (d, *J* = 12.5 Hz, 1H), 3.88 (s, 1H), 3.83 - 3.78 (m, 1H), 3.70 (d, *J =* 12.5 Hz, 1H), 3.63 (dd, *J =* 9.1*,* 3.4 Hz, 1H), 3.57 (s, 2H), 2.52 (s, 4H), 1.80 (s, 4H).

MS(m/z): 310.2 [M+Na]⁺

### Example 76. Preparation of (2S,3R,4S,5S)-2-(4-(morpholinomethyl) phenoxy)tetrahydro-2H-pyran-3,4,5-triol

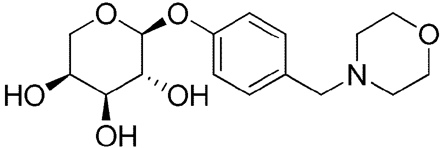

Example 76 was able to obtain the target compound using the same method as Example 4.

¹H NMR (400 MHz, DMSO-d6) δ %) ¹H NMR (400 MHz, dmso) δ 7.20 (d, *J =* 8.5 Hz, 2H), 6.95 (d, *J =* 8.5 Hz, 2H), 5.22 (s, 1H), 4.89 (s, 1H), 4.80 (d, *J =* 6.9 Hz, 1H), 4.66 (s, 1H), 3.69 (d, *J =* 6.6 Hz, 2H), 3.56 (t, *J* = 8.4 Hz, 5H), 3.44 (d, *J =* 8.1 Hz, 1H), 3.38 (s, 2H), 3.17 (d, *J =* 4*.*3 Hz, 1H), 2.32 (s, 4H).

MS(m/z): 326.3 [M+Na]⁺

### Example 77. Preparation of (3S,4R,5R,6R)-5-azido-6-(4-(4-methoxybenzyl)phenoxy)tetrahydro-2H-pyran-3,4-diol

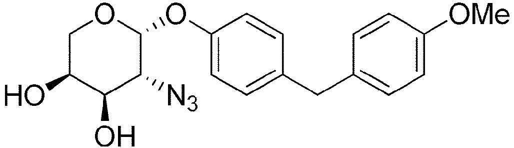

Example 77 was able to obtain the target compound using the same method as Example 69.

¹H NMR (400 MHz, DMSO-d6) δ 7.08 (t, *J* = 9.5 Hz, 4H), 6.99 (d, *J =* 8.6 Hz, 2H), 6.81 (d, *J =* 8.6 Hz, 2H), 5.53 (d, *J =* 3.4 Hz, 1H), 4.18 (dd, *J =* 10.5, 3.2 Hz, 1H), 3.97 - 3.90 (m, 2H), 3.84 (s, 2H), 3.74 (s, 3H), 3.64 (ddd, *J =* 13.8, 11.6, 2.8 Hz, 2H).

### Example 78. Preparation of (3S,4R,5R,6R)-5-azido-6-(4-(4-methoxybenzyl)phenoxy)tetrahydro-2H-pyran-3,4-dio1

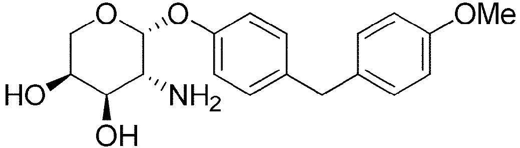

Example 78 was able to obtain the target compound using the same method as Example 70.

¹H NMR (400 MHz, DMSO-d6) δ 7.10 (t, *J =* 9.5 Hz, 4H), 7.01 (d, *J=* 8.6 Hz, 2H), 6.79 (d, *J =* 8.6 Hz, 2H), 5.46 (d, *J =* 3.4 Hz, 1H), 3.95 (dd, *J =* 10.5, 3.2 Hz, 1H), 3.88 - 3.80 (m, 2H), 3.77 (s, 2H), 3.74 (s, 3H), 3.34 (ddd, *J =* 13.8, 11.6, 2.8 Hz, 2H).

### Example 79. Preparation of (2R,3R,4S,5S)-4,5-dihydroxy-2-(4-(4-methoxybenzyl)phenoxy)tetrahydro-2H-pyran-3-yl 10-aminodecanoate

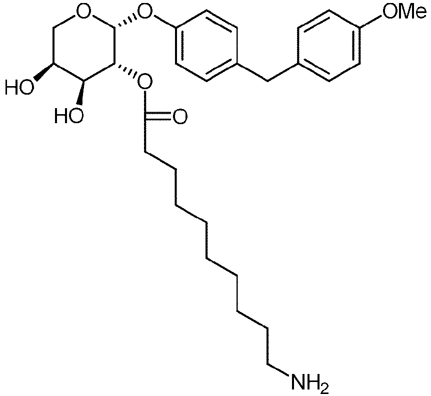

Example 79 was able to obtain the target compound using the same method as Example 62.

¹H NMR (400 MHz, DMSO-d6) δ 7.08 (d, *J =* 8.1 Hz, 4H), 6.89 (d, *J =* 8.4 Hz, 2H), 6.84 - 6.77 (m, 2H), 5.52 (d, *J* = 3.5 Hz, 1H), 4.98 (dd, *J =* 10.1, 3.5 Hz, 1H), 3.96 (dd, *J =* 10.3, 3.3 Hz, 1H), 3.75 (q, *J* = 12.8 Hz, 4H), 3.68 (s, 3H), 3.51 (dd, *J =* 12.3, 2.1 Hz, 1H), 2.85 (q,*J* = 6.6 Hz, 2H), 2.27 (t, *J =* 7.2 Hz, 2H), 1.52 - 1.42 (m, 2H), 1.36 - 1.27 (m, 6H), 1.15 - 1.11 (m, 8H).

MS(m/z): 516.2 [M+H]⁺

### Example 80. Preparation of (2R,3R,4S,SS)-2-((4'-methoxy-[1,1'-biphenyl]-3-yl)oxy)tetrahydro-2H-pyran-3,4,5-triol

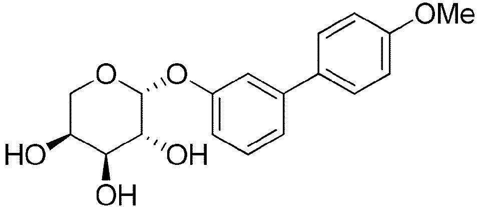

Example 80 was able to obtain the target compound using the same method as in Preparation Example 6.

¹H NMR (400 MHz, DMSO-d6) δ 7.57 (d, *J =* 8.7 Hz, 2H), 7.32 (t, *J =* 7.8 Hz, 1H), 7.22 (d, *J* = 8.6 Hz, 2H), 7.03 - 6.93 (m, 3H), 5.52 (s, 1H), 4.93 (d, *J =* 5.0 Hz, 1H), 4.76 (s, 1H), 4.66 (s, 1H), 3.77 (bs, 6H), 3.72 (d, *J =* 12.0 Hz, 1H), 3.50 (d, *J=* 11.8 Hz, 1H).

### Example 81. Preparation of (2R,3R,4S,5S)-2-(4-(6-methoxybenzofuran-2-yl)phenoxy)tetrahydro-2H-pyran-3,4,5-triol

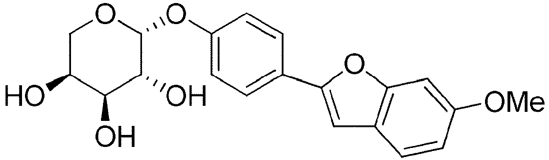

Example 81 was able to obtain the target compound using the same method as in Example 1.

¹H NMR (400 MHz, DMSO-d6) δ 7.77 (d, *J =* 8.8 Hz, 2H), 7.50 - 7.42 (m, 1H), 7.21 (s, 1H), 7.19 - 7.08 (m, 3H), 6.89 - 6.82 (m, 1H), 5.50 (s, 1H), 4.97 (s, 1H), 4.76 (s, 1H), 4.67 (s, 1H), 3.89 (s, 1H), 3.79 (bs, 5H), 3.68 (d, *J =* 11.7 Hz, 1H), 3.51 (d, *J =* 10.0 Hz, 1H).

### Example 82. Preparation of (2R,3R,4S,5S)-2-(4-(5-methoxy-1H-indol-1-yl)phenoxy)tetrahydro-2H-pyran-3,4,5-triol

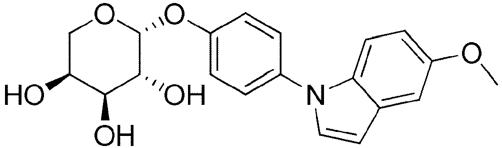

Example 82 was able to obtain the target compound using the same method as in Preparation Example 6.

¹H NMR (400 MHz, DMSO-d6) δ 7.49 (d, *J* = 3.0 Hz, 1H), 7.45 (d, *J* = 8.8 Hz, 2H), 7.36 (d, *J =* 8.9 Hz, 1H), 7.21 (d, *J* = 8.8 Hz, 2H), 7.13 (d, *J* = 2.1 Hz, 1H), 6.80 (dd, *J =* 8.9, 2.3 Hz, 1H), 6.56 (d, *J* = 2.7 Hz, 1H), 5.50 (s, 1H), 3.84 - 3.79 (m, 3H), 3.78 - 3.73 (m, 4H), 3.55 (dd, *J =* 11.7, 1.7 Hz, 1H).

### Example 83. Preparation of (2S,3R,4S,5S)-2-(4-(5-methoxy-1H-indol-1-yl) phenoxy)tetrahydro-2H-pyran-3,4,5-triol

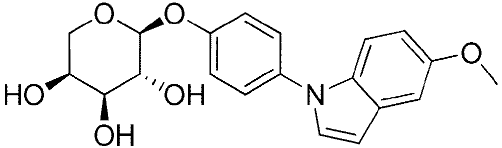

Example 83 was able to obtain the target compound using the same method as Example 82.

¹H NMR (400 MHz, CD₃OD) δ 7.49 (d, *J* = 8.8 Hz, 2H), 7.43 (d, *J* = 3.2 Hz, 1H), 7.41 (d, *J =* 9.0 Hz, 1H), 7.28 (d, *J =* 8.8 Hz, 2H), 7.17 (d, *J =* 2.2 Hz, 1H), 6.87 (dd, *J =* 9.0*,* 2.3 Hz, 1H), 6.61 (d, *J =* 2.8 Hz, 1H), 4.95 (d, *J* = 7.1 Hz, 1H), 3.96 (dd, *J* = 12.3, 2.5 Hz, 1H), 3.90 (s, 1H), 3.86 (s, 3H), 3.84 - 3.81 (m, 1H), 3.79 - 3.73 (m, 2H), 3.66 (dd, *J =* 9.0, 3.4 Hz, 1H).

### Example 84. Preparation of (2R,3R,4S,5S)-2-((4'-methoxy-[1,1'-biphenyl]-4-yl)oxy)tetrahydro-2H-pyran-3,4,5-triol

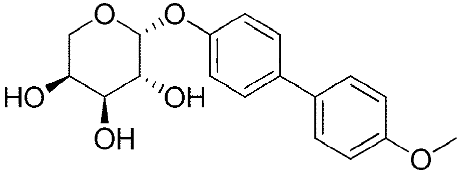

Example 84 was able to obtain the target compound using the same method as Example 80.

¹H NMR (400 MHz, DMSO-d6) δ 7.51 (dd, *J* = 8.8, 2.9 Hz, 4H), 7.08 (d, *J =* 8.7 Hz, 2H), 6.97 (d, *J =* 8.8 Hz, 2H), 5.45 (d, *J =* 2.2 Hz, 1H), 3.80 (d, *J =* 9.4 Hz, 3H), 3.74 (s, 3H), 3.69 (d, *J* = 11.8 Hz, 1H), 3.50 (d, *J =* 10.4 Hz, 1H).

### Example 85. Preparation of (2R,3R,4S,5S)-2-(4-(6-methoxypyridin-3-yl)phenoxy)tetrahydro-2H-pyran-3,4,5-triol

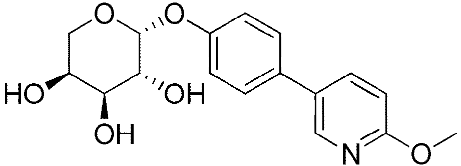

Example 85 was able to obtain the target compound using the same method as Example 80.

¹H NMR (400 MHz, DMSO-d6) δ 8.38 (d, *J =* 1.9 Hz, 1H), 7.94 (dd, *J =* 8.6, 2.5 Hz, 1H), 7.56 (d, *J =* 8.7 Hz, 2H), 7.11 (d, *J =* 8.7 Hz, 2H), 6.87 (d, *J =* 8.7 Hz, 1H), 5.47 (s, 1H), 3.79 (s, 3H), 3.72 (s, 3H), 3.69 (d, *J =* 12.2 Hz, 1H), 3.50 (dd, *J =* 11.9, 1.8 Hz, 1H).

### Example 86. Preparation of (2R,3R,4S,5S)-2-((4'-isopropyl-[1,1'-biphenyl]-4-yl) oxy) tetrahydro-2 H-pyran -3,4,5-triol

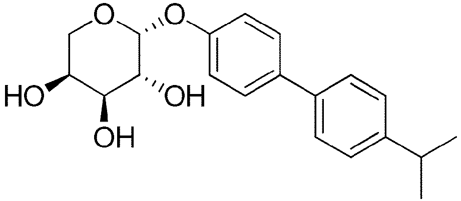

Example 86 was able to obtain the target compound using the same method as Example 80.

¹H NMR (400 MHz, CD₃OD) δ 7.62 (d, *J =* 8.7 Hz, 2H), 7.58 (d, *J =* 8.2 Hz, 2H), 7.35 (d, *J* = 8.1 Hz, 2H), 7.22 (d, *J =* 8.7 Hz, 2H), 5.60 (d, *J=* 2.9 Hz, 1H), 3.97 (dd, *J =* 7.3, 4.1 Hz, 3H), 3.92 (d, *J =* 12.8 Hz, 1H), 3.68 (d, *J =* 10.9 Hz, 1H), 3.02 - 2.94 (m, 1H), 1.32 (d, *J* = 6.9 Hz, 6H).

### Example 87. Preparation of (2S,3R,4S,5S)-2-((4'-isopropyl-[1,1'-biphenyl]-4-yl)oxy)tetrahydro-2H-pyran-3,4,5-triol

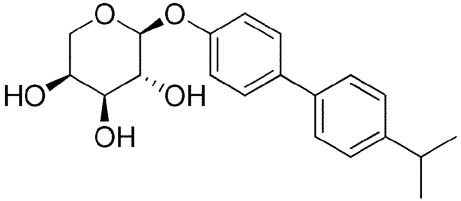

Example 87 was able to obtain the target compound using the same method as Example 80.

¹H NMR (400 MHz, CD₃OD) δ 7.60 (d, *J* = 8.8 Hz, 2H), 7.56 (d, *J* = 8.2 Hz, 2H), 7.34 (d, *J =* 8.1 Hz, 2H), 7.17 (d, *J=* 8.7 Hz, 2H), 4.93 (d, *J =* 7.1 Hz, 1H), 3.93 (d, *J=* 12.3 Hz, 1H), 3.88 (s, 1H), 3.83 - 3.78 (m, 1H), 3.74 (d, *J =* 11.4 Hz, 1H), 3.64 (dd, *J=* 9.0, 3.4 Hz, 1H), 2.97 (dt, *J =* 14.1, 7.1 Hz, 1H), 1.31 (d, *J =* 6.9 Hz, 6H).

### Example 88. Preparation of (2R,3R,4S,5S)-2-(4-(6-isopropylbenzo[d]oxazol-2-yl)phenoxy)tetrahydro-2H-pyran-3,4,5-triol

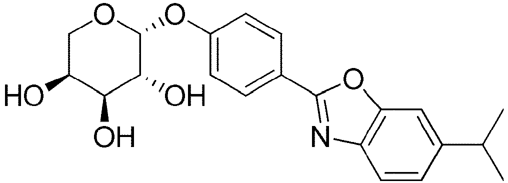

Example 88 was able to obtain the target compound using the same method as Example 1.

¹H NMR (400 MHz, CD₃OD) δ 8.21 (d, *J =* 8.9 Hz, 2H), 7.67 (d, *J =* 8.1 Hz, 1H), 7.61 (s, 1H), 7.35 (d, *J =* 8.9 Hz, 3H), 5.71 (d, *J =* 1.0 Hz, 1H), 4.00 (s, 2H), 3.96 (s, 1H), 3.88 (d, *J =* 11.5 Hz, 1H), 3.70 (dd, *J =* 12.4, 2.2 Hz, 1H), 3.12 (dt, *J* = 13.7, 7.0 Hz, 1H), 1.36 (d, *J =* 6.9 Hz, 6H).

### Example 89. Preparation of (2S,3R,4S,5S)-2-(4-(6-isopropylbenzo[d]oxazol-2-yl)phenoxy)tetrahydro-2H-pyran-3,4,5-triol

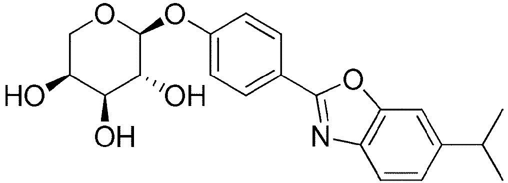

Example 89 was able to obtain the target compound using the same method as Example 1.

¹H NMR (400 MHz, CD₃OD) δ 8.15 (d, *J* = 8.9 Hz, 2H), 7.59 (d, *J* = 8.2 Hz, 1H), 7.53 (s, 1H), 7.29 (d, *J =* 8.2 Hz, 1H), 7.25 (d, *J* = 8.9 Hz, 2H), 5.00 (d, *J=* 7.1 Hz, 1H), 3.96 (dd, *J* = 12.3, 2.6 Hz, 1H), 3.90 (s, 1H), 3.89 - 3.83 (m, 1H), 3.78 (d, *J =* 11.3 Hz, 1H), 3.66 (dd, *J =* 9.0, 3.4 Hz, 1H), 3.07 (dt, *J* = 13.8, 7.1 Hz, 1H), 1.32 (d, *J =* 6.9 Hz, 6H).

### Example 90. Preparation of (2R,3R,4S,5S)-2-(4-(5-methyl-1H-indol-1-yl) phenoxy)tetrahydro-2H-pyran-3,4,5-triol

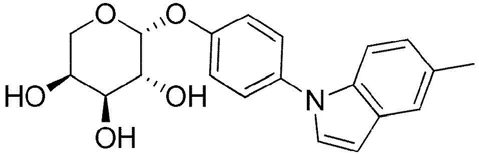

Example 90 was able to obtain the target compound using the same method as Example 82.

¹H NMR (400 MHz, DMSO-d6) δ 7.50 (d, *J* = 3.1 Hz, 1H), 7.46 (d, *J* = 8.9 Hz, 2H), 7.40 (s, 1H), 7.35 (d, *J =* 8.4 Hz, 1H), 7.20 (d, *J =* 8.9 Hz, 2H), 6.98 (d, *J =* 8.3 Hz, 1H), 6.54 (d, *J =* 3.1 Hz, 1H), 5.49 (s, 1H), 4.98 (d, *J =* 5.1 Hz, 1H), 4.77 (d, *J =* 4.4 Hz, 1H), 4.69 (d, *J =* 3.5 Hz, 1H), 3.79 (s, 3H), 3.72 (d, *J =* 11.7 Hz, 1H), 3.53 (d, *J =* 9.8 Hz, 1H), 2.37 (s, 3H).

### Example 91. Preparation of (2R,3R,4S,5S)-2-(4-(5-methoxybenzo[d] oxazol-2-yl)phenoxy)tetrahydro-2H-pyran-3,4,5-triol

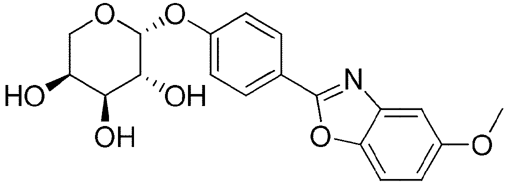

Example 91 was able to obtain the target compound using the same method as Example 1.

¹H NMR (400 MHz, CD₃OD) δ 8.20 (d, *J* = 8.9 Hz, 2H), 7.60 (d, *J* = 8.8 Hz, 1H), 7.34 (d, *J =* 8.9 Hz, 2H), 7.29 (d, *J =* 2.3 Hz, 1H), 7.03 (dd, *J =* 8.9, 2.5 Hz, 1H), 5.70 (s, 1H), 4.00 (s, 2H), 3.96 (s, 1H), 3.88 (d, *J =* 15.2 Hz, 4H), 3.70 (dd, *J =* 12.3, 2.2 Hz, 1H).

### Example 92. Preparation of (2S,3R,4S,5S)-2-(4-(5-methoxybenzo[d]oxazol-2-yl)phenoxy)tetrahydro-2H-pyran-3,4,5-triol

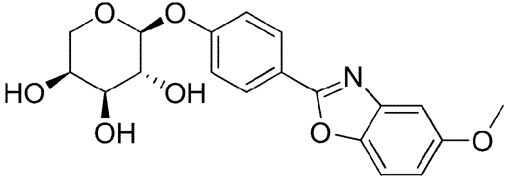

Example 92 was able to obtain the target compound using the same method as Example 1.

¹H NMR (400 MHz, CD₃OD) δ 8.19 (d, *J =* 8.9 Hz, 2H), 7.60 (d, *J =* 8.9 Hz, 1H), 7.29 (d, *J* = 8.8 Hz, 3H), 7.02 (dd, *J =* 8.9, 2.5 Hz, 1H), 5.04 (d, *J =* 7.1 Hz, 1H), 3.95 (dd, *J =* 12.3, 2.6 Hz, 1H), 3.90 (s, 4H), 3.84 (dd, *J =* 8.8, 7.3 Hz, 1H), 3.80 (d, *J =* 12.2 Hz, 1H), 3.66 (dd, *J =* 9.0, 3.4 Hz, 1H).

### Example 93 Preparation of (2R,3R,4S,5R)-2-(4-(5-methoxybenzo[d]oxazol-2-yl)phenoxy)tetrahydro-2H-pyran-3,4,5-triol

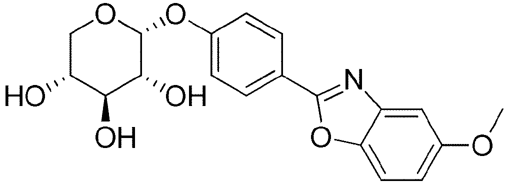

Example 93 was able to obtain the target compound using the same method as Example 1.

¹H NMR (400 MHz, CD₃OD) δ 8.21 (d, *J* = 8.8 Hz, 2H), 7.60 (d, *J* = 9.0 Hz, 1H), 7.34 (d, *J* = 8.9 Hz, 2H), 7.28 (d, *J =* 2.3 Hz, 1H), 7.03 (dd, *J =* 8.9, 2.5 Hz, 1H), 5.66 (d, *J =* 3.4 Hz, 1H), 3.91 (s, 3H), 3.83 - 3.78 (m, 1H), 3.68 (dd, *J =* 9.8, 4.8 Hz, 1H), 3.63 - 3.56 (m, 2H), 3.53 (d, *J* = 10.0 Hz, 1H).

### Example 94. Preparation of (2S,3R,4S,5R)-2-(4-(5-methoxybenzo[d]oxazol-2-yl)phenoxy)tetrahydro-2H-pyran-3,4,5-triol

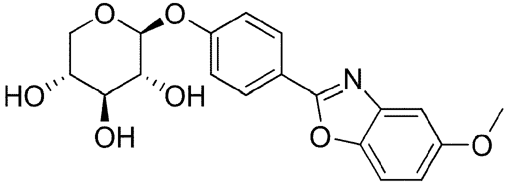

Example 94 was able to obtain the target compound using the same method as Example 1.

¹H NMR (400 MHz, CD₃OD) δ 8.19 (d, *J =* 8.9 Hz, 2H), 7.59 (d, *J =* 8.8 Hz, 1H), 7.27 (dd, *J* = 5.6, 3.1 Hz, 3H), 7.02 (dd, *J =* 8.9, 2.5 Hz, 1H), 5.06 (d, *J =* 7.0 Hz, 1H), 3.96 (dd, *J =* 11.2, 5.1 Hz, 1H), 3.90 (s, 3H), 3.59 (dd, *J =* 15.9, 7.3 Hz, 1H), 3.51 - 3.42 (m, 3H).

### Example 95 Preparation of (2R,3R,4S,5S)-2-(4-(6-ethylbenzofuran-3-yl)phenoxy) tetrahydro-2H-pyra-3,4,5-triol

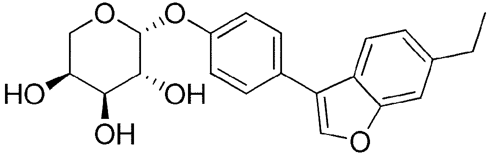

Example 95 was able to obtain the target compound using the same method as Example 1.

¹H NMR (400 MHz, CD₃OD) δ 7.98 (s, 1H), 7.78 (d, *J* = 8.0 Hz, 1H), 7.67 (d, *J* = 8.7 Hz, 2H), 7.42 (s, 1H), 7.26 (d, *J* = 8.8 Hz, 2H), 7.22 (d, *J* = 8.6 Hz, 1H), 5.61 (d, *J* = 3.0 Hz, 1H), 4.01 - 3.92 (m, 4H), 3.69 (d, *J* = 10.4 Hz, 1H), 2.81 (q, *J* = 7.6 Hz, 2H), 1.32 (t, *J* = 7.6 Hz, 3H).

### Example 96. Preparation of (2S,3R,4S,5S)-2-(4-(6-ethylbenzofuran-3-yl) phenoxy)tetrahydro-2H-pyran-3,4,5-triol

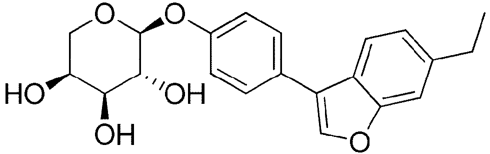

Example 96 was able to obtain the target compound using the same method as Example 1.

¹H NMR (400 MHz, CD₃OD) δ 8.01 (s, 1H), 7.79 (d, *J* = 8.1 Hz, 1H), 7.68 (d, *J* = 8.7 Hz, 2H), 7.44 (s, 1H), 7.23 (dd, *J* = 7.7, 4.4 Hz, 3H), 4.95 (d, *J* = 7.1 Hz, 1H), 3.95 (dd, *J* = 12.3, 2.5 Hz, 1H), 3.89 (s, 1H), 3.85 - 3.80 (m, 1H), 3.75 (d, *J* = 11.5 Hz, 1H), 3.65 (dd, *J* = 9.1, 3.4 Hz, 1H), 2.81 (q, *J* = 7.6 Hz, 2H), 1.32 (t, *J* = 7.6 Hz, 3H).

### Example 97. Preparation of (2R,3R,4S,5S)-2-(3-(7-isopropylbenzo[d]oxazol-2-yl)phenoxy)tetrahydro-2H-pyran-3,4,5-triol

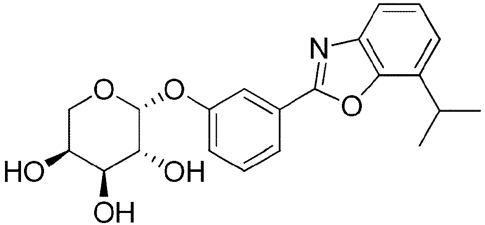

Example 97 was able to obtain the target compound using the same method as Example 1.

¹H NMR (400 MHz, CD₃OD) δ 8.02 (s, 1H), 7.94 (d, *J* = 7.7 Hz, 1H), 7.62 (dd, *J* = 7.8, 0.9 Hz, 1H), 7.58 (t, *J* = 8.0 Hz, 1H), 7.38 (ddd, *J* = 26.3, 11.3, 4.8 Hz, 3H), 5.69 (d, *J* = 2.7 Hz, 1H), 4.02 (t, *J* = 3.3 Hz, 2H), 3.99 - 3.93 (m, 2H), 3.73 (dd, *J* = 12.2, 1.9 Hz, 1H), 3.48 (dt, *J* = 13.7, 7.0 Hz, 1H), 1.49 (d, *J* = 6.9 Hz, 6H).

### Example 98. Preparation of (2S,3R,4S,5S)-2-(3-(7-isopropylbenzo[d]oxazol-2-yl)phenoxy)tetrahydro-2H-pyran-3,4,5-triol

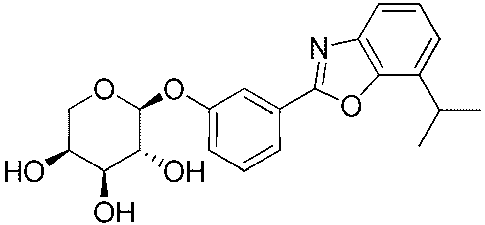

Example 98 was able to obtain the target compound using the same method as Example 1.

¹H NMR (400 MHz, CD₃OD) δ 7.96 (s, 1H), 7.94 (d, *J* = 7.9 Hz, 1H), 7.61 (dd, *J* = 7.8, 0.9 Hz, 1H), 7.57 (t, *J* = 7.9 Hz, 1H), 7.35 (ddd, *J* = 13.7, 11.8, 7.5 Hz, 3H), 5.01 (d, *J* = 7.1 Hz, 1H), 4.01 - 3.97 (m, 1H), 3.92 (s, 1H), 3.89 - 3.85 (m, 1H), 3.81 (d, *J* = 11.8 Hz, 1H), 3.68 (dd, *J* = 9.1, 3.3 Hz, 1H), 3.47 (dt, *J* = 13.9, 6.9 Hz, 1H), 1.49 (d, *J* = 7.0 Hz, 6H).

### Example 99. Preparation of (2R,3R,4S,5S)-2-(2-chloro-4-(5-methylbenzo [d]oxazol-2-yl)phenoxy)tetrahydro-2H-pyran-3,4,5-triol

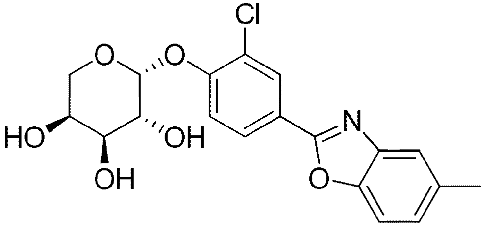

Example 99 was able to obtain the target compound using the same method as Example 1.

¹H NMR (400 MHz, CD₃OD) δ 8.31 - 8.26 (m, 1H), 8.18 (dd, *J* = 8.2, 1.3 Hz, 1H), 7.64 - 7.59 (m, 2H), 7.55 (d, *J* = 8.7 Hz, 1H), 7.30 (d, *J* = 8.0 Hz, 1H), 5.90 (d, *J* = 1.4 Hz, 1H), 4.08 (s, 2H), 3.99 (s, 1H), 3.90 (d, *J* = 12.7 Hz, 1H), 3.71 (d, *J* = 12.5 Hz, 1H), 2.53 (s, 3H).

### Example 100. Preparation of (2S,3R,4S,5S)-2-(2-chloro-4-(5-methylbenzo[d]oxazol-2-yl)phenoxy) tetrahydro-2H-pyran-3,4,5-triol

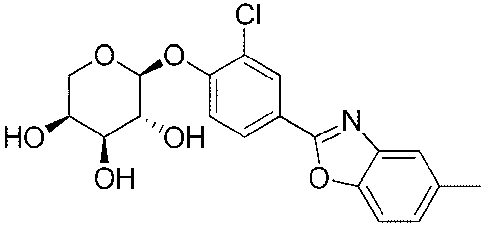

Example 100 was able to obtain the target compound using the same method as Example 1.

¹H NMR (400 MHz, CD₃OD) δ 8.28 (d, *J* = 2.0 Hz, 1H), 8.19 (dd, *J* = 8.7, 2.0 Hz, 1H), 7.67 (d, *J* = 8.4 Hz, 1H), 7.63 (s, 1H), 7.52 (d, *J* = 8.8 Hz, 1H), 7.31 (d, *J* = 8.4 Hz, 1H), 5.21 (d, *J* = 6.9 Hz, 1H), 3.93 (dd, *J* = 12.1, 3.1 Hz, 1H), 3.90 - 3.85 (m, 2H), 3.79 (d, *J* = 11.4 Hz, 1H), 3.66 (dd, *J* = 8.6, 3.3 Hz, 1H), 2.54 (s, 3H).

### Example 101. Preparation of (2R,3R,4S,5R)-2-(2-fluoro-4-(4-isopropylphenoxy)phenoxy)tetrahydro-2H-pyran-3,4,5-triol

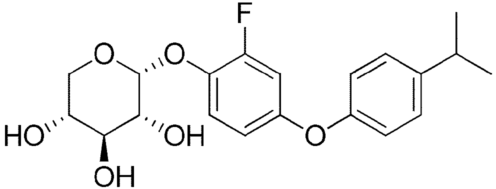

Example 101 was able to obtain the target compound using the same method as Example 1.

¹H NMR (400 MHz, DMSO-d6) δ7.23 (dt, *J =* 9.2, 5.0 Hz, 3H), 6.97 - 6.90 (m, 3H), 6.73 (ddd, *J* = 9.0, 2.9, 1.5 Hz, 1H), 5.37 (d, *J* = 3.6 Hz, 1H), 5.14 (d, *J* = 5.8 Hz, 1H), 5.04 (s, 1H), 5.01 (d, *J* = 4.9 Hz, 1H), 3.58 - 3.34 (m, 5H), 2.86 (p, *J* = 6.9 Hz, 1H), 1.17 (d, *J* = 6.9 Hz, 6H).

### Example 102. Preparation of (2S,3R,4S,5S)-2-(2-chloro-4-(5-methoxybenzo[d] oxazol-2-yl)phenoxy)tetrahydro-2H-pyran-3,4,5-triol

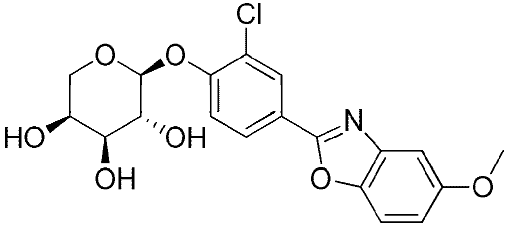

Example 102 was able to obtain the target compound using the same method as Example 1.

¹H NMR (400 MHz, CD₃OD) δ 8.26 (d, *J* = 2.0 Hz, 1H), 8.16 (dd, *J* = 8.7, 2.0 Hz, 1H), 7.64 (d, *J* = 8.9 Hz, 1H), 7.49 (d, *J* = 8.8 Hz, 1H), 7.32 (d, *J* = 2.3 Hz, 1H), 7.06 (dd, *J* = 8.9, 2.5 Hz, 1H), 5.19 (d, *J* = 6.8 Hz, 1H), 4.00 - 3.88 (m, 6H), 3.81 (d, *J* = 11.8 Hz, 1H), 3.69 (dd, *J* = 8.7, 3.3 Hz, 1H).

### Example 103. Preparation of (2R,3R,4S,5S)-2-(2-chloro-4-(5-methoxybenzo[d] oxazol-2-yl)phenoxy)tetrahydro-2H-pyran-3,4,5-triol

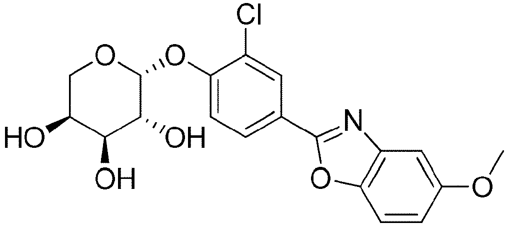

Example 103 was able to obtain the target compound using the same method as Example 1.

¹H NMR (400 MHz, CD₃OD) δ8.24 (d, *J* = 2.0 Hz, 1H), 8.14 (dd, *J* = 8.7, 2.1 Hz, 1H), 7.60 (d, *J* = 8.9 Hz, 1H), 7.51 (d, *J* = 8.8 Hz, 1H), 7.28 (d, *J* = 2.4 Hz, 1H), 7.03 (dd, *J* = 8.9*,* 2.5 Hz, 1H), 5.87 (d, *J =* 2.8 Hz, 1H), 4.12 - 4.04 (m, 2H), 3.99 (s, 1H), 3.91 (d, *J* = 9.7 Hz, 4H), 3.71 (dd, *J* = 12.3, 1.9 Hz, 1H).

### Example 104. Preparation of (2S,3R,4S,5S)-2-(2-fluoro-4-(5-methylbenzo[d]oxazol-2-yl)phenoxy)tetrahydro-2H-pyran-3,4,5-triol

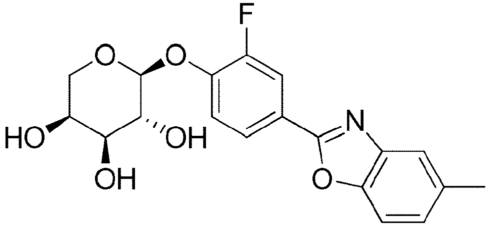

Example 104 was able to obtain the target compound using the same method as Example 1.

¹H NMR (400 MHz, CD₃OD) δ 8.04 (t, *J* = 9.2 Hz, 2H), 7.66 - 7.56 (m, 2H), 7.51 (t, *J* = 8.6 Hz, 1H), 7.30 (d, *J* = 8.4 Hz, 1H), 5.14 (d, *J* = 7.0 Hz, 1H), 3.97 (dd, *J* = 12.2, 2.7 Hz, 1H), 3.93 - 3.86 (m, 2H), 3.80 (d, *J* = 12.1 Hz, 1H), 3.68 (dd, *J* = 8.8, 3.4 Hz, 1H), 2.53 (s, 3H).

### Example 105. Preparation of (2R,3R,4S,5S)-2-(2-fluoro-4-(5-methylbenzo[d]oxazol-2-yl)phenoxy)tetrahydro-2H-pyran 3,4,5-triol

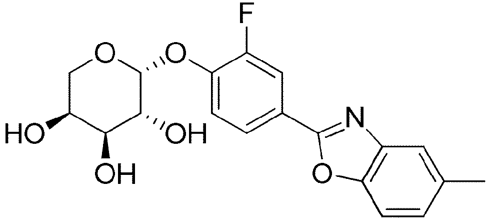

Example 105 was able to obtain the target compound using the same method as Example 1.

¹H NMR (400 MHz, CD₃OD) δ 8.03 (dd, *J =* 15.6, 5.1 Hz, 2H), 7.66 - 7.50 (m, 3H), 7.30 (d, *J* = 8.2 Hz, 1H), 5.79 (d, *J* = 1.7 Hz, 1H), 4.08 - 3.90 (m, 4H), 3.73 (dd, *J* = 12.3, 2.0 Hz, 1H), 2.53 (s, 3H).

### Example 106. Preparation of (2S,3R,4S,5S)-2-(3-(5-methoxybenzo[d]oxazol-2-yl)phenoxy)tetrahydro-2H-pyran-3,4,5-triol

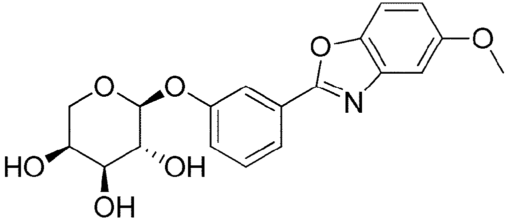

Example 106 was able to obtain the target compound using the same method as Example 1.

¹H NMR (400 MHz, CD₃OD) δ 7.96 (d, *J* = 7.9 Hz, 2H), 7.73 (d, *J* = 8.8 Hz, 1H), 7.63 (t, *J* = 8.1 Hz, 1H), 7.43 - 7.36 (m, 2H), 7.13 (dd, *J* = 8.8, 2.6 Hz, 1H), 5.07 (d, *J* = 7.0 Hz, 1H), 3.98 (d, *J* = 15.5 Hz, 4H), 3.91 (s, 1H), 3.84 (dd, *J* = 16.1, 9.7 Hz, 2H), 3.69 (dd, *J* = 8.8, 3.4 Hz, 1H).

### Example 107. Preparation of (2R,3R,4S,5S)-2-(3-(5-methoxybenzo[d]oxazol-2-yl)phenoxy)tetrahydro-2H-pyran-3,4,5-triol

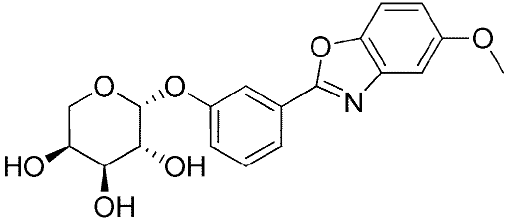

Example 107 was able to obtain the target compound using the same method as Example 1.

¹H NMR (400 MHz, CD₃OD) δ 8.01 (s, 1H), 7.96 (d, *J* = 7.8 Hz, 1H), 7.74 (d, *J* = 8.9 Hz, 1H), 7.63 (t, *J* = 8.0 Hz, 1H), 7.43 (d, *J* = 2.5 Hz, 2H), 7.13 (dd, *J* = 8.9, 2.5 Hz, 1H), 5.72 (d, *J* = 2.0 Hz, 1H), 4.02 (s, 2H), 3.99 - 3.91 (m, 5H), 3.74 (dd, *J* = 12.2, 2.0 Hz, 1H).

### Example 108. Preparation of (2S,3R,4S,5S)-2-(4-(5-methylbenzo[d]oxazol-2-yl)phenoxy)tetrahydro-2H-pyran-3,4,5-triol

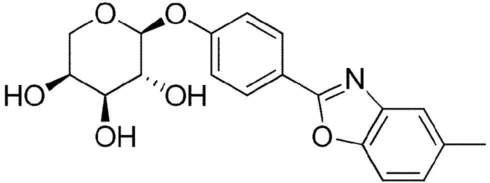

Example 108 was able to obtain the target compound using the same method as Example 1.

¹H NMR (400 MHz, CD₃OD) δ 8.25 (d, *J* = 8.9 Hz, 2H), 7.65 (d, *J* = 8.4 Hz, 1H), 7.62 (s, 1H), 7.35 - 7.28 (m, 3H), 5.08 (d, *J* = 7.1 Hz, 1H), 3.96 (dd, *J* = 12.2, 2.7 Hz, 1H), 3.90 (s, 1H), 3.82 (dd, *J* = 14.3, 8.3 Hz, 2H), 3.66 (dd, *J* = 8.9, 3.5 Hz, 1H), 2.55 (s, 3H).

### Example 109. Preparation of (2R,3R,4S,5S)-2-(4-(5-methylbenzo[d]oxazol-2-yl)phenoxy)tetrahydro-2H-pyran-3,4,5-triol

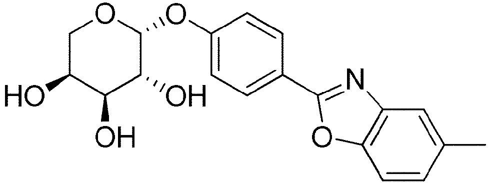

Example 109 was able to obtain the target compound using the same method as Example 1.

¹H NMR (400 MHz, CD₃OD) δ 8.21 (d, *J* = 8.9 Hz, 2H), 7.59 (d, *J* = 8.3 Hz, 1H), 7.56 (s, 1H), 7.34 (d, *J* = 8.9 Hz, 2H), 7.26 (d, *J* = 8.2 Hz, 1H), 5.71 (s, 1H), 4.01 (s, 2H), 3.96 (s, 1H), 3.89 (d, *J* = 12.3 Hz, 1H), 3.70 (dd, *J* = 12.3, 2.1 Hz, 1H).

### Example 110. Preparation of (2R,3R,4S,5S)-2-(4-phenethylphenoxy)tetrahydro-2H-pyran-3,4,5-triol

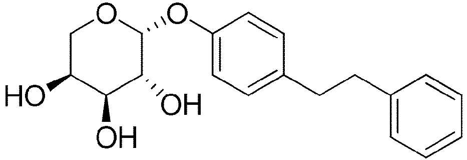

Example 110 was able to obtain the target compound using the same method as Example 1.

*820¹H NMR (400 MHz, DMSO-d6) δ 7.27 - 7.08 (m, 7H), 6.93 - 6.88 (m, 2H), 5.35 (d, *J* = 2.5 Hz, 1H), 4.95 (d, *J* = 5.7 Hz, 1H), 4.75 (d, *J* = 4.7 Hz, 1H), 4.68 (d, *J* = 3.5 Hz, 1H), 3.77 - 3.70 (m, 3H), 3.66 (d, *J* = 11.9 Hz, 1H), 3.47 - 3.44 (m, 1H), 2.87 - 2.73 (m, 4H).

MS(m/z): 331.2 [M+H]⁺

### Example 111. Preparation of (2R,3R,4S,5S)-2-(4-((E)-styryl)phenoxy)tetrahydro-2H-pyran-3,4,5-triol

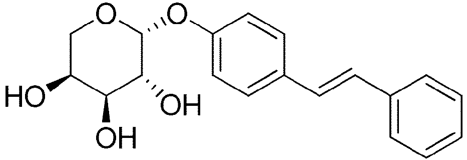

Example 111 was able to obtain the target compound using the same method as Example 1.

¹H NMR (400 MHz, DMSO-d6) δ7.54 (dd, *J =* 10.4, 8.2 Hz, 4H), 7.34 (t, *J* = 7.6 Hz, 2H), 7.25 - 7.20 (m, 1H), 7.17 (s, 1H), 7.11 (s, 1H), 7.03 (d, *J* = 8.7 Hz, 2H), 5.46 (s, 1H), 4.96 (d, *J* = 5.4 Hz, 1H), 4.76 (d, *J* = 4.9 Hz, 1H), 4.68 (d, *J* = 3.6 Hz, 1H), 3.80 - 3.73 (m, 3H), 3.67 (d, *J* = 11.7 Hz, 1H), 3.50 (dd, *J* = 12.0, 2.2 Hz, 1H.

MS(m/z): 351.1 [M+ Na]⁺

### Example 112. Preparation of (2R,3R,4S,5S)-2-(4-(bis(4-methoxyphenyl) methyl)phenoxy)tetrahydro-2H-pyran-3,4,5-triol

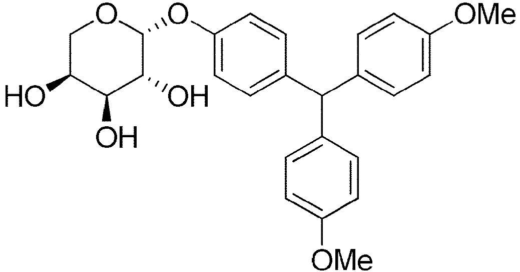

Example 112 was able to obtain the target compound using the same method as Example 1.

MS(m/z): 329.2 [M+H]⁺

### Example 113. Preparation of (2R,3R,4S,5S)-2-((4'-(4-isopropylbenzyl)-[1,1'-biphenyl]-4-yl)oxy)tetrahydro-2H-pyran-3,4,5-triol

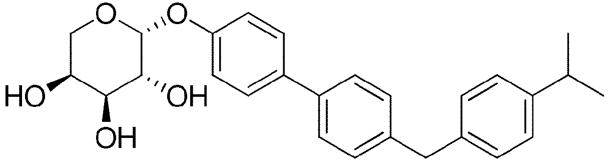

Example 113 was able to obtain the target compound using the same method as Example 1.

¹H NMR (400 MHz, DMSO-d6) δ 7.56 - 7.48 (m, 4H), 7.28 - 7.23 (m, 2H), 7.14 (d, *J* = 1.1 Hz, 4H), 7.10 - 7.05 (m, 2H), 5.46 (d, *J* = 2.4 Hz, 1H), 4.96 (d, *J* = 5.4 Hz, 1H), 4.76 (d, *J* = 4.8 Hz, 1H), 4.67 (d, *J* = 3.6 Hz, 1H), 3.89 (s, 2H), 3.81 - 3.74 (m, 3H), 3.68 (d, *J* = 11.8 Hz, 1H), 3.49 (dd, *J* = 12.0, 2.4 Hz, 1H), 2.81 (p, *J* = 6.9 Hz, 1H), 1.15 (d, *J* = 6.9 Hz, 6H).

MS(m/z): 435.5 [M+H]⁺

### Example 114. Preparation of furan-2-yl (4-(((2S,3R,4S,5S)-3,4,5-trihydroxytetrahydro-2H-pyran-2-yl)oxy)phenyl)methanone

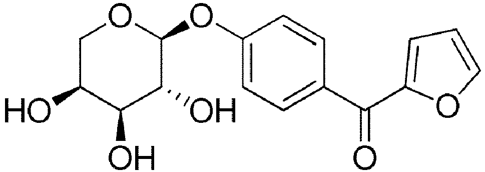

Example 114 was synthesized through Preparation Example 2, and the target compound was obtained by hydrolyzing according to Preparation Example 5 after introducing a substituent in the α/β form.

¹H NMR (400 MHz, DMSO-d6) δ 8.10 (d, *J =* 0.8 Hz, 1H), 7.94 (d, *J =* 8.9 Hz, 2H), 7.40 - 7.37 (m, 1H), 7.16 (d, *J* = 8.8 Hz, 2H), 6.78 (dd, *J* = 3.5, 1.7 Hz, 1H), 5.30 (d, *J* = 5.1 Hz, 1H), 5.01 (d, *J* = 6.8 Hz, 1H), 4.90 (d, *J* = 5.6 Hz, 1H), 4.70 (d, *J* = 4.1 Hz, 1H), 3.75 - 3.63 (m, 4H), 3.51 - 3.47 (m, 1H).

### Example 115. Preparation of furan-2-yl(4-(((2R,3R,4S,5S)-3,4,5-trihydroxytetrahydro-2H-pyran-2-yl)oxy)phenyl)methanone

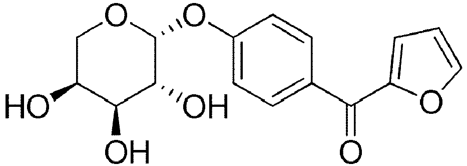

Example 115 was able to obtain the target compound using the same method as Example 114.

¹H NMR (400 MHz, DMSO-d6) δ 8.10 (d, *J* = 0.9 Hz, 1H), 7.94 (d, *J* = 8.7 Hz, 2H), 7.40 - 7.37 (m, 1H), 7.19 (d, *J* = 8.8 Hz, 2H), 6.78 (dd, *J* = 3.5, 1.5 Hz, 1H), 5.61 (d, *J* = 0.9 Hz, 1H), 5.07 - 5.04 (m, 1H), 4.83 (d, *J* = 3.4 Hz, 1H), 4.74 (d, *J* = 3.4 Hz, 1H), 3.80 (d, *J* = 8.4 Hz, 3H), 3.67 (d, *J* = 11.8 Hz, 1H), 3.54 (dd, *J* = 11.9, 2.0 Hz, 1H).

### Example 116. Preparation of (2R,3R,4S,5S)-2-(4-(4-methoxyphenethyl) phenoxy)tetrahydro-2H-pyran-3,4,5-triol

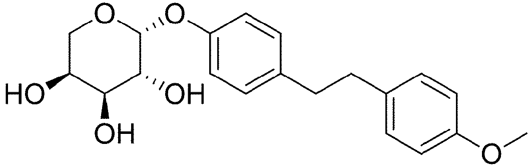

Example 116 was synthesized through step 15 of Preparation Example 1, and the target compound was obtained by hydrolyzing according to Preparation Example 5 after introducing a substituent in the α/β form.

¹H NMR (400 MHz, DMSO-d6) δ7.09 (d, *J* = 8.6 Hz, 4H), 6.94 - 6.87 (m, 2H), 6.85 - 6.76 (m, 2H), 5.36 (d, *J* = 2.4 Hz, 1H), 4.91 (d, *J* = 5.7 Hz, 1H), 4.74 (d, *J* = 4.8 Hz, 1H), 4.65 (d, *J* = 3.5 Hz, 1H), 3.79 - 3.71 (m, 3H), 3.67 (d, *J* = 13.6 Hz, 4H), 3.47 (dd, *J* = 12.0, 2.3 Hz, 1H), 2.75 (s, 4H).

MS(m/z): 361.4 [M+H]⁺

### Example 117. Preparation of (2R,3R,4S,5S)-2-(4-(4-isopropylphenethyl) phenoxy)tetrahydro-2H-pyran-3,4,5-triol

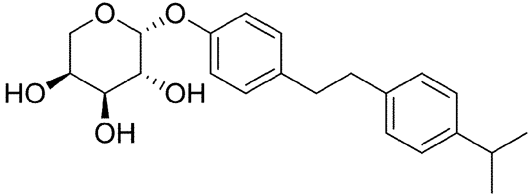

Example 117 was able to obtain the target compound using the same method as in Example 116.

MS(m/z): 373.4 [M+H]⁺

### Example 118. Preparation of (2R,3R,4S,5S)-2-(4-((E)-4-methoxystyryl) phenoxy)tetrahydro-2H-pyran-3,4,5-triol

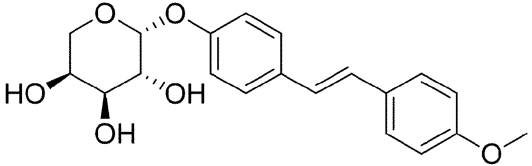

Example 118 was able to obtain the target compound using the same method as in Example 116.

MS(m/z): 359.3 [M+H]⁺

### Example 119. Preparation of (2R,3R,4S,5S)-2-(4-((E)-4-isopropylstyryl) phenoxy)tetrahydro-2H-pyran-3,4,5-triol

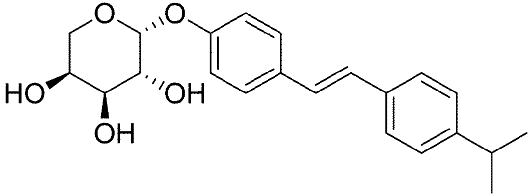

Example 119 was able to obtain the target compound using the same method as in Example 116.

MS(m/z): 371.4 [M+H]⁺

### Example 120. Preparation of (2R,3R,4S,5S)-2-(4-(benzo[d][1,3]dioxol-5-yl) phenoxy)tetrahydro-2H-pyran-3,4,5-triol

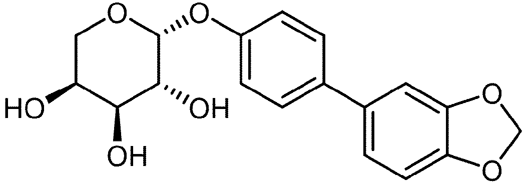

Example 120 was able to obtain the target compound using the same method as in Example 1.

MS(m/z): 347.3 [M+H]⁺

### Example 121. Preparation of (2R,3R,4S,5S)-2-(4-(2,3-dihydrobenzo[b]1,4 ] dioxin-6-yl)phenoxy)tetrahydro-2H-pyran-3,4,5-triol

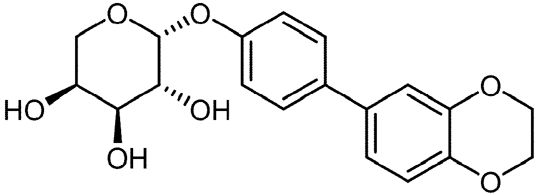

Example 121 was able to obtain the target compound using the same method as in Example 1.

MS(m/z): 361.3 [M+H]⁺

### Example 122. Preparation of (2R,3R,4S,SS)-2-((4'-chloro-[1,1'-biphenyl]-4-yl)oxy)tetrahydro-2H-pyran-3,4,5-triol

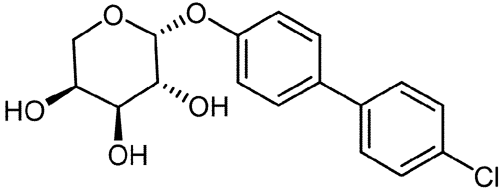

Example 122 was able to obtain the target compound using the same method as in Example 1.

1H NMR (400 MHz, DMSO-d6) δ 7.66 - 7.54 (m, 4H), 7.50 - 7.37 (m, 2H), 7.14 - 7.07 (m, 2H), 5.48 (d, *J* = 2.3 Hz, 1H), 4.98 (d, *J* = 4.5 Hz, 1H), 4.78 (s, 1H), 4.69 (d, *J* = 3.5 Hz, 1H), 3.77 (s, 3H), 3.68 (d, *J* = 11.8 Hz, 1H), 3.50 (dd, *J* = 11.9, 2.5 Hz, 1H).

MS(m/z): 337.7 [M+H]⁺

### Example 123. Preparation of (2R,3R,4S,SS)-2-((4"-isopropyl-[1,1':4',1"-terphenyl-4-yl)oxy)tetrahydro-2H-pyran-3,4,5-triol

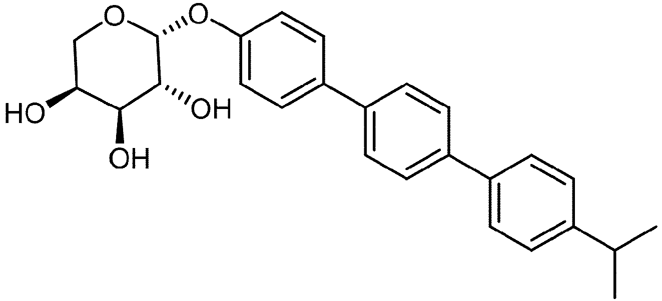

Example 123 was able to obtain the target compound using the same method as in Example 1.

MS(m/z): 421.5 [M+H]⁺

### Example 124. Preparation of (2R,3R,4S,5S)-2-(3-(phenylethynyl)phenoxy) tetrahydro-2H-pyran-3,4,5-triol

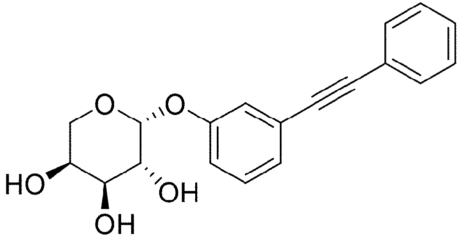

Example 124 was synthesized through step 14 of Preparation Example 1, and the target compound was obtained by hydrolyzing according to Preparation Example 5 after introducing a substituent in the α/β form.

MS(m/z): 327.3 [M+H]⁺

### Example A : Tablets

According to an embodiment of the present invention, a mixture of 1 Kg of a compound active ingredient, 4 kg of lactose, 1.2 kg of potato starch, 0.2 kg of talc, and 0.1 kg of magnesium stearate was compressed in a conventional manner, and each tablet contains 10 mg of an active ingredient to obtain a tablet.

### Example B : Aqueous solutions or suspensions

A solution is prepared from 1 g of the compound active ingredient according to an embodiment of the present invention, 9.38 g of NaH₂PO₄ ° 2 2H₂O, 28.48 g of Na₂HPO₄ ° 12H₂O, and 0.1 g of benzalkonium chloride in 940 ml of water distilled twice. PH is adjusted to 6.8, 1 L of solution is prepared, and sterilized by irradiation. The solution can be used in the form of eye drops.

### Example C : injection solution

A solution of 100 g of the compound active ingredient and 5 g of disodium hydrogen phosphate according to an embodiment of the present invention is adjusted to pH 6.5 using 2N hydrochloric acid in 3 L of water distilled twice, sterilized and filtered, moved to an injection vial, freeze-dried under sterilization conditions, and sealed under sterilization conditions. Each injection vial contains 5 mg of an active ingredient.

### Experimental Example 1: In Vitro Medicinal Efficacy: HRE (Hypoxia Response Element) luciferase assay

It has been reported that in the brains of AD patients, blood supply is mostly insufficient, resulting in oxygen levels being up to 50% lower than those of normal individuals, leading to increased oxidative stress (ROS) and neuroinflammation. It has also been studied that when the HIF-1α (hypoxia inducible factor-1) factor binds to the HRE (hypoxia response element), it increases VEGF (vascular endothelial growth factor), which promotes the formation of new blood vessels, thereby increasing oxygen levels in the brain and helping to inhibit nerve cell damage.

A reporter gene containing luciferase, capable of verifying the level of HRE expression, was inserted into HEK-293 human embryonic kidney cells, and HRE/Luc/HEK293 cells were constructed by inserting a plasmid (HRE/Luc) with luciferase attached downstream of the HRE promoter that binds to HIF-1α using lipofectamine (Invitrogen).

To perform HRE luciferase analysis, HRE/Luc/HEK293 cells were seeded in 96-well culture plates at a cell density of 4 x 10⁴ per well in minimal essential medium (MEM) containing 10% fetal bovine serum. After one day, the cell medium was removed and replaced with medium mixed with various concentrations of example compounds. Six hours after compound pretreatment, HRE/Luc/HEK293 cells were cultured under hypoxic conditions (O₂ 0.1%) for 18 hours. Subsequently, the cells were washed with phosphate buffer, lysed with lysis buffer, and then the luciferase reagent (Promega) for luciferase analysis was added. Afterward, changes in HRE expression levels associated with HIF-1 signaling were evaluated by measuring luminescence using an Infinite200 (Tecan).

Changes in HRE expression levels for the compounds of the present invention (HRE luciferase assay results) are as described in Table 2 below. As a result of evaluating 35 compounds among Examples 1 to 36, a number of compounds were identified that induced more than a twofold increase in HRE activity at the same treatment concentration (100 µM); in particular, the compounds of Examples 7, 8, 27, 33, 35, and 36 were confirmed to induce HRE expression at a high level of more than threefold at a low concentration of 10 µM (Table 2). In addition, as a result of evaluating 69 compounds among Examples 37-109, numerous compounds were identified that induced more than a threefold increase in HRE activity at the same treatment concentration (10 µM); in particular, the compounds of Examples 41, 43, 57, 67, 68, 92, 94, 102, 104, 106, 107, 108, and 109 were confirmed to induce HRE expression at a high level of more than threefold even at a low concentration of 1 µM. (Table 2)

**[Table 2]**

| Example | HRE-increasing | | Example | HKt-increasing | | Example | HRE-inceasing | |
|---|---|---|---|---|---|---|---|---|
| | 100uM | 10uM | | 100uM | 10uM | | 100uM | 10uM |
| 1 | 2.2 | 1.2 | 37 | 1.3 | 1.1 | 75 | 1.0 | 1.0 |
| 2 | 3.2 | 2.5 | 38 | 2.2 | 1.3 | 76 | 1.0 | <1.0 |
| 3 | 3.1 | 1.5 | 39 | 1.3 | 1.1 | 77 | 1.3 | 1.0 |
| 4 | <1 | 1.1 | 40 | 3.1 | 2.6 | 78 | 2.4 | 12 |
| 5 | 1.9 | 1.2 | 41 | 3.2 | 3 | 79 | 1.7 | 1.0 |
| 6 | 1.5 | <1 | 42 | 3.1 | 2.4 | 80 | - | - |
| 7 | <1 | 3.5 | 43 | 2.5 | 3.3 | 81 | 3.6 | 2.7 |
| 8 | - | 4.2 | 44 | 1.3 | 1.1 | 82 | 2.3 | 1.4 |
| 9 | <1 | 1.2 | 45 | 1.3 | 1.1 | 83 | 3.3 | 2.2 |
| 10 | <1 | 1 | 46 | 1.8 | 1.1 | 84 | 3.5 | 2.5 |
| 11 | 2.4 | 2.4 | 47 | 1.2 | 1.0 | 85 | 2.6 | 1.3 |
| 12 | 1.6 | 1.2 | 48 | 2.3 | 1.3 | 86 | 3.2 | 1.9 |
| 13 | 2.4 | 1.4 | 49 | 1.1 | 1.0 | 87 | 3.1 | 2.4 |
| 14 | 2.5 | 1.9 | 50 | 1.4 | 1.0 | 88 | 3.1 | 1.7 |
| 15 | 2.2 | 1.1 | 51 | 2.9 | 1.3 | 89 | <1.0 | 2.8 |
| 16 | <1 | 1 | 54 | 1.5 | 1 | 90 | 2.4 | 1.7 |
| 17 | 1.5 | 1.2 | 55 | 2.9 | 1.8 | 91 | 2.8 | 2.4 |
| 18 | 1.3 | 1.2 | 56 | 1.4 | 2.7 | 92 | 1.6 | 3.0 |
| 19 | 1.8 | 1.3 | 57 | 2.8 | 3.2 | 93 | 2.3 | 2.8 |
| 20 | 1.1 | 1 | 58 | 2.2 | 2.6 | 94 | 2.2 | 3.0 |
| 21 | 2.4 | 2.3 | 59 | 2.8 | 1.8 | 95 | 3.1 | 2.6 |
| 22 | 2.8 | 2.5 | 60 | 1.6 | 2.3 | 96 | 2.9 | 2.6 |
| 23 | 2.7 | 1.6 | 61 | 1.3 | 1.1 | 97 | 1.2 | 1.1 |
| 24 | 1.1 | 1.1 | 62 | 2.2 | 2.3 | 98 | 1.4 | 1.1 |
| 25 | - | - | 63 | 2.3 | 2.4 | 99 | 3.6 | 2.8 |
| 26 | 2.5 | 2.1 | 64 | 2.3 | 2.4 | 100 | 2.4 | 2.7 |
| 27 | <1 | 3.7 | 65 | 2.4 | 2.6 | 101 | 2.5 | 3.7 |
| 28 | - | 1.8 | 66 | 2.2 | 2.9 | 102 | 1.8 | 3.1 |
| 29 | 2.6 | 2.7 | 67 | 2.6 | 3.5 | 103 | 2.9 | 2.6 |
| 30 | 1.4 | 1.1 | 68 | 4.4 | 4.3 | 104 | 2 | 3.6 |
| 31 | 1.6 | 1.1 | 69 | 3.0 | 1.7 | 105 | 3.7 | 2.5 |
| 32 | 1.8 | 1.3 | 70 | 3.1 | 1.2 | 106 | 1.7 | 3.4 |
| 33 | 2.5 | 3.8 | 71 | 2 | 1.3 | 107 | 4.2 | 3.1 |
| 34 | 1 | 1.1 | 72 | 1.3 | 1.0 | 108 | 1.6 | 3.6 |
| 35 | <1 | 3.7 | 73 | 3 | 2.1 | 109 | 4.4 | 3.2 |
| 36 | <1 | 3.5 | 74 | 3 | 1.9 | 114 | 1.3 | 1.1 |
| 115 | 1.4 | 1 | 52 | 3.6 | 2 | | | |

### Experimental Example 2. In vivo efficacy evaluation: Scopolamine-induced cognitive impairment model

The effect of improving cognitive function for compounds of some embodiments, which showed excellent efficacy in the in vitro assay described above, was evaluated in an animal model.

A memory loss rodent animal model induced by scopolamine intraperitoneal administration is used, and an evaluation method for memory uses a Y-maze test and a passive avoidance test (PAT). Scopolamine is a representative experimental model showing an impairment of cognitive function similar to AD patients by blocking neurotransmitters when administered to an animal as an antagonist of a muscarinic acetylcholine receptor, and as a cognitive function evaluation technique, a Y-maze test and a passive avoidance experiment, which are general and representative methods, were used, and in order to objectify the efficacy levels of the compounds, a group administered with donepezil (Aricept, Eisa) as a positive control material was separately provided to compare the efficacy levels of the compounds.

In order to evaluate cognitive function, 5-week-old male ICR mice were used, the compounds to be evaluated were prepared at a dose of 50 mg/kg/ml and repeatedly orally administered at a predetermined time for 7 days, and the positive control material was carried out at 5 mg/kg/ml. After the last administration, after 30 minutes of intraperitoneal administration of 1 mg/kg/2 ml of scopolamine, a Y-maze test and a passive avoidance test, which are cognitive function evaluations, were performed.

The Y-maze test is a method for evaluating the ability to sequentially act as an experiment for measuring short-term memory. The measurement equipment is composed of three branches, each branch has a length of 42 cm, a width of 3 cm, and a height of 12 cm, and an angle at which three branches are folded is 120°. Experimental animals are put into each branch for 8 minutes, and the number of times of entering the branches to the tail of the experimental animal and the case of being sequentially received in each branch are scored (actual alternation). The alternation behavior is defined as entering not to overlap all three, and is calculated by the following equation. 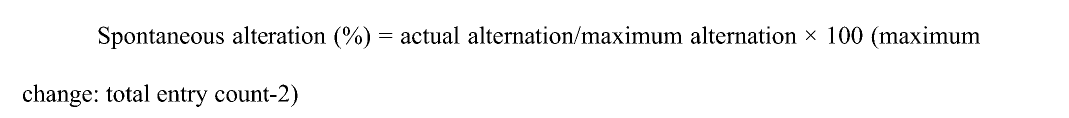 Spontaneous alteration (%) = actual alternation/maximum alternation × 100 (maximum change: total entry count-2)

In addition, the passive avoidance test was conducted using an avoidance learning box (Jeondo B&P, Korea, Seoul), and the avoidance learning box is divided into a dark room and a bright room, and when a laboratory animal is put into a bright room, the subject goes beyond the dark room. At the moment, an electric shock of 0.5 mA is applied for 5 seconds, and when the next day experimental animal is put back into a bright room, an electric shock in a dark room is memorized and stay in a bright room. In this case, a step-through latency is measured to evaluate memory. The efficacy of the compounds of the present invention against the normal group and the control group in the Y-maze test and the passive avoidance test was described in the following Table 3 (Scopolamine-induced cognitive impairment model result).

The experimental results are shown in Table 3 below in the Y-maze experiment with spontaneous modification (%) and time (sec) staying in a bright room in a passive avoidance test, and the results are shown as an effect (%) compared to a normal group and a control group. As a result, Example 3 showed a cognitive function recovery of 59% and 91% compared to normal animals in a Y-maze experiment and a manual evasion experiment, and it has a level equal to or higher than that of donepezil (such as set 35%, 94%), which is a positive control material. In addition, in the Y-maze experiment, 50% or more of the efficacy is a compound of Examples 2, 7, 15, 20, 22, 25, 31, 41, 56, and the like, and the effect of 50% or more in a passive avoidance test is shown in Examples 7, 30, 31, and 56.

**[Table 3]**

| Example | in vivo | | in vivo(effect) | | Example | in vivo | | in vivo(effect) | |
|---|---|---|---|---|---|---|---|---|---|
| | Y-maze (%) | PAT (sec) | Y-maze (%) | PAT (%) | | Y-maze (%) | PAT (sec) | Y-maze (%) | PAT (%) |
| 1 | 64 | 133 | 38 | 35 | 20 | 69 | 142 | 61 | 38 |
| 2 | 63 | 134 | 51 | 25 | 21 | - | - | - | - |
| 3 | 61 | 206 | 59 | 91 | 22 | 65 | 145 | 56 | 36 |
| 4 | - | - | - | - | 23 | 59 | 87 | 41 | 22 |
| 5 | - | - | - | - | 24 | - | - | - | - |
| 6 | 53 | 109 | -6 | 10 | 25 | 62 | 42 | 65 | 1 |
| 7 | 65 | 179 | 57 | 73 | 26 | - | - | - | - |
| 8 | 58 | 58 | 43 | 11 | 27 | 62 | 80 | 44 | 28 |
| 9 | 57 | 127 | 9 | 32 | 28 | - | - | - | - |
| 10 | - | - | - | - | 29 | - | - | - | - |
| 11 | - | - | - | - | 30 | 63 | 194 | 45 | 60 |
| 12 | - | - | - | - | 31 | 68 | 204 | 71 | 65 |
| 13 | 57 | 149 | 17 | 37 | 32 | - | - | - | - |
| 14 | 57 | 129 | 7 | 33 | 33 | 56 | 58 | 30 | 10 |
| 15 | 66 | 142 | 66 | 30 | 34 | - | - | - | - |
| 16 | - | - | - | - | 35 | 59 | 141 | 41 | 45 |
| 17 | - | - | - | - | 36 | 59 | 76 | 42 | 17 |
| 18 | - | - | - | - | 41 | 59.8 | 69.6 | 54 | 16 |
| 19 | | | | | 56 | 61.8 | 217.3 | 64 | 81 |
| normal | 69-78 (avg. 73) | 222~283 (avg.258) | 100 | 100 | control | 49~55 (avg. 52) | 20~92 (avg. 49) | 0 | 0 |
| Donepezil | 56-74 (avg. 67) | 173~251 (avg.219) | 35-111 (avg. 72) | 59-95 (avg. 82) | | | | | |

### Experimental Example 3. Ex-Vivo Efficacy Evaluation: Long Term Potentiation (LTP) Experiment

A long term potentiation (LTP) experiment is provided to be usefully used as an experimental model for evaluating synaptic operation of memory learning, and in particular, is interpreted as a very important result in studying degenerative brain diseases related to memory such as AD. In the present experiment, a cholinergic nervous system, which is a key for learning and memory, is temporarily blocked with scopolamine, and then some example compounds were evaluated.

After extracting the hippocampus from the brains of 7-day-old rats, the hippocampal slices were cultured for 14 days using the Organotypic hippocampal slice cultures (OHSCs) method and then used for experiments. The cultured hippocampal slice tissues were mounted on a microelectrode array (MEA; Multi-Channel Systems, Germany) system, and an MEA1060 amplifier was attached. Then, 300 µM of scopolamine, a neurotoxic stimulant, and 20 µM of an evaluation substance were added to the culture medium. Subsequently, after inducing LTP in the CA1 region of the brain, changes in response are measured by field excitatory postsynaptic potentials (fEPSPs). At this time, Long-term potential (LTP) induction was performed using Theta-Burst stimulation (TBS), consisting of stimulating with High-frequency stimulation (HFS) at 100 Hz for 1 second a total of 3 times at 5-minute intervals.

Table 4 shows the results of these Long Term Potentiation (LTP) experiments All drug-treated groups showed increased fEPSP values compared to the scopolamine group, with the degree of activity exceeding 130% in the order of Example 3 (194.05%) > Example 34 (152.49%) > Example 36 (152.18%) > Example 23 (139.28%) > Example 22 (134.14%), and the LTP activity of Example 3 (DM3159) was higher than that of the normal group (147%) and was significantly superior to the activity of donepezil (155.64%), a global standard AD treatment.

**[Table 4]**

| Example | fEPSPs | Example | fEPSPs |
|---|---|---|---|
| | 20uM | | 20uM |
| 1 | - | 19 | |
| 2 | 117.08 | 20 | |
| 3 | 194.05 | 21 | - |
| 4 | - | 22 | 134.14 |
| 5 | - | 23 | 139.28 |
| 6 | - | 24 | - |
| 7 | 110.55 | 25 | 107.49 |
| 8 | - | 26 | - |
| 9 | - | 27 | - |
| 10 | - | 28 | - |
| 11 | - | 29 | - |
| 12 | - | 30 | - |
| 13 | - | 31 | 124.20 |
| 14 | - | 32 | - |
| 15 | - | 33 | 152.49 |
| 16 | - | 34 | - |
| 17 | - | 35 | 123.97 |
| 18 | - | 36 | 152.18 |
| | - | Donepezil | 155.64 |

## Claims

1. A compound represented by the following Chemical Formula I, an isomer, a hydrate, a solvate, a prodrug, or a pharmaceutically acceptable salt thereof: 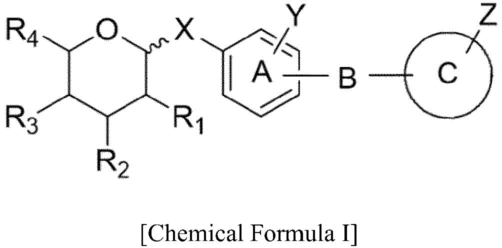 wherein,
R₁, R₂, and R₃ are each independently hydrogen, hydroxy, methoxy, fluoro, azide, amine, 10-aminodecanoate, acetyl, or benzyl;
R₄ is hydrogen or methyl;
X is oxygen, nitrogen, sulfur, or -CH₂-;
A is phenyl which is unsubstituted or substituted with one or more Y;
each substituent Y is selected from the group consisting of hydrogen, straight-chain or branched C₁-₃ alkyl, and halogen;
B is a direct bond, -[CH₂]ₙ-, -C(CH₃)₂-, -C(CF₃)₂-, -O-, -NH-, -SO₂-, 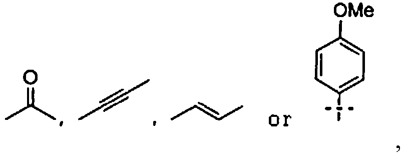 where n is an integer from 1 to 3;
ring C is C₅-₇ cycloalkyl, C₅-₇ heterocycloalkyl, phenyl, biphenyl, naphthyl, aryl, or heteroaryl which is unsubstituted or substituted with one or more Z; and
each substituent Z is selected from the group consisting of straight-chain or branched C₁-₆ alkyl, cycloalkyl, phenyl, heteroaryl, halogen, amine, cyano, nitro, alkoxy, and thiol.

2. The compound, isomer, hydrate, solvate, prodrug, or pharmaceutically acceptable salt according to claim 1, wherein
at least two or more of R₁, R₂, and R₃ are each hydroxy; and
R₄ is hydrogen.

3. The compound, isomer, hydrate, solvate, prodrug, or pharmaceutically acceptable salt according to claim 1, wherein
R₁, R₂, and R₃ are each hydroxy; and
R₄ is hydrogen.

4. The compound, isomer, hydrate, solvate, prodrug, or pharmaceutically acceptable salt according to claim 1, wherein
X is oxygen.

5. The compound, isomer, hydrate, solvate, prodrug, or pharmaceutically acceptable salt according to claim 1, wherein
A is unsubstituted phenyl.

6. The compound, isomer, hydrate, solvate, prodrug, or pharmaceutically acceptable salt according to claim 1, wherein
A is phenyl substituted with Y; and
the substituent Y is halogen.

7. The compound, isomer, hydrate, solvate, prodrug, or pharmaceutically acceptable salt according to claim 1, wherein
X-A in the Chemical Formula I is 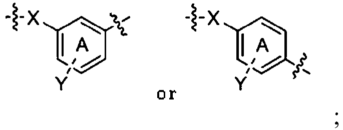 and
the substituent Y is a straight-chain or branched C₁-₃ alkyl or halogen.

8. The compound, isomer, hydrate, solvate, prodrug, or pharmaceutically acceptable salt according to claim 1, wherein
B is a direct bond.

9. The compound, isomer, hydrate, solvate, prodrug, or pharmaceutically acceptable salt according to claim 1, wherein
B is -CH₂-.

10. The compound, isomer, hydrate, solvate, prodrug, or pharmaceutically acceptable salt according to claim 1, wherein
B is -O-.

11. The compound, isomer, hydrate, solvate, prodrug, or pharmaceutically acceptable salt according to claim 1, wherein
ring C is phenyl which is unsubstituted or substituted with Z.

12. The compound, isomer, hydrate, solvate, prodrug, or pharmaceutically acceptable salt according to claim 1, wherein
B-ring C in the Chemical Formula I is

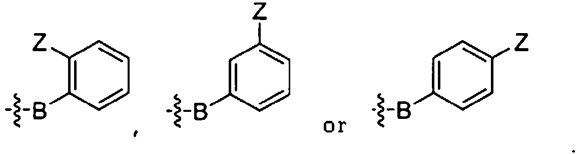

13. The compound, isomer, hydrate, solvate, prodrug, or pharmaceutically acceptable salt according to claim 1, wherein
ring C is naphthyl or heteroaryl which is unsubstituted or substituted with Z.

14. The compound, isomer, hydrate, solvate, prodrug, or pharmaceutically acceptable salt according to claim 1, wherein
ring C is C₅-₇ cycloalkyl, C₅-₇ heterocycloalkyl, aryl, or heteroaryl which is unsubstituted or substituted with Z, wherein the cycloalkyl, heterocycloalkyl, aryl, or heteroaryl is a monocyclic, bicyclic, or tricyclic ring compound.

15. The compound, isomer, hydrate, solvate, prodrug, or pharmaceutically acceptable salt according to claim 1, wherein
B-ring C in the Chemical Formula I is

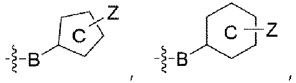

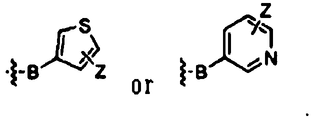

16. The compound, isomer, hydrate, solvate, prodrug, or pharmaceutically acceptable salt according to claim 1, wherein
the substituent Z is straight-chain or branched-chain C₁₋₆ alkyl, halogen, amine, or alkoxy.

17. The compound, isomer, hydrate, solvate, prodrug, or pharmaceutically acceptable salt according to claim 1, wherein
such compound is selected from the group consisting of the following from <1> to <124> compounds:
<1>(2R,3R,4S,5S)-2-(4-benzyloxyphenoxy)tetrahydro-2*H*-pyran-3,4,5-triol,
<2>(2R,3R,4S,5S)-2-(4-(4-methylbenzyl)phenoxy)tetrahydro-2*H*-pyran-3,4,5-triol,
<3>(2R,3R,4S,5S)-2-(4-(4-methoxybenzyl)phenoxy)tetrahydro-2*H*-pyran-3,4,5-triol,
<4>(2S,3R,4S,5S)-2-(4-(4-methoxybenzyl)phenoxy)tetrahydro-2*H*-pyran-3,4-diol,
<5>(3S,4S,5R,6R)-5-methoxy-6-(4-(4-methoxybenzyl)phenoxy)tetrahydro-2*H*-pyran-3,4-diol,
<6>(2R,3S,4R,5S,6S)-2-(4-(4-methoxybenzyl)phenoxy)-6-methyltetrahydro-2*H*-pyran-3,4,5-triol,
<7>(2R,3R,4S,5S)-2-(4-(4-isopropylbenzyl)phenoxy)tetrahydro-2*H*-pyran-3,4,5-triol,
<8>(2R,3R,4S,5S)-2-(2-chloro-4-(4-isopropylbenzyl)phenoxy)tetrahydro-2*H*-pyran-3,4,5-triol,
<9>(2R,3R,4S,5S)-2-(4-(4-phenoxybenzyl)phenoxy)tetrahydro-2*H*-pyran-3,4,5-triol,
<10>(2R,3R,4S,5S)-2-(4-(4-(trifluoromethoxy)benzyl)phenoxy)tetrahydro-2*H*-pyran-3,4,5-triol,
<11>(2R,3R,4S,5S)-2-(4-(4-(methylthio)benzyl)phenoxy)tetrahydro-2*H*-pyran-3,4,5-triol,
<12>(2S,3R,4S,5S)-2-(4-(4-(dimethylamino)benzyl)phenoxy)tetrahydro-2*H*-pyran-3,4,5-triol,
<13>(2R,3R,4S,5S)-2-(4-(4-fluorobenzyl)phenoxy)tetrahydro-2*H*-pyran-3,4,5-triol,
<14>(2R,3R,4S,5S)-2-(4-(4-chlorobenzyl)phenoxy)tetrahydro-2*H*-pyran-3,4,5-triol,
<15>(2R,3R,4S,5S)-2-(4-(3-methoxybenzyl)phenoxy)tetrahydro-2*H*-pyran-3,4,5-triol,
<16>(2R,3R,4S,5S)-2-(4-(3,5-bis(trifluoromethyl)benzyl)phenoxy)tetrahydro-2*H*-pyran-3,4,5-triol,
<17>(2R,3R,4S,5S)-2-(4-(3,4-dimethoxybenzyl)phenoxy)tetrahydro-2H-pyran-3,4-diol,
<18>(2R,3R,4S,5S)-2-(4-(3,4,5-trimethoxybenzyl)phenoxy)tetrahydro-2*H*-pyran-3,4,5-triol,
<19>(2R,3R,4S,5S)-2-(4-(naphthalen-2-ylmethyl)phenoxy)tetrahydro-2*H*-pyran-3,4,5-triol,
<20>(2R,3R,4S,5S)-2-(4-(6-methoxynaphthalen-2-yl)methyl)phenoxy)tetrahydro-2*H*-pyran-3,4,5-triol,
<21>(2R,3R,4S,5S)-2-(4-(cyclopentylmethyl)phenoxy)tetrahydro-2*H*-pyran-3,4,5-triol,
<22>(2R,3R,4S,5S)-2-(4-(cyclohexylmethyl)phenoxy)tetrahydro-2*H*-pyran-3,4,5-triol,
<23>(2R,3R,4S,5S)-2-(4-(thiophen-3-ylmethyl)phenoxy)tetrahydro-2*H*-pyran-3,4,5-triol,
<24>(2R,3R,4S,5S)-2-(4-(pyridin-3-ylmethyl)phenoxy)tetrahydro-2*H*-pyran-3,4,5-triol,
<25>(2R,3R,4S,5S)-2-(3-(4-methoxybenzyl)phenoxy)tetrahydro-2*H*-pyran-3,4,5-triol,
<26>(2R,3R,4S,5S)-2-((4-benzylphenyl)thio)tetrahydro-2*H*-pyran-3,4,5-thiol,
<27>(2R,3R,4S,5S)-2-((4-(4-isopropylbenzyl)phenyl)thio)tetrahydro-2*H*-pyran-3,4,5-triol,
<28>(2S,3R,4S,5S)-2-((4-(4-isopropylbenzyl)phenyl)thio)tetrahydro-2*H*-pyran-3,4,5-triol,
<29>(2R,3R,4S,5S)-2-((4-(4-methoxybenzyl)phenyl)thio)tetrahydro-2*H*-pyran-3,4,5-thiol,
<30>(2R,3R,4S,5S)-2-(4-(2-(4-methoxyphenyl)propan-2-yl)phenoxy)tetrahydro-2*H*-pyran-3,4,5-triol,
<31>(2R,3R,4S,5S)-2-(4-(2-(4-ethoxyphenyl)propan-2-yl)phenoxy)tetrahydro-2*H*-pyran-3,4,5-triol,
<32>(2R,3R,4S,5S)-2-(4-(1,1,1,3,3,3-hexafluoro-2-(4-methoxyphenyl)propan-2-yl)phenoxy)tetrahydro-2*H*-pyran-3,4,5-triol,
<33>(2R,3R,4S,5 S)-2-(4-(para-tolyloxy)phenoxy)tetrahydro-2H-pyran-3,4,5-triol,
<34>(2R,3R,4S,5S)-2-(4-(3,5-dimethylphenoxy)phenoxy)tetrahydro-2*H*-pyran-3,4,5-triol,
<35>(2R,3R,4S,5S)-2-(4-(4-isopropylphenoxy)phenoxy)tetrahydro-2*H*-pyran-3,4,5-triol,
<36>(2R,3R,4S,5S)-2-(4-(4-(tert-butyl)phenoxy)phenoxy)tetrahydro-2*H*-pyran-3,4,5-triol,
<37>(2R,3R,4S,5S)-2-(4-(3-chloro-4-methoxybenzyl)phenoxy)tetrahydro-2*H*-pyran-3,4,5-triol,
<38>(2R,3R,4S,5S)-2-(3-chloro-4-(4-methoxybenzyl)phenoxy)tetrahydro-2*H*-pyran-3,4,5-triol,
<39>(2R,3R,4S,5S)-2-(4-(4-ethoxybenzyl)phenoxy)tetrahydro-2H-pyran-3,4,5-triol,
<40>(2R,3R,4S,5S)-2-(3-chloro-4-(4-isopropylbenzyl)phenoxy)tetrahydro-2*H*-pyran-3,4,5-triol,
<41>(2R,3R,4S,5S)-2-(2-fluoro-4-(4-isopropylbenzyl)phenoxy)tetrahydro-2*H*-pyran-3,4,5-triol,
<42>(2R,3R,4S,5R)-2-(4-(4-methoxybenzyl)phenoxy)tetrahydro-2*H*-pyran 3,4,5-triol,
<43>(2R,3R,4S,5R)-2-(4-(4-isopropylbenzyl)phenoxy)tetrahydro-2*H*-pyran-3,4,5-triol,
<44>(2R,3R,4S,5S)-2-(4-(4-isopropoxybenzyl)phenoxy)tetrahydro-2*H*-pyran-3,4,5-triol,
<45>(2R,3R,4S,5S)-2-(4-(4-(cyclopentyloxy)benzyl)phenoxy)tetrahydro-2*H*-pyran-3,4,5-triol,
<46>(2R,3R,4S,5S)-2-(2-fluoro-4-(4-methoxybenzyl)phenoxy)tetrahydro-2*H*-pyran-3,4,5-triol,
<47>(2R,3R,4S,5S)-2-(3-fluoro-4-(4-methoxybenzyl)phenoxy)tetrahydro-2*H*-pyran-3,4,5-triol,
<48>(2R,3R,4S,5S)-2-(3-fluoro-4-(4-isopropylbenzyl)phenoxy)tetrahydro-2*H*-pyran-3,4,5-triol,
<49>(2R,3R,4S,5S)-2-(4-(2-chloro-4-methoxybenzyl)phenoxy)tetrahydro-2*H*-pyran-3,4,5-triol,
<50>(2R,3R,4S,5S)-2-(4-(2-fluoro-4-methoxybenzyl)phenoxy)tetrahydro-2*H*-pyran-3,4,5-triol,
<51>(2R,3R,4S,5S)-2-(2-chloro-4-(4-methoxybenzyl)phenoxy)tetrahydro-2*H*-pyran-3,4,5-triol,
<52>(2R,3R,4S,5S)-2-(4-((4'-isopropyl-[1,1'-biphenyl]-4yl)methyl)phenoxy)tetrahydro-2*H-*pyran-3,4,5-triol,
<53>(2R,3R,4S,5S)-2-(4-((5-methoxybenzo[d]oxazol-2yl)methyl)phenoxy)tetrahydro-2*H-*pyran-3,4,5-triol,
<54>(2R,3R,4S,5S)-2-(3-(4-isopropylphenoxy)phenoxy)tetrahydro-2*H*-pyran-3,4,5-triol,
<55>(2R,3R,4S,5S)-2-(4-(4-methoxyphenoxy)phenoxy)tetrahydro-2*H*-pyran-3,4,5-triol,
<56>(2R,3R,4S,5R)-2-(4-(4-isopropylphenoxy)phenoxy)tetrahydro-2*H*-pyran-3,4,5-triol,
<57>(2R,3R,4S,5S)-2-(2-chloro-4-(4-isopropylphenoxy)phenoxy)tetrahydro-2*H*-pyran-3,4,5-triol,
<58>(2R,3R,4R,5S)-5-fluoro-2-(4-(4-isopropylphenoxy)phenoxy)tetrahydro-2*H*-pyran-3,4-diol,
<59>(2R,3R,4S,5R,6R)-2-(hydroxymethyl)-6-(4-(4-isopropylphenoxy)phenoxy)tetrahydro-2*H-*pyran-3,4,5-triol,
<60>(2R,3R,4S,5S)-2-(4-(4-isopropylphenoxy)phenoxy)-4-methoxy-tetrahydro-2*H*-pyran-3,5-diol,
<61>(2R,3R,4R,5S)-2-(4-(4-isopropylphenoxy)phenoxy)-5-methoxy-tetrahydro-2*H*-pyran-3,4-diol,
<62>(3S,4S,5R,6R)-6-(4-(4-isopropylphenoxy)phenoxy)-5-methoxytetrahydro-2*H*-pyran-3,4-diol,
<63>(3S,4S,5R,6R)-5-fluoro-6-(4-(4-isopropylphenoxy)phenoxy)tetrahydro-2*H*-pyran-3,4-diol,
<64>(2R,3R,4R,5R)-5-fluoro-2-(4-(4-isopropylphenoxy)phenoxy)tetrahydro-2*H*-pyran-3,4-diol,
<65>(3S,4S,5S,6R)-5-fluoro-6-(4-(4-isopropylphenoxy)phenoxy)tetrahydro-2*H*-pyran-3,4-diol,
<66>(3S,4R,6R)-6-(4-(4-isopropylphenoxy)phenoxy)tetrahydro-2H-pyran-3,4-diol,
<67>(2R,3R,4S,5R)-2-(2-chloro-4-(4-isopropylphenoxy)phenoxy)tetrahydro-2*H*-pyran-3,4,5-triol,
<68>(2R,3R,4S, 5 S)-2-(2-fluoro-4-(4-isopropylphenoxy)phenoxy)tetrahydro-2*H*-pyran-3,4,5-triol,
<69>(3S,4R,5R,6R)-5-azido-6-(4-(4-isopropylphenoxy)phenoxy)tetrahydro-2*H*-pyran-3,4-diol,
<70>(3S,4R,5R,6R)-5-amino-6-(4-(4-isopropylphenoxy)phenoxy)tetrahydro-2*H*-pyran-3,4-diol,
<71>(4-methoxyphenyl)(4-(((2R,3R,4S,5S)-3,4,5-trihydroxytetrahydro-2*H*-pyran-2-yl)oxy)phenyl)methanone,
<72>( 4-(4-(((2S,3R,4S,5S)-3,4,5-trihydroxytetrahydro-2*H*-pyran-2-yl)oxy)benzoyl)benzonitrile,
<73>(2R,3R,4S,5S)-2-(4-(((1r,4R)-4-ethylcyclohexyl)methyl)phenoxy)tetrahydro-2*H*-pyran-3,4,5-triol,
<74>(2R,3R,4S,5S)-2-(4-(((1r,4R)-4-isopropylcyclohexyl)methyl)phenoxy)tetrahydro-2*H-*pyran-3,4,5-triol,
<75>(2S,3R,4S,5S)-2-(4-((pyrrolidin-1-ylmethyl)phenoxy)tetrahydro-2*H*-pyran-3,4,5-triol,
<76>(2S,3R,4S,5S)-2-(4-(morpholinomethyl)phenoxy)tetrahydro-2*H*-pyran-3,4,5-triol,
<77>(3S,4R,5R,6R)-5-azido-6-(4-(4-methoxybenzyl)phenoxy)tetrahydro-2*H*-pyran-3,4-diol,
<78>(3S,4R,5R,6R)-5-azido-6-(4-(4-methoxybenzyl)phenoxy)tetrahydro-2*H*-pyran-3,4-diol,
<79>(2R,3R,4S,5S)-4,5-dihydroxy-2-(4-(4-methoxybenzyl)phenoxy)tetrahydro-2*H*-pyran-3-yl 10-aminodecanoate,
<80>(2R,3R,4S,5S)-2-((4'-methoxy-[1,1'-biphenyl]-3-yl)oxy)tetrahydro-2*H*-pyran-3,4,5-triol,
<81>(2R,3R,4S,5S)-2-(4-(6-methoxybenzofuran-2-yl)phenoxy)tetrahydro-2*H*-pyran-3,4,5-triol,
<82>(2R,3R,4S,5S)-2-(4-(5-methoxy-1*H*-indol-1-yl)phenoxy)tetrahydro-2*H*-pyran-3,4,5-triol,
<83>(2S,3R,4S,5S)-2-(4-(5-methoxy-*1H*-indol-1-yl)phenoxy)tetrahydro-*2H*-pyran-3,4,5-triol,
<84>(2R,3R,4S,5S)-2-((4'-methoxy-[1,1'-biphenyl]-4-yl)oxy)tetrahydro-*2H*-pyran-3,4,5-triol,
<85>(2R,3R,4S,5S)-2-(4-(6-methoxypyridin-3-yl)phenoxy)tetrahydro-*2H*-pyran-3,4,5-triol,
<86>(2R,3R,4S,5S)-2-((4'-isopropyl-1,1'-biphenyl]-4-yl)oxy)tetrahydro-*2H*-pyran-3,4,5-triol,
<87>(2S,3R,4S,5S)-2-((4'-isopropyl-1,1'-biphenyl]-4-yl)oxy)tetrahydro-*2H*-pyran-3,4,5-triol,
<88>(2R,3R,4S,5S)-2-(4-(6-isopropylbenzo[d]oxazol-2-yl)phenoxy)tetrahydro-*2H*-pyran-3,4,5-triol,
<89>(2S,3R,4S,5S)-2-(4-(6-isopropylbenzo[d]oxazol-2-yl)phenoxy)tetrahydro-*2H*-pyran-3,4,5-triol,
<90>(2R,3R,4S,5S)-2-(4-(5-methyl-*1H*-indol-1-yl)phenoxy)tetrahydro-*2H*-pyran-3,4,5-triol,
<91>(2R,3R,4S,5S)-2-(4-(5-methoxybenzo[d]oxazol-2-yl)phenoxy)tetrahydro-*2H*-pyran-3,4,5-triol,
<92>(2S,3R,4S,5S)-2-(4-(5-methoxybenzo[d]oxazol-2-yl)phenoxy)tetrahydro-*2H*-pyran-3,4,5-triol,
<93>(2R,3R,4S,5R)-2-(4-(5-methoxybenzo[d]oxazol-2-yl)phenoxy)tetrahydro-2*H*-pyran-3,4,5-triol,
<94>(2S,3R,4S,5R)-2-(4-(5-methoxybenzo[d]oxazol-2-yl)phenoxy)tetrahydro-2*H*-pyran-3,4,5-triol,
<95>(2R,3R,4S,5S)-2-(4-(6-ethylbenzofuran-3-yl)phenoxy)tetrahydro-*2H*-pyran-3,4,5-triol,
<96>(2S,3R,4S,5S)-2-(4-(6-ethylbenzofuran-3-yl)phenoxy)tetrahydro-*2H*-pyran-3,4,5-triol,
<97>(2R,3R,4S,5S)-2-(3-(7-isopropylbenzo[d]oxazol-2-yl)phenoxy)tetrahydro-*2H*-pyran-3,4,5-triol,
<98>(2S,3R,4S,5S)-2-(3-(7-isopropylbenzo[d]oxazol-2-yl)phenoxy)tetrahydro-*2H*-pyran-3,4,5-triol,
<99>(2R,3R,4S,5S)-2-(2-chloro-4-(5-methylbenzo[d]oxazol-2-yl)phenoxy)tetrahydro-*2H-*pyran-3,4,5-triol,
<100>(2S,3R,4S,5S)-2-(2-chloro-4-(5-methylbenzo[d]oxazol-2-yl)phenoxy)tetrahydro-*2H-*pyran-3,4,5-triol,
<101>(2R,3R,4S,5R)-2-(2-fluoro-4-(4-isopropylphenoxy)phenoxy)tetrahydro-*2H*-pyran-3,4,5-triol,
<102>(2 S,3R,4S,5 S)-2-(2-chloro-4-(5-methoxybenzo[d]oxazol-2-yl)phenoxy)tetrahydro-2*H-*pyran-3,4,5-triol,
<103>(2R,3R,4S,5S)-2-(2--chloro-4-(5-methoxybenzo[d]oxazol-2-yl)phenoxy)tetrahydro-*2H-*pyran-3,4,5-triol,
<104>(2S,3R,4S,5S)-2-(2-fluoro-4-(5-methylbenzo[d]oxazol-2-yl)phenoxy)tetrahydro-*2H-*pyran-3,4,5-triol,
<105>(2R,3R,4S,5S)-2-(2-fluoro-4-(5-methylbenzo[d]oxazol-2-yl)phenoxy)tetrahydro-*2H-*pyran-3,4,5-triol,
<106>(2S,3R,4S,5S)-2-(3-(5-methoxybenzo[d]oxazo-2-yl)phenoxy)tetrahydro-*2H*-pyran-3,4,5-triol,
<107>(2R,3R,4S,5S)-2-(3-(5-methoxybenzo[d]oxazol-2-yl)phenoxy)tetrahydro-2*H*-pyran-3,4,5-triol,
<108>(2S,3R,4S,5S)-2-(4-(5-methylbenzo[d]oxazol-2-yl)phenoxy)tetrahydro-*2H*-pyran-3,4,5-triol,
<109>(2R,3R,4S,5S)-2-(4-(5-methylbenzo[d]oxazol-2-yl)phenoxy)tetrahydro-*2H*-pyran-3,4,5-triol,
<110>(2R,3R,4S,5S)-2-(4-phenethylphenoxy)tetrahydro-*2H*-pyran-3,4,5-triol,
<111>(2R,3R,4S,5S)-2-(4-((E)-styryl)phenoxy)tetrahydro-*2H*-pyran-3,4,5-triol,
<112>(2R,3R,4S,5S)-2-(4-(bis(4-methoxyphenyl)methyl)phenoxy)tetrahydro-*2H*-pyran-3,4,5-triol,
<113>(2R,3R,4S,5S)-2-((4'-(4-isopropylbenzyl)-[1,1'-biphenyl]-4-yl)oxy)tetrahydro-*2H*-pyran-3,4,5-triol,
<114>(Furan-2-yl(4-(((2S,3R,4S,5S)-3,4,5-trihydroxytetrahydro-*2H*-pyran-2-yl)oxy)phenyl)methanone,
<115>(Furan-2-yl(4-(((2R,3R,4S,5S)-3,4,5-trihydroxytetrahydro-*2H*-pyran-2-yl)oxy)phenyl)methanone,
<116>(2R,3R,4S,5S)-2-(4-(4-methoxyphenethyl)phenoxy)tetrahydro-*2H*-pyran-3,4,5-triol,
<117>(2R,3R,4S,5S)-2-(4-(4-isopropylphenethyl)phenoxy)tetrahydro-*2H*-pyran-3,4,5-triol,
<118>(2R,3R,4S,5S)-2-(4-((E)-4-methoxystyryl)phenoxy)tetrahydro-2*H*-pyran-3,4,5-triol,
<119>(2R,3R,4S,5S)-2-(4-((E)-4-isopropylstyryl)phenoxy)tetrahydro-*2H*-pyran-3,4,5-triol,
<120>(2R,3R,4S,5S)-2-(4-(benzo[d][1,3]dioxol-5-yl)phenoxy)tetrahydro-*2H*-pyran-3,4,5-triol,
<121>(2R,3R,4S,5S)-2-(4-(2,3-dihydrobenzo[b][1,4]dioxin-6-yl)phenoxy)tetrahydro-*2H-*pyran-3,4,5-triol,
<122>(2R,3R,4S,5S)-2-((4'-chloro-[1,1'-biphenyl]-4-yl)oxy)tetrahydro-*2H*-pyran-3,4,5-triol,
<123>(2R,3R,4S,5S)-2-((4"-isopropyl-[1,1':4',1"-terphenyl-4-yl)oxy)tetrahydro-*2H*-pyran-3,4,5-triol,
<124>(2R,3R,4S,5S)-2-(3-(phenylethynyl)phenoxy)tetrahydro-*2H*-pyran-3,4,5-triol.

18. The compound, isomer, hydrate, solvate, prodrug, or pharmaceutically acceptable salt according to any one of claims 1 to 17, wherein
the compound has a function of regenerating and protecting brain neurons by targeting GPCR T1R3.

19. The compound, isomer, hydrate, solvate, prodrug, or pharmaceutically acceptable salt according to claim 18, wherein
the compound is for preventing or treating a degenerative brain disease.

20. A pharmaceutical composition for preventing or treating a degenerative brain disease, comprising the compound, isomer, hydrate, solvate, prodrug, or pharmaceutically acceptable salt according to any one of claims 1 to 17.

21. The pharmaceutical composition according to claim 20, wherein
the composition has a function of regenerating and protecting brain neurons by targeting GPCR T1R3.

22. The pharmaceutical composition according to claim 20, wherein
the degenerative brain disease is at least one selected from the group consisting of stroke, dementia, Parkinson's disease, dizziness, epilepsy, peripheral neuromuscular disease, brain tumor, brain lesion disorder, brain edema, paralysis, meningitis, encephalitis, concussion, pituitary tumor, Huntington's disease, tuberculous meningitis, ataxia, mad cow disease, glioblastosis, agoraphobia, cluster headache syndrome, amnesia, Dandy-Walker syndrome, Tourette syndrome, Wernicke-Korsakoff syndrome, meningioma, and Gerstmann syndrome.

23. A food composition for preventing a degenerative brain disease, comprising the compound, isomer, hydrate, solvate, prodrug, or pharmaceutically acceptable salt according to any one of claims 1 to 17.
